# EUROPÄISCHE PATENTANMELDUNG

(11) **EP 2 336 160 A2**
(43) Veröffentlichungstag der Anmeldung: **22.06.2011**
(21) Anmeldenummer: 10179255.4
(22) Anmeldetag: 02.02.2004
(51) Int. Cl.: C07K 14/47, G01N 33/68

(54) **Amyloid-beta(1-42)-Oligomere, Derivate davon und Antikörper dafür, Verfahren zu deren Herstellung und deren Verwendung**

(30) Priorität: 31.01.2003 DE 10303974
(62) Teilanmeldung aus: 04707201.2
(71) Anmelder: Abbott GmbH & Co. KG, 65205 Wiesbaden (DE)
(72) Erfinder: Hillen, Heinz, 67454, Haßloch (DE); Striebinger, Andreas, 67346 Speyer (DE); Krantz, Carsten, 4123 Allschwill (CH); Moeller, Achim, 67269 Grünstadt (DE); Mueller, Reinhold, 67105 Schifferstadt (DE)
(74) Vertreter: Reitstötter - Kinzebach

(57) **Zusammenfassung**

Amyloid-β(1-42)-Oligomere, Derivate davon und Antikörper dafür, Verfahren zu deren Herstellung und deren Verwendung.

Die vorliegende Erfindung betrifft neuromodulierende Oligomere des Amyloid-β(1-42)-Proteins, ein spezielles Herstellungsverfahren, mit dem die Oligomere in reproduzierbarer Weise und großer Ausbeute erhältlich sind, sowie die Verwendung der Oligomere als Diagnostika und Therapeutika, zur Erzeugung oligomerspezifischer Antikörper und zum Auffinden von Substanzen, die mit den Oligomeren und deren Bildung in Wechselwirkung treten können. Entsprechende Verfahren zur Erzeugung der Antikörper bzw. zum Auffinden der Substanzen werden genauso beschrieben wie die die Antikörper selbst und die Verwendung der Antikörper bzw. Substanzen als Diagnostika und Therapeutika.

Die Erfindung betrifft in gleicher Weise auch Derivate der Oligomere, insbesondere quervernetzte Oligomere und Oligomere auf Basis verkürzter Formen des Amyloid-β(1-42)-Proteins davon, deren Herstellung und deren Verwendung.

## Beschreibung

Amyloid-β(1-42)-Oligomere, Derivate davon und Antikörper dafür, Verfahren zu deren Herstellung und deren Verwendung.

Die vorliegende Erfindung betrifft neuromodulierende Oligomere des Amyloid-β(1-42)-Proteins, ein spezielles Herstellungsverfahren, mit dem die Oligomere in reproduzierbarer Weise und großer Ausbeute erhältlich sind, sowie die Verwendung der Oligomere als Diagnostika und Therapeutika, zur Erzeugung oligomerspezifischer Antikörper und zum Auffinden von Substanzen, die mit den Oligomeren und deren Bildung in Wechselwirkung treten können.

Die Erfindung betrifft in gleicher Weise auch Derivate der Oligomere, insbesondere quervernetzte Oligomere und Oligomere auf Basis verkürzter Formen des Amyloid-β(1-42)-Proteins davon, deren Herstellung und deren Verwendung.

Entsprechende Verfahren zur Erzeugung der Antikörper bzw. zum Auffinden der Substanzen werden genauso beschrieben wie die Antikörper selbst und die Verwendung der Antikörper bzw. Substanzen als Diagnostika und Therapeutika.

Amyloid-β(1-42)-Protein, kurz auch als Aβ(1-42) bezeichnet, ist ein zentraler Bestandteil unlöslicher, aus Proteinen, Lipiden, Kohlehydraten und Salzen zusammengesetzter extrazellulärer Ablagerungen (senile oder neuritische Plaques) in den Gehirnen von Alzheimer- und Down-Syndrom-Patienten (C.L. Masters et al. PNAS 82, 4245 - 4249, 1985). Dieses in wässriger Umgebung zur Polymerisation neigende Protein kann in sehr verschiedenen molekularen Formen vorliegen.

Eine einfache Korrelation unlöslicher Proteinablagerung mit dem Auftreten oder der Progredienz von Demenzerkrankungen, wie z.B. Alzheimer, hat sich als nicht überzeugend erwiesen (R.D.Terry et al Ann.Neurol. 30. 572-580 (1991), D.W. Dickson et al. Neurobiol. Aging 16, 285-298 (1995)). Hingegen scheint der Verlust von Synapsen und kognitiver Wahrnehmung besser mit löslichen Formen des Aβ(1-42) zu korrelieren (L.F. Lue et al. Am. J. Pathol. 155, 853-862,(1999), C.A. McLean et al. Ann. Neurol. 46, 860-866 (1999)).

Zu den löslichen Formen von Aβ(1-42) gibt es im Wesentlichen zwei verschiedene Hypothesen über die molekularen Formen, die ursächlich für Demenzerkrankungen, wie Alzheimer, sein sollen.

Zum einen wird eine cytotoxische Wirkung von Aβ(1-4.2)-Protofibrillen postuliert. Dies sind noch lösliche, fibrilläre, höher aggregierte Aβ(1-42)-Formen mit Molekulargewichten im Bereich von 150-250 kDa (Arispe et al. PNAS 90. 567 (1993),

Lashuel et al., Nature 418, 291 (2002)), die aufgrund Poren formender Eigenschaften einen unkontrollierten Einstrom von Calcium durch die Membranen neuronaler Zellen bewirken sollen.

Zum anderen werden oligomere Aß(1-42)-Derivate mit Molekulargewichten im Bereich von 15-30 kDa (M.P. Lambert et al. PNAS 95, 6448-6453 (1998)) beschrieben. Diese auch als von Amyloid abstammende, diffundierbare und zur Demenz beitragende Liganden (ADDL's für *Amyloid Derived Dementing Ligands,* vgl. US-A 6,218,506 und WO 01/10900, oder für *Amyloid Derived Diffusible Ligands,* vgl. Lambert et al. *supra*) bezeichneten, nicht fibrillären Oligomere finden sich in Präparationen, die einen inhibierenden Einfluss auf die Langzeitpotenzierungsrate von Neuronen in hippocampalen Schnitten zeigen.

Der Stand der bisherigen Oligomerenforschung ist allerdings durch große Unklarheit über die eigentlich relevante Spezies charakterisiert. Die Angaben in der Literatur gehen weit auseinander. So werden in der US-A 6,218,506 ADDLs mit 3 bis 12 Untereinheiten beschrieben, wohingegen die in WO 01/10900 beschriebenen ADDLs können bis zu 24 Untereinheiten aufweisen.

Insbesondere werden wenigstens zwei Formen diskutiert, die bei gelelektrophoretischer Analyse unter nicht denaturierenden Bedingungen Molekulargewichte im Bereich von 27 bis 28 kDa und 23 bis 24 kDa (US-A 6,218,506) oder von etwa 26 kDa bis 28 kDa (WO 01/10900) bzw. 17 kDa und 27 kDa (M.P. Lambert et al. PNAS 95, 6448-6453 (1998)), und bei SDSgelelelektrophoretischer Analyse unter denaturierenden Bedingungen Molekulargewichte von 17 kDa und 22 kDa (M.P. Lambert et al. PNAS 95, 6448-6453 (1998)) bzw. von etwa 22 kDa bis etwa 24 kDa und von etwa 18 kDa bis etwa 19 kDa (WO 01/10900) aufweisen. Auch SDS-stabile Aß(1-42)-Oligomere mit Molekulargewichten im Bereich von 8 kDa und 12 kDa wurden durch Western Blot-Verfahren an Gehirnen von Alzheimer-Patienten bereits nachgewiesen (C.A. McLean et al. Ann. Neurol. 46, 860-866 (1999)).

Nachteilig an den beschriebenen Herstellvorschriften ist, dass sie inhomogene Oligomer-Präparationen ergeben.

So konnten die für die Neuromodulation verantwortlichen molekularen Formen von Aβ(1-42) noch nicht in reproduzierbarer Weise zur Verfügung gestellt, geschweige denn in eindeutiger Weise identifiziert werden.

Die Bedeutung einer homogenen Präparation kann aber z. B. aus dem Verlauf einer ersten klinischen Studie zur aktiven Immunisierung an Alzheimer-Patienten ermessen werden. Die Vakzinierung mit einer präaggregierten Form des Aβ(1-42) hat zu erheblichen Nebenwirkungen (Mengioencephalitis, Blutungen) bei einem Teil der Patienten geführt, da die gebildeten Antikörper auch die vermutlich für die Auskleidungen von Zellen notwendigen Aβ(1-42)-Formen erkannten, was zu Entzündungsreaktionen führte (D. Schenk.; Nat. Rev. Neurosci. 3, 824-828 (2002)).

Für eine maßgeschneiderte Therapie, die speziell auf die Neutralisierung der eigentlich schädigenden Proteinform abzielt, ist deswegen deren Identifizierung und definierte Herstellung unabdingbare Voraussetzung.

Darüber hinaus ist das Auftreten von N-terminal verkürzten Formen des Aβ(1-42)-Proteins in Zusammenhang mit der Alzheimerschen Erkrankung erwähnt worden. Bereits 1985 wurden in den Ablagerungen von Gehirnen verstorbener Alzheimer-Patienten neben dem Aβ(1-42) auch N-terminal verkürzte Formen nachgewiesen (C. Masters et al., PNAS 82, 4245-4249 (1985)). So ist auch bekannt, dass bestimmte im Gehirn vorkommende Proteasen, wie Neprilysin (NEP 24.11) oder IDE (kurz für "Insulin Degrading Enzyme") Aβ(1-42) abbauen können (D.J. Selkoe, Neuron 32, 177-180, (2001)).

Die Bedeutung der N-terminal verkürzten Formen in der Pathogenese der Alzheimerschen Erkrankung ist jedoch unklar (E.B. Lee et al, JBS 278, 4458-4466 (2003)). Interessanterweise akkumulieren einige Patienten mit sporadischer oder familiärer Alzheimerscher Erkrankung oder Down-Syndrom bevorzugt diese verkürzten Formen (J. Näslund et al, PNAS 91, 8378-8382, (1994), C. Russo et al., Nature 405,531-532, (2000), T.C. Saido et al, Neuron 14, 457-466, (1995)). In einer neueren Arbeit (N. Sergeant et al, J. of Neurochemistry 85,1581-1591, (2003)) wurde gezeigt, dass 60% aller unlöslichen Aß-Peptide in Gehirnen verstorbener Alzheimer-Patienten auf N-terminal verkürzten Formen basieren.

Antikörper, die gegen monomeres Aβ(1-42)-Protein und bestimmte Fragment davon gerichtet sind, wurden bereits beschrieben.

So betreffen die WO 03/016466 und WO 03/016467 den monoklonalen Antikörper 266 und Analoga davon, denen eine bestimmte Glykosylierungsstelle in CDR2 fehlt. Auch humanisierte Versionen davon (Hu-266) sind bekannt. In diesen Druckschriften sind auch weitere monkolonale Antikörper erwähnt, die wie der Antikörper 226 Epitope im Bereich der Aminosäuren 13-28 von Aβ(1-42) erkennen. Hierzu gehören die Antikörper 4G8 (auch in Lue et al. (1999), supra, erwähnt) und 1 C2. Ferner ist in McLean et al. (1999), supra, der monoklonale Antikörper 1 E8 erwähnt, der ein Epitop im Bereich der Aminosäuren 18-22 von Aβ(1-42) erkennen soll.

Zudem sind eine Reihe weiterer Antikörper bekannt, welche Epitope N-terminaler Sequenzen des Aβ(1-42) erkennen. Hierzu gehören der kommerziell erhältliche, monoklonale Antikörper 6E10 (auch in WO 01/10900; Näslund et al. (1994), supra; Sergeant et al. (2003), supra, erwähnt) sowie die monoklonalen Antikörper 3D6 und 10D5 (siehe WO 031016467) sowie Ban50 und NAB228 (Lee et al. (2003), supra).

Ferner sind noch die monoklonalen Antikörper WO2, 21 F12 und 3D6 sowie das polyklonale Serum ADA42 zu nennen (Sergeant et al. (2003), supra).

Eine Übersicht über anti-Aβ(1-42)-Antikörper, die sich in präklinischen Prüfungen befinden, findet sich Schenk et al. (2002), supra.

Die der vorliegenden Erfindung zugrunde liegende Aufgabe, die für die neuromodulierende und insbesondere neuronenschädigende Wirkung von Aβ(1-42) ursächlichen und bei Demenzerkrankungen, wie der Alzheimerschen Erkrankung und des Down-Syndroms, verstärkt auftretenden molekularen Formen zur Verfügung zu stellen, löst die Erfindung durch bestimmte Aß(1-42)-Oligomere, die in Form homogener Präparationen mit einem speziellen Verfahren aus monomerem Aβ(1-42)-Protein erhältlich sind, sowie durch bestimmte Derivate dieser Oligomere, insbesondere quervernetzte Oligomere und Oligomere auf Basis verkürzter Aβ(1-42)-Formen. Das Herstellungsverfahren ermöglicht es, schlecht in wässrigen Medien lösliches, peptidsynthetisches Aβ(1-42)-Protein in großer Ausbeute in definierte lösliche Oligomere zu überführen.

Gegenstand der vorliegenden Erfindung sind daher die in den Patentansprüchen definierten Gegenstände.

So betrifft die vorliegende Erfindung Oligomere des Amyloid-β(1-42)-Proteins, wobei die Oligomere in der SDS-Gelelektrophorese ein apparentes Molekulargewicht von etwa 15 kDa, 20 kDa, 38 kDa bzw. 48 kDa aufweisen, oder Derivate der Oligomere mit einem gegebenenfalls der Derivatisierung entsprechend veränderten Molekulargewicht.

Amyloid-β(1-42)-Protein steht für ein 42 Aminosäuren aufweisendes Polypeptid, welches sich durch proteolytische Prozessierung vom Amyloid-Vorläuferprotein (APP für *Amyloid Precursor Protein*) ableitet. Neben humanen Varianten gehören auch Isoformen des Amyloid-β(1-42)-Proteins dazu, die in anderen Organismen als Menschen, insbesondere weiteren Säugern, vor allem Ratten, vorkommen.

Gemäß einer besonderen Ausführungsform betrifft die vorliegende Erfindung Oligomere humaner Amyloid-β(1-42)-Proteine. Zu humanen Amyloid-β(1-42)-Proteinen gehören insbesondere das Protein mit der Aminosäuresequenz SEQ ID NO:1, sowie Muteine und allele Varianten davon, die sich von besagter Sequenz insbesondere durch Aminosäurenaustausch ableiten. In diesem Zusammenhang sind vor allem folgende Aminosäuresubstitutionen zu nennen: A21 G, E22K, E22Q, E22G und D23N. So gehören zu den Muteinen bzw. allelen Varianten des Amyloid-β(1-42)-Proteins erfindungsgemäß insbesondere Proteine mit einer Aminosäuresequenz SEQ ID NO:1, in der eine oder mehrere unter Alanin 21, Glutaminsäure 22 und Asparaginsäure 23 ausgewählte Aminosäuren mit einer anderen, vorzugsweise unter Glycin, Lysin, Glutamin und Asparagin ausgewählten, Aminosäure substituiert sind. Erfindungsgemäß von besonderer Bedeutung sind Substitutionen an Position 22 insbesondere mit Glutamin oder Glycin.

Gemäß einer weiteren besonderen Ausführungsform betrifft die vorliegende Erfindung Oligomere von Ratten-Amyloid-β(1-42)-Proteinen. Zu Ratten-Amyloid-β(1-42)-Proteinen gehören insbesondere das Protein mit der Aminosäuresequenz SEQ ID NO:2, sowie Muteine und allele Varianten davon, die sich von besagter Sequenz insbesondere durch Aminosäurenaustausch ableiten. In diesem Zusammenhang sind vor allem solche Aminosäuresubstitutionen zu nennen, die den für die humane Sequenz erläuterten Aminosäuresubstitutionen entsprechen.

Amyloid-β(1-42)-Protein kann nach bekannten peptidsynthetischen Verfahren oder rekombinant hergestellt werden. Darüber hinaus sind eine Reihe dieser Proteine kommerziell erhältlich. Gleiches gilt auch für Muteine und allele Varianten.

Die erfindungsgemäßen Oligomere sind durch Oligomerisierung von Amyloid-β(1-42)-Protein erhältlich. Die Oligomerisierung beinhaltet eine nichtkovalente Aggregation monomeren Amyloid-Proteins, so dass davon auszugehen ist, dass die erfindungsgemäßen Oligomere aus mehreren Amyloid-β(1-42)-Proteinmonomeren zusammengesetzt sind.

Je nach Oligomerisierungsgrad weisen die erfindungsgemäßen Oligomere unterschiedliche Molekulargewichte auf. So können den Oligomeren mittels denaturierender Gelelektrophorese apparente Molekulargewichte zugeordnet werden. Diese betragen etwa 15 kDa für das Oligomer A1, etwa 20 kDa für das Oligomer A2, etwa 38 kDa für das Oligomer B1 und etwa 48 kDa für das Oligomer B2, wenn man die Gelelektrophorese unter denaturierenden Standardbedingungen (Tris-Glycin-Gel, 4-20 %, vgl. Lämmli UK, Nature 227, 680-685 (1970)) durchführt, wobei folgende Standardproteine bei gleichen Bedingungen folgende apparente Molekulargewichte aufweisen: Myosin 250 kDa, Rinder-Serumalbumin 98 kDa, Glutaminhydrogenase 64 kDa, Carboanhydrase 36 kDa, Myoglobin 30 kDa, Lysozym 16 kDa, Aprotinin 6 kDa, Insulin B-Kette 4 kDa (vgl. Blue Pre-stained Standard). Einem weiteren Aspekt zufolge betragen die Molekulargwichte für die Oligomere B etwa 64 bis 90 kDa, wenn man die Gelelektrophorese unter nativen Standardbedingungen (Tris-Glycin-Gel, 4-20 %) durchführt, wobei folgende Standardproteine bei gleichen Bedingungen folgende apparente Molekulargewichte aufweisen: Myosin 250 kDa, Rinder-Serumalbumin 98 kDa, Glutaminhydrogenase 64 kDa, Carboanhydrase 36 kDa, Myoglobin 30 kDa, Lysozym 16 kDa, Aprotinin 6 kDa, Insulin B-Kette 4 kDa (vgl. Blue Pre-stained Standard).

Einem weiteren Aspekt zufolge sind die erfindungsgemäßen Oligomere durch eine Affinität zu neuronalen Zellen gekennzeichnet. Es ist davon auszugehen, dass die Oligomere an bestimmte Zelloberflächenproteine, insbesondere Rezeptoren, binden.

Demnach betrifft die vorliegende Erfindung auch ein Verfahren zur Bestimmung der Bindung eines erfindungsgemäßen Oligomers an eine vorgegebene zelluläre Struktur, wobei man
i) das erfindungsgemäße Oligomer auf die zelluläre Struktur einwirken läßt und
ii) bestimmt, ob das erfindungsgemäße Oligomer an die zelluläre Struktur bindet.

Einem weiteren Aspekt zufolge sind die erfindungsgemäßen Oligomere durch eine neuromodulierende Wirkung gekennzeichnet. Diese neuromodulierende Wirkung kann sich insbesondere in einer verminderten Überlebensfähigkeit von neuronalen Zellen, beispielsweise Neuroblastom-Zellen manifestieren (Neurotoxizität), wenn man wenigstens ein erfindungsgemäßes Oligomer auf eine Kultur dieser Zellen einwirken lässt. Dabei kann die Überlebensfähigkeit der Zellen in an sich bekannter Weise beurteilt werden, beispielsweise indem man das Ausmaß der bei Einwirkung der erfindungsgemäßen Oligomere bewirkten Apoptose bestimmt. Hierzu stehen geeignete Testverfahren, beispielsweise kolorimetrische Verfahren auf Basis von 3-[4,5-Dimethylthiazol-2-yl]-2,5-diphenyltetrazoliumbromid (MTT), zur Verfügung. *In vivo* kann sich diese neuromodulierende Wirkung insbesondere in einer Modulation der Feuerungsrate von Neuronen manifestieren.

Demnach betrifft die vorliegende Erfindung auch ein Verfahren zur Bestimmung der Aktivität, insbesondere der Neurotoxizität, eines erfindungsgemäßen Oligomers, wobei man
i) das erfindungsgemäße Oligomer auf Zellen einwirken läßt und
ii) bestimmt, ob sich der Zustand der Zellen verändert.

Für besagte Verfahren kann man erfindungsgemäßes Oligomer in der oben beschriebenen Weise bereitstellen, z.B. in Form einer der oben angegebenen Zusammensetzungen. Die Zellen bzw. zellulären Strukturen werden zweckmäßigerweise in vitro, insbesondere als Zellkultur bzw. bei zellulären Strukturen auch als Homogenate, bereitgestellt. Hierbei dienen neuronale Zellen und insbesondere Neuroblastomzellen zur Bestimmung der Neurotoxizität. Alternativ können die Zellen auch in vivo, insbesondere als Teil eines Organismus, z.B. eines Versuchstiers, oder ex vivo, bereitgestellt werden.

Der Zustand der Zellen wird in der Regel zumindest einmal vor und zumindest einmal nach dem Einwirken des Oligomers bestimmt. Ergibt ein Vergleich zwischen dem Zustand vor und dem Zustand nach dem Einwirken eine Abweichung, besitzt das getestete Oligomer Aktivität.

Die Art des zu bestimmenden Zustandes richtet sich nach der Art der zu bestimmenden Aktivität. Eine Neurotoxizität lässt sich bestimmen, indem man beispielsweise die Überlebensfähigkeit bestimmt. Dazu kann man den Anteil lebender Zellen bezogen auf die Gesamtzellzahl vor und nach dem Einwirken des Oligomers bestimmen und miteinander vergleichen.

Aufbauend auf obigen Verfahren zur Bestimmung der Bindung oder Aktivität, kann man bestimmten Ausführungsformen zufolge Substanzen dahingehend testen, ob sie die Bindung erfindungsgemäßer Oligomere inhibieren und/oder deren Aktivität modulieren, also insbesondere verringern oder im wesentlichen vollständig hemmen (inhibieren), oder verstärken.

Im Prinzip wird dazu das Verfahren wenigstens zweimal durchgeführt, einmal in Gegenwart der Testsubstanz und ein weiteres Mal ohne Testsubstanz. Hierzu wird die Testsubstanz den Oligomeren in der Regel nach ihrer Bereitstellung zugesetzt.

Soll allerdings bestimmt werden, ob eine zu testende Substanz die Bildung der Oligomere beeinflusst oder nicht, wird die Substanz zweckmäßigerweise bereits vor Bildung der Oligomere, also bereits vor ihrer Bereitstellung, beispielsweise dem oder den für die Oligomerbildung eingesetzten Edukt(en), zugesetzt. So kann man das erfindungsgemäße Herstellverfahren unter Zusatz der Testsubstanz durchführen und anschließend bestimmen, ob und in welchem Ausmaß die Oligomere gebildet werden. Dazu kann man bestimmen, ob die auf diese Weise erhaltenen Verfahrensprodukte die Eigenschaften der erfindungsgemäßen Oligomere besitzen, also beispielsweise deren Molekulargewicht, Bindungsfähigkeit und Aktivität haben.

Im Sinne einer möglichst ausgeprägten neuromodulierenden Wirkung sind die erfindungsgemäßen Oligomere B zu bevorzugen. Auch Derivate dieser Oligomere sind in dieser Hinsicht bevorzugt, wobei insbesondere die Oligomere auf Basis eines N-terminal verkürzten Aβ(1-42)-Proteins hervorzuheben sind.

Aus verfahrenstechnischen Gründen können die Oligomere A bzw. die Oligomere B als Gemisch in Form von Zusammensetzungen anfallen, die neben den Oligomeren ferner geringe Anteile an weiteren Polypeptiden, insbesondere monomerem Amyloid-β(1-42)-Protein und gegebenenfalls auch höhermolekularer Formen aggregierten Amyloid-β(1-42)-Proteins enthalten. Derartige Zusammensetzungen sind ebenfalls Gegenstand der vorliegenden Erfindung und zeichnen sich insbesondere dadurch aus, dass der Anteil an erfindungsgemäßem(n) Oligomer(en) bezogen auf die Gesamtheit der vom Amyloid-β(1-42)-Protein abstammenden Proteine wenigstens 70 Gew.-%, vorzugsweise wenigstes 90 Gew.-% und insbesondere wenigstens 95 Gew.-% beträgt. Bei einer Präparation der Oligomere A liegt der Anteil des Oligomers A2 (20 kDa-Bande im SDS-Gel) bei wenigstens 50 Gew.-% und vorzugsweise bei wenigstens 70 Gew.-% und insbesondere bei wenigstens 85 Gew.- %. Bei einer Präparation der Oligomere B liegt der Anteil der Oligomere B1 und B2 (38 kDa- und 48 kDa-Banden im SDS-Gel) bei wenigstens 60 Gew.-%, vorzugsweise bei wenigstens 75 Gew.-% und insbesondere bei wenigstens 90 Gew.-%.

Die erfindungsgemäßen Oligomere können auch derivatisiert sein. Zweck derartiger Derivatisierungen kann es beispielsweise sein, die physikochemischen Eigenschaften der Oligomere insbesondere im Hinblick auf die Bioverfügbarkeit zu modulieren, die Oligomere mit einer nachweisbaren Markierung zu versehen oder zu immobiliseren, z.B. an Träger koppeln zu können. Markierung und Immobilisierung haben insbesondere für diagnostische Anwendungen Bedeutung.

Geeignete im proteinbiochemischen Bereich geläufige Markierungen sind dem Fachmann hinlänglich bekannt. Hierzu gehören Fluoreszenz-Markierungen, beispielsweise bestimmte Fluorescein- und Tetramethylrhodamin-Derivate, lumineszierende Markierungen, kolorimetrische Markierungen, radioaktive Markierungen und magnetische Markierungen sowie Markierungen mit Affinität zu komplementären Bindungspartnern, wie Biotin- und Streptavidin-Derivate.

Hinsichtlich der apparenten Molekulargewichte ist zu beachten, dass die Oligomerderivate Molekulargewichte aufweisen, die im Vergleich zu den nicht derivatisierten Oligomeren entsprechend erhöht sein können, wobei die Aggregationszahl aber die gleiche ist. So weist beispielsweise ein auf dem Aβ(1-42)-Oligomer B1 mit einem Molekulargewicht im SDS-Gel von 38 kDa aufbauendes Biotin-Derivat ein Molekulargewich von 42 kDa auf.

Gemäß einer besonderen Ausführungsform der vorliegenden Erfindung sind die Oligomere quervernetzt. Geeignete Quervernetzungsmittel sind dem Fachmann bekannt und stellen in der Regel bifunktionelle Reagenzien dar, wie Formaldehyd, Glutardialdehyd, Disuccinimidylsuberat, Dithiobis(succinimidylpropionat), Disuccinimidyltartrat, Disulfosuccinimidyltartrat, Dimethyladipimidat, Dimethylpimelidat, Dimethylsuberimidat, Dimethyl-3,3'-dithiobispropionimidat, N-γ-maleinimidobutyloxysuccinimidester, Succinimidyl-4(N-maleinimidomethyl)-cyclohexan-1-carboxylat, N-Succinimidyl-(4-iodacetyl)aminobenzoat und N-Succinimidyl-3-(2-pyridyldithio)propionat.

Derartige quervernetzte Oligomere haben den Vorteil, dass sie stabilisiert sind und ihre Oligomerisierung in der Regel nicht mehr reversibel ist. Sie eignen sich daher besonders für den Einsatz in diagnostischen Testsystemen bzw. als Immunogen zur Herstellung oligomerspezifischer Antikörper.

Zu den Derivaten erfindungsgemäßer Oligomere gehören auch Oligomere von Fragmenten des Amyloid-β(1-42)-Proteins. Bevorzugt sind diejenigen Fragmente, die durch Einwirkung natürlich vorkommender Proteasen erhältlich sind. Insbesondere die durch Proteolyse unter nicht denaturierenden Bedingungen in physiologischen Puffern erhältlichen Fragmente besitzen eine im Vergleich zum Amyloid-β(1-42)-Protein erhöhte proteolytische Stabilität. Bevorzugt werden Fragmente, die durch Einwirkung von Endopeptidasen erhältlich sind. Gemäß einer besonderen Ausführungsform der vorliegenden Erfindung sind die Fragmente erhältlich durch Einwirkung von Trypsin, Chymotrypsin, Thermolysin, Elastase, Papain oder Endoproteinase GluC. Einem weiteren Aspekt zufolge sind diejenigen Fragmente bevorzugt, deren erfindungsgemäße Oligomere durch eine neuromodulierende Wirkung gekennzeichnet sind. Zur weiteren Erläuterung dieses Aspekts wird auf die entsprechenden Ausführungen zur neuromodulierenden Wirkung erfindungsgemäßer Oligomere des Amyloid-β(1-42)-Proteins verwiesen.

Strukturell sind bevorzugte Fragmente insbesondere dadurch gekennzeichnet, dass sie sich vom Amyloid-β(1-42)-Protein durch Abspaltung N-terminaler Sequenzen ableiten. Einem Aspekt zufolge kann es sich bei diesen N-terminalen Sequenzen um Fragmente mit bis zu 23, vorzugsweise mit bis zu 21 und insbesondere mit bis zu 19 Aminosäuren der N-terminalen Sequenz des Amyloid-β(1-42)-Proteins handeln. Dementsprechend werden erfindungsgemäß Fragmente des Aβ(1-42)-Proteins bevorzugt, deren Sequenz die Aminosäuren 24 bis 42, vorzugsweise 22 bis 42 und insbesondere 20 bis 42 in kontinuierlicher Abfolge umfasst. Einem weiteren Aspekt zufolge kann es sich bei den abzuspaltenden N-terminalen Sequenzen um Sequenzen mit mindestens 7, vorzugsweise mit mindestens 9 und insbesondere mit mindestens 11 Aminosäuren der N-terminalen Sequenz des Amyloid-β(1-42)-Proteins handeln. Dementsprechend werden erfindungsgemäß insbesondere Fragmente des Amyloid-β(1-42)-Proteins bevorzugt, die N-terminal um 7 bis 23, vorzugsweise 9 bis 21 und insbesondere 11 bis 19 Aminosäuren verkürzt sind. Diese Fragmente entsprechen der Formel Aß(x-42), wobei x für 8 bis 24, vorzugsweise 10 bis 22 und insbesondere 12 bis 20 steht. Erfindungsgemäß sind die Aß(x-42)-Fragmente daher vorzugsweise auszuwählen unter folgenden Fragmenten: Aβ(8-42), Aβ(9-42), Aβ(10-42), Aβ(11-42), Aβ(12-42), Aβ(13-42), Aβ(14-42), Aβ(15-42), Aβ(16-42), Aβ(17-42), Aβ(18-42), Aβ(19-42), Aβ(20-42), Aβ(21-42), Aβ(22-42), Aβ(23-42) und Aβ(24-42). Spezielle Fragmente sind das durch Einwirkung von Thermolysin erhältliche Amyloid-β(20-42)-Fragment sowie das durch Einwirkung von Endoproteinase GluC erhältliche Amyloid-β(12-42)-Fragment.

Zu erfindungsgemäßen Derivaten gehören auch solche Formen, die sich von einem Amyloid-β(1-42)-Protein ableiten, das C-terminal 1 oder 2 weitere Aminosäuren aufweist. So gehören beispielsweise Oligomere des Aβ(1-43)-Proteins oder obigen Ausführungen entsprechende Derivate davon dazu.

Das erfindungsgemäße Verfahren zur Herstellung der Oligomere kann im Wesentlichen drei Schritte beinhalten, von denen der erste Schritt optional aber vorteilhaft ist, der zweite Schritt für die Herstellung der Oligomere A und B zwingend ist und der dritte Schritt der Herstellung der erfindungsgemäßen Oligomere B dient.

Schritt 1 betrifft die Entfaltung des Proteins. Dazu kann man Wasserstoffbrückenbrechende Agenzien, wie z.B. Hexafluorisopropanol (HFIP), auf das Protein einwirken lassen. Einwirkungszeiten von wenigen Minuten, beispielsweise etwa 10 bis 60 Minuten, reichen dann aus, wenn die Einwirkungstemperatur etwa 20 bis 50°C und insbesondere etwa 35 bis 40°C beträgt. Anschließendes Lösen des zur Trockne eingedampften Rückstandes, vorzugsweise in konzentrierter Form, in geeigneten, mit wässrigen Puffern mischbaren, organischen Lösungsmitteln, wie z. B. Dimethylsulfoxid (DMSO), ergibt eine in Schritt 2 des erfindungsgemäßen Verfahrens einsetzbare Suspension des zumindest partiell entfalteten Proteins. Erforderlichenfalls kann die Stammsuspension zwischenzeitlich bei tiefer Temperatur, beispielsweise bei etwa -20°C gelagert werden.

Alternativ zu obigem Schritt 1 kann man das Protein in leicht saurer, vorzugsweise wässriger Lösung, beispielsweise einer etwa 10 mM wässrigen HCl-Lösung, aufnehmen. Nach einer in der Regel wenige Minuten umfassenden Inkubationszeit werden unlösliche Bestandteile abzentrifugiert. Einige Minuten bei 10.000 g sind zweckmäßig. Diese Verfahrensschritte werden vorzugsweise bei Raumtemperatur, d.h. einer Temperatur im Bereich von 20 bis 30°C durchgeführt. Der nach Zentrifugation erhaltene Überstand enthält das Amyloid-β(1-42)-Protein und kann zwischenzeitlich bei tiefer Temperatur, beispielsweise bei etwa -20°C gelagert werden.

Schritt 2 betrifft die Oligomerisierung des Proteins zu den Oligomeren A. Dazu lässt man ein Detergenz auf das gegebenenfalls zumindest partiell entfaltete Protein einwirken, bis ausreichend Oligomer A entstanden ist.

Bevorzugt verwendet werden ionische Detergenzien, insbesondere anionische Detergenzien.

Gemäß einer besonderen Ausführungsform verwendet man ein Detergenz der Formel (I):

R-X,

worin
der Rest R für gegebenenfalls verzweigtes Alkyl mit 6 bis 20 und vorzugsweise 10 bis 14 Kohlenstoffatomen oder gegebenenfalls verzweigtes Alkenyl mit 6 bis 20 und vorzugsweise 10 bis 14 Kohlenstoffatomen steht, der Rest X für eine saure Gruppe oder deren Salz steht, wobei X vorzugsweise ausgewählt ist unter -COO⁻M⁺, -SO₃⁻M⁺ und vor allem -OSO₃-M⁺ und M⁺ für ein Wasserstoffkation oder ein vorzugsweise unter Alkali- und Erdalkalimetallkationen und Ammoniumkationen ausgewähltes anorganisches oder organisches Kation steht.

Vorteilhaft sind Detergenzien der Formel (I), worin R für unverzweigtes Alkyl steht, wobei hiervon insbesondere Alk-1-yl-Reste zu nennen sind. Besonders bevorzugt ist Natriumdodecylsulfat (SDS). Auch Laurylsäure und Ölsäure lassen sich vorteilhaft einsetzen. Besonders vorteilhaft ist auch das Na-Salz des Detergenz Lauroylsarcosin (auch als Sarkosyl NL-30 oder Gardol^{®} bekannt).

Insbesondere die Einwirkungszeit des Detergenz hängt davon ab, ob - und wenn ja, in welchem Ausmaß - das in die Oligomerisierung eingetragene Protein entfaltet ist. Wurde das Protein gemäß Schritt 1 zuvor mit einem Wasserstoffbrücken-brechenden Agens, also insbesondere mit Hexafluorisopropanol, behandelt, reichen Einwirkungszeiten im Bereich von wenigen Stunden, vorteilhafterweise etwa 1 bis 20 und insbesondere etwa 2 bis 10 Stunden dann aus, wenn die Einwirkungstemperatur etwa 20 bis 50°C und insbesondere etwa 35 bis 40 °C beträgt. Wird von weniger oder im Wesentlichen nicht entfaltetem Protein ausgegangen, sind entsprechend längere Einwirkungszeiten zweckmäßig. Wurde das Amyloid-β(1-42)-Protein beispielsweise gemäß der oben alternativ zu Schritt 1 angegebenen Vorgehensweise vorbehandelt oder wird es direkt in Schritt 2 eingetragen, reichen Einwirkungszeiten im Bereich von etwa 5 bis 30 Stunden und insbesondere von etwa 10 bis 20 Stunden dann aus, wenn die Einwirkungstemperatur etwa 20 bis 50°C und insbesondere etwa 35 bis 40 °C beträgt. Nach der Inkubation werden unlösliche Bestandteile vorteilhafterweise abzentrifugiert. Einige Minuten bei 10.000 g sind zweckmäßig.

Die zu wählende Detergenz-Konzentration hängt vom verwendeten Detergenz ab. Wird SDS verwendet, so erweist sich eine Konzentration im Bereich von 0,01 bis 1 Gew.-%, vorzugsweise von 0,05 bis 0,5 Gew.-%, beispielsweise von etwa 0,2 Gew.- % als zweckmäßig. Werden Laurylsäure oder Ölsäure verwendet, sind etwas höhere Konzentrationen zweckmäßig, etwa in einem Bereich von 0,05 bis 2 Gew.-%, vorzugsweise von 0,1 bis 0,5 Gew.-%, beispielsweise von etwa 0,5 Gew.-%.

Die Einwirkung des Detergenz sollte bei einer Salzkonzentration etwa im physiologischen Bereich erfolgen. So sind insbesondere NaCl-Konzentration im Bereich von 50 bis 500 mM, vorzugsweise von 100 bis 200 mM und besonders bei etwa 140 mM zweckmäßig.

Die resultierende, die Oligomere A, d.h. insbesondere die Oligomere A1 und/oder A2 enthaltende Lösung kann zwischenzeitlich bei tiefer Temperatur, beispielsweise bei etwa -20°C gelagert werden. Sie kann als solche der erfindungsgemäßen Verwendung bzw. der weiteren Umsetzung zu den Oligomeren B zugeführt werden, oder es können sich zunächst weitere Aufarbeitungs- bzw. Reinigungsschritte anschließen, mit denen insbesondere eine weitere Anreicherung wenigstens eines der erfindungsgemäßen Oligomere A erzielt wird. Insbesondere kann man mittels chromatographischer Verfahren die erfindungsgemäßen Oligomere von weiteren, in der Lösung enthaltenden Proteinbestandteilen und insbesondere solchen, die sich vom Amyloid-β(1-42)-Protein ableiten, abtrennen. Insbesondere bieten sich zu diesem Zweck affinitätschromatographische Verfahren an, bei denen beispielsweise spezifische Antikörper, also insbesondere auch die erfindungsgemäßen oligomerspezifischen Antikörper, zum Einsatz kommen können.

Schritt 3 betrifft die Oligomerisierung zu den Oligomeren B. Vorzugsweise wird eine Oligomer A enthaltende Zusammensetzung als Edukt für diesen Schritt gewählt. Insofern besitzen die Oligomere A erfindungsgemäß Bedeutung als Zwischenprodukte zur Herstellung der Oligomere B. Stammt diese Zusammensetzung aus Schritt 2, so enthält sie regelmäßig Detergenz und eine Salzkonzentration im physiologischen Bereich. Dann ist es zweckmäßig, die Detergenzwirkung bzw. die Salzkonzentration zu verringern. Dies kann durch eine Verringerung der Detergenz- bzw. Salzkonzentration, z.B. durch Verdünnen, zweckmäßigerweise mit Wasser oder einem Puffer niedrigerer Salzkonzentration, z. B. Tris-HCl, pH 7,3, erfolgen. Verdünnungsfaktoren im Bereich von etwa 2 bis 10, vorteilhafterweise im Bereich von etwa 3 bis 8 und insbesondere von etwa 4 haben sich als geeignet erwiesen. Die Verringerung der Detergenzwirkung kann auch durch Zugabe von Substanzen erzielt werden, welche die Detergenzwirkung neutralisieren können. Hierzu gehören beispielsweise Substanzen, welche die Detergenzien zu komplexieren vermögen, wie Substanzen, die Zellen im Zuge von Reinigungs- und Extraktionsmaßnahmen zu stabilisieren vermögen, z.B. bestimmte EO/PO-Blockcopolymere, insbesondere das unter dem Handelsnamen Pluronic® F 68 geführte Blockcopolymer. Geeignet sind auch alkoxylierte und insbesondere ethoxylierte Alkylphenole wie die ethoxylierten t-Octylphenole der Triton® X-Serie, insbsondere Triton® X100, 3-(3-Cholamidopropyl-dimethylammonio)-1-propansulfonat (CHAPS®) oder alkoxylierte und insbesondere ethoxylierte Sorbitanfettsäureester wie die der Tween®-Serie, insbesondere Tween® 20, in Konzentrationsbereichen im Bereich oder oberhalb der jeweiligen kritischen Mizellenkonzentration.

Anschließend inkubiert man die Lösung, bis ausreichend Oligomer B entstanden ist. Einwirkungszeiten im Bereich von mehreren Stunden, vorzugsweise im Bereich von etwa 10 bis 30 Stunden und insbesondere im Bereich von etwa 15 bis 25 Stunden reichen dann aus, wenn die Einwirkungstemperatur etwa 20 bis 50°C und insbesondere etwa 35 bis 40 °C beträgt. Anschließend kann man die Lösung konzentrieren und eventuelle Rückstände abzentrifugieren. Auch hier erweisen sich wenige Minuten bei 10.000 g als zweckmäßig. Der nach Zentrifugation erhaltene Überstand enthält Oligomere B.

Die resultierende, die Oligomere B, d.h. insbesondere die Oligomere B1 und/oder B2 enthaltende Lösung kann zwischenzeitlich bei tiefer Temperatur, beispielsweise bei etwa -80°C gelagert werden. Sie kann als solche der erfindungsgemäßen Verwendung zugeführt werden oder es können sich zunächst weitere Aufarbeitungs- bzw. Reinigungsschritte anschließen. Diesbezüglich wird auf die obige Ausführungen zu entsprechenden Maßnahmen im Zusammenhang mit den Oligomeren A Bezug genommen.

Weitere Aufarbeitungs- bzw. Reinigungsschritte können auch zu dem Zweck durchgeführt werden, das zur Herstellung der Oligomere verwendete Detergenz im Wesentlichen vollständig zu entfernen. Beispielsweise können die Oligomere B zunächst aus der Detergenz-haltigen Lösung ausgefällt, gewonnen und erneut in Detergenz-freiem Medium gelöst werden. Maßnahmen zum Ausfällen von Proteinen sind dem Fachmann hinlänglich bekannt. Erfindungsgemäß hat sich die Zugabe wässrig-methanolischer Essigsäure als zweckmäßig erwiesen. Ohne an einen bestimmten Mechanismus gebunden zu sein, scheint das Detergenz bzw. die Variation von Detergenz- und Salzkonzentrationen das Protein in definierte lösliche Formen zu bringen, die sich von der in wässrigen physiologischen Puffern gelösten Ausgangsform des Proteins klar unterscheiden, was sich z.B. mittels denaturierender oder nativer Gelelektrophorese oder Gelpermeationschromatographie feststellen lässt. Dies ist erstaunlich, da Detergenzien normalerweise Proteinaggregate zu desaggregieren, d. h. in Untereinheiten zu zerlegen vermögen, wohingegen erfindungsgemäß ausgehend von einem zur Aggregation neigenden Monomer definierte Oligomere erhalten werden.

Zur Herstellung von Derivaten erfindungsgemäßer Oligomere geht man zweckmäßigerweise so vor, dass man das vorstehend beschriebene erfindungsgemäße Verfahren an bereits entsprechend derivatisiertem Amyloid-β(1-42)-Protein durchführt. Alternativ kann auch eine Derivatisierung des Oligomers vorgenommen werden, wodurch die Struktur des Oligomers allerdings nicht verändert werden sollte. Geeignete proteinchemische Maßnahmen sind dem Fachmann bekannt.

Die Quervernetzung erfindungsgemäßer Oligomere oder Derivate davon kann in an sich bekannter Weise erfolgen. Verwendet man beispielsweise Glutardialdehyd als Quervernetzungsmittel, kann eine aus dem erfindungsgemäßen Verfahrensschritt 2 oder 3 resultierende Lösung mit einer Glutardialdehyd-Lösung behandelt werden. Nach wenigen Stunden bei Raumtemperatur haben die Oligomere mit dem Glutardialdehyd reagiert. Die Reaktion kann dann in an sich bekannter Weise durch Umsetzung des überschüssigen Glutardialdehyds mit allgemein zu diesem Zweck bekannten Reagenzien wie Ethanolamin gestoppt werden. Je nachdem, ob man die erfindungsgemäßen Oligomere A oder B quervernetzt, erhält man eine Lösung erfindungsgemäßer quervernetzter Oligomere, die mit A-CL bzw. B-CL bezeichnet werden.

Insbesondere für die gegebenenfalls quervernetzten Oligomere B bzw. Derivate davon ist es möglich, im Anschluss an deren Synthese die Salzkonzentration wieder zu erhöhen, ohne die Stabilität der Oligomere zu beeinträchtigen. Dies ist im Hinblick auf die Verwendung der Oligomere wichtig, z.B. für den Fall, dass bei der Verwendung physiologische Bedingungen zweckmäßig sind (zelluläre Anwendungen, *in vivo* Anwendungen).

Oligomere von Fragmenten des Amyloid-β(1-42)-Proteins können im Prinzip entweder ausgehend von entsprechenden Monomerfragmenten durch Oligomerisierung oder ausgehend von Oligomeren des Amyloid-β(1-42)-Proteins durch Proteolyse hergestellt werden. So kann der zweiten Verfahrensvariante folgend eine aus den erfindungsgemäßen Verfahrensschritten 2 oder 3 resultierende Lösung mit Protease behandelt werden. Ist der gewünschte Proteolysegrad erreicht, wird die Protease in allgemein bekannter Weise inaktiviert. Die resultierenden Oligomere können dann in Anlehnung an hier bereits beschriebene Vorgehensweisen gewonnen sowie erforderlichenfalls durch weitere Aufarbeitungs- bzw. Reinigungsschritte weiterverarbeitet werden.

Die erfindungsgemässen Oligomere und die diese enthaltenden Zusammensetzungen zeichnen sich durch ihre Homogenität und Stabilität aus. Sie sind in physiologischen Medien, z.B. physiologischer Kochsalzlösung, löslich und unterscheiden sich von fibrillären Formen durch ihre eher globuläre Erscheinung. Sie besitzen den Vorteil, eine Raumstruktur aufzuweisen, die sich von anderen Aβ(1-42)-Formen, insbesondere dem Monomer, nicht toxischen Oligomeren, dem Aβ(1-42) umfassenden Vorläufermolekül APP, Protofibrillen und Fibrillen, deutlich unterscheidet. Sie eignen sich daher in besonderem Maße für die Erzeugung spezifischer Antikörper sowohl in vivo als auch in vitro und ermöglichen z.B. eine spezifische aktive Immunisierung. Dabei kann es entscheidend sein, für die Immunisierung nur diejenigen Oligomere zu verwenden, die für die Krankheit auslösend sind. Andere Formen des Aβ(1-42), wie das monomere Molekül oder kleinere Oligomere können für wichtige Signalfunktionen im Organismus notwendig sein. Ebenso kann die Entfernung der fibrillären Ablagerungen, die vermutlich für die Auskleidung der Zellen von Bedeutung sind, den Organismus schädigen.

In gleicher Weise lassen sich mit den erfindungsgemässen, homogenen Oligomeren und diesen enthaltenden Zusammensetzungen Therapiemöglichkeiten realisieren, wie die passive Immunisierung, der Einsatz von Destabilisatoren der oligomeren Formen und der Einsatz von Rezeptor(partial)agonisten oder -antagonisten. So ermöglicht eine homogene Oligomerenpräparation auch eine gezielte Herstellung von polyklonalen oder monoklonen Antikörpern für die passive Immunisierung. Auch die Auffindung von krankheitsrelevanten Rezeptormolekülen oder Signalmolekülen, die durch diese oligomere Form beeinflusst werden, ist mit den erfindungsgemässen Oligomeren und diesen enthaltenden Zusammensetzungen möglich.

Die erfindungsgemäßen Oligomere besitzen aufgrund ihrer Beteiligung an Amyloid-ß-Protein-assoziierten physiologischen Prozessen diagnostischen und therapeutischen Wert. So ist die Verwendung erfindungsgemäßer Oligomere und Derivate davon, gegebenenfalls in Form einer entsprechenden Zusammensetzung, in diagnostischen *in vitro* und *in vivo* Nachweisverfahren Gegenstand der vorliegenden Erfindung.

Zu Amyloid-ß-Protein-assoziierten physiologischen Prozessen gehören solche, die mit Ablagerungen von Amyloidproteinen (Amyloidosen) zu tun haben. Hierzu zählen sowohl solche Prozesse, die zu strukturellen Veränderungen von Nervengewebe führen und beispielsweise bei der Alzheimer-Krankheit und dem Down-Syndrom von Bedeutung sind, als auch solche Prozesse, die andere Gewebe betreffen, wie Amyloid-Microangiopathien, beispielsweise die Congophile Amyloidangiopathie (CAA).

Ein weiterer Gegenstand der vorliegenden Erfindung ist die Verwendung erfindungsgemäßer Oligomere und Derivate davon, gegebenenfalls in Form einer entsprechenden Zusammensetzung, für die Erzeugung von oligomerspezifischen Antikörpern.

Hier sind die erfindungsgemäßen Oligomere auf Basis verkürzter Formen des Aβ(1-42)-Proteins von besonderer Bedeutung: Wegen des fehlenden N-Terminus lässt sich mit ihnen eine deutlich selektivere Immunantwort erzeugen als mit dem Aβ(1-42)-Protein. Während der stark immunogene N-Terminus im klassischen Aβ(1-42)-Protein vornehmlich zu Antikörpern führt, die für diesen Bereich des Moleküls spezifisch sind, z.B. die Antikörper 6E10 (F. Signet) und BAM-10 (Fa. Sigma, St. Louis), die laut Angaben der Hersteller gegen den N-Terminus (6E10: Aβ(1-17) und BAM-10 Aβ(1-12)) gerichtet sind, erkennen die mit den erfindungsgemäßen Oligomeren auf Basis verkürzter Aβ(1-42)-Formen erhältlichen Antikörper oligomerspezifische Bereiche, wodurch eine Selektivität gegenüber anderen Aβ(1-42)-Formen erzielt wird. Dieser Vorteil kann insbesondere für eine aktive Vakzinierung genutzt werden, da die nach Impfung resultierende selektivere Immunantwort (versus APP, Monomerformen, Protofibrillen, Fibrillen, Plaques) auch weniger Nebenwirkungen (z.B. Gehirnblutungen, Beeinträchtigung der physiologischen neurotrophischen Aktivität der in situ Monomer-Form) nach sich zieht. Auch lassen sich für eine passive Immunisierung, d.h. eine Antikörpertherapie, insbesondere monoklonale Antikörper besser herstellen und selektieren.

Ein Aspekt dieser Verwendung ist die Erzeugung von oligomerspezifischen Antikörpern im Rahmen einer Therapie.

Demnach ist ein weiterer Gegenstand der vorliegenden Erfindung die Verwendung eines erfindungsgemäßen Oligomers oder Derivates davon im therapeutischen Bereich, insbesondere als Vakzin.

Ein solches Vakzin stellt in der Regel eine pharmazeutische Zusammensetzung dar, die wenigstens ein erfindungsgemäßes Oligomer und/oder wenigstens ein erfindungsgemäßes Derivat davon enthält. Insbesondere kann hierzu eine der erfindungsgemäßen, zwei oder mehrere Oligomere beinhaltende Zusammensetzungen, oder eine Kombination aus verschiedenen Zusammensetzungen, verwendet werden. So kann insbesondere mit den Oligomeren B, d.h. B1 und B2, oder Derivaten davon, geimpft werden. Ferner kann die Zusammensetzung einen physiologisch verträglichen Träger und gegebenenfalls weitere Hilfsstoffe, beispielsweise Immunstimulantien, enthalten.

Während geeignete Träger im Prinzip beliebig gewählt werden können, richtet sich die Art des Trägers in der Regel nach dem Verabreichungsweg. So können die erfindungsgemäßen Vakzine insbesondere in einer zur parenteralen, beispielsweise intravenösen, intramuskulären und subkutanen Verabreichung geeigneten Form formuliert werden. In diesen Fällen beinhaltet der Träger vorzugsweise Wasser, Kochsalzlösung, Alkohol, ein Fett, ein Wachs und/oder einen Puffer.

Es können beliebige einer Vielzahl von Immunstimulantien in den erfindungsgemäßen Vakzinen verwendet werden. Beispielsweise kann ein Adjuvans mit einbezogen werden. Die meisten Adjuvantien enthalten eine Substanz, die das Antigen vor einer raschen Katabolisierung schützen sollen, wie Aluminiumhydroxid oder ein Mineralöl, sowie ein von Lipid A, Bortadella pertussis oder Mycobacterium tuberculosis abgeleitetes Protein. Geeignete Adjuvantien sind in der Regel kommerziell erhältlich, beispielsweise komplettes oder inkomplettes Freund-Adjuvans; AS-2; Aluminiumsalze, wie Aluminiumhydroxid (gegebenenfalls alas Gel) oder Aluminiumphosphat; Calcium-, Eisen- oder Zinksalze; eine unlösliche Suspension acylierten Tyrosins; acylierte Zucker; kationisch oder anionisch derivatisierte Polysaccharide; Polyphosphazene; biologisch abbaubare Mikrosphären; Monophosphoryl-Lipid A. Cytokine, wie GM-CSF oder Interleukin-2, -7 oder-12 können ebenfalls als Adjuvantien verwendet werden.

Des weiteren betrifft die vorliegende Erfindung ein Verfahren zur Herstellung von Antikörpern, wobei man
i) einen Wirt mit wenigstens einem erfindungsgemäßen Oligomer, Derivat davon oder einer Zusammensetzung immunisiert; und
ii) ein als Antwort auf die Immunisierung gebildetes Antikörper-haltiges Serum des Wirts gewinnt.

Gemäß einer besonderen Ausführungsform des Verfahrens zur Herstellung der Antikörper werden zur Immunisierung Immunisierungscocktails verabreicht, die Gemische verschiedener erfindungsgemäßer Oligomere bzw. Oligomer-Derivate enthalten. Insbesondere kann es zweckmäßig sein, im Zuge des Verfahrens mehrere Immunisierungscocktails zu verabreichen, deren Oligomer-Zusammensetzung sich unterscheidet.

Sind die zu verwendenden Oligomere bzw. Oligomer-Derivate nicht oder nur schwach immunogen, so kann man ihre Immunogenität dadurch erhöhen, daß man sie an Träger, vorzugsweise ein Trägerprotein, wie Keyhole Limpet Hemocyanin (KLH), Limulus Polyphenus Hemocyanin ((LPH), Rinderserumalbumin (BSA) oder Ovalbumin (OVA), koppelt. Dazu stehen dem Fachmann eine Reihe von Kopplungsmöglichkeiten zur Verfügung, die allgemein bekannt sind. Zweckmäßig kann beispielsweise die Umsetzung mit Glutardialdehyd sein, beispielsweise durch Inkubation des Oligomers oder Oligomer-Gemisches mit einem geeigneten Peptid oder Peptid-Gemisch in Wasser oder einem wässrigen Lösungsmittel. Diese Umsetzung kann bequemerweise bei Umgebungstemperatur, also in der Regel Raumtemperatur, durchgeführt werden. Es kann allerdings auch zweckmäßig sein, zu kühlen oder leicht zu erwärmen. In der Regel führt die Umsetzung innerhalb weniger Stunden zum erwünschten Ergebnis, eine Reaktionsdauer von beispielsweise 2 h liegt im üblichen Bereich. Die Glutardialdehyd-Konzentration liegt in der Regel im ppm- bis %-Bereich, zweckmäßigerweise von 10 ppm bis zu 1 %, vorzugsweise von 100 ppm bis 0,5 %. Die Optimierung der Reaktionsparameter liegt im Bereich des fachmännischen Könnens und sollte berücksichtigen, das die Oligomere A und B bzw. Oligomer-Derivate unter den gewählten Reaktionsbedingungen stabil sind.

Zur Herstellung der Immunisierungscocktails werden die zu verwendenden Komponenten zunächst zusammengegeben. Es ist von Vorteil, das resultierende Komponenten-Gemisch zunächst zu inkubieren. Dies erfolgt bequemerweise bei Umgebungstemperatur, in der Regel also bei Raumtemperatur. Es kann allerdings zweckmäßig sein, das Gemisch zu kühlen oder leicht zu erwärmen. Die Inkubationsdauer beträgt in der Regel einige Minuten bis wenige Stunden, eine Inkubationszeit von 1 h hat sich als vorteilhaft erwiesen.

Zusätzlich zum Antigen enthalten Immunisierungscocktails in der Regel weitere Hilfsstoffe, insbesondere üblicherweise zur Immunisierung eingesetzte Adjuvantien, z.B. Freund-Adjuvanz. Insbesondere verwendet man komplettes Freund-Adjuvanz für die erste Immunisierung wohingegen alle weiteren Immunisierungen mit inkomplettem Freund-Adjuvanz durchgeführt werden. Zur Herstellung der Immunisierungscocktails wird das Antigen (Immunogen), vorzugsweise als oben beschriebenes Komponenten-Gemisch, zu dem oder den Hilfsstoffen gegeben. In der Regel wird dabei das Antigen emulgiert.

Als Wirt eignen sich insbesondere Nager oder auch Kaninchen. Diesen oder anderen geeigneten Wirten werden die Immunisierungscocktails, vorzugsweise subkutan, injiziert. Die Antikörpertiter können mit einem Immunoassay, beispielsweise kompetitiv mit einem gegen Wirt-IgG gerichteten Schaf-Antiserum und markiertem Oligomer, bestimmt werden. So kann gegen Ende der Immunisierung entschieden werden, ob ein bestimmter Wirt zur Antikörpergewinnung geeignet ist. Werden beispielsweise vier Immunisierungen durchgeführt, so kann man nach der dritten Immunisierung den Antikörpertiter bestimmen und dann aus Tieren, die einen ausreichenden Antikörpertiter aufweisen, Antikörper gewinnen.

Zur Gewinnung gebildeter Antikörper ist es bevorzugt, den Wirten über mehrere Wochen oder Monate Blut abzunehmen. Abschließend kann man den Wirt ausbluten lassen. Aus dem gewonnen Blut kann in an sich bekannter Weise Serum gewonnen werden, das die erwünschten Antikörper enthält. Das so erhaltene Vollserum kann erforderlichenfalls in fachmännischer Weise weiter aufgereinigt werden, um die darin enthaltene Antikörperfraktion und insbesondere die Oligomer-erkennenden Antikörper anzureichern.

Gemäß einer besonderen Ausführungsform dieses Verfahrens selektiert man wenigstens einen Antikörper des Serums, der das als Immunogen verwendete Oligomer, ein Derivat davon oder wenigstens ein in der als Immunogen verwendeten Zusammensetzung enthaltenes Oligomer oder Derivat davon spezifisch erkennt. Spezifität meint in diesem Zusammenhang eine höhere Bindungsaffinität des Antikörpers für das Immunogen als für andere, insbesondere verwandte Proteine, vor allem im Vergleich zu monomerem Amyloid-β(1-42)-Protein sowie oligomeren oder multimeren Amyloid-β(1-42)-Protein-Aggregaten, die ein höheres Molekulargewicht aufweisen als die erfindungsgemäßen Oligomere. Auch monoklonale oligomerspezifische Antikörper können auf diese Art gewonnen werden. Hierzu ist es allerdings bevorzugt, den Wirten Milzgewebe zu entnehmen und ausgehend von den so gewonnenen Milzlymphocyten auf übliche Art und Weise Hybridome zu etablieren, welche die monoklonalen Antikörper produzieren.

### Detailierte Beschreibung der Antikörperherstellung

Teil des Immunsystem eines Säugers sind B-Lymphozyten, die als Gesamtheit ein aus Hunderten von Millionen unterschiedlicher Antikörperspezifitäten zusammengesetztes Antikörper-Repertoire beinhalten. Eine normale Immunantwort gegen ein bestimmtes Antigen bedeutet die Selektion eines oder mehrerer, das Antigen spezifisch bindender Antikörper aus diesem Repertoire, und der Erfolg einer Immunantwort fußt zumindest zum Teil auf der Fähigkeit dieser Antikörper, das stimullerende Antigen spezifisch zu erkennen (und letztendlich zu eliminieren) und dabei andere Moleküle aus der Umgebung der Antikörper zu ignorieren.

Die Nützlichkeit von Antikörpern, die ein bestimmtes Zielantigen spezifisch erkennen, hat zur Entwicklung der monoklonalen Antikörpertechnologie geführt. Standardisierte Hybridomtechnologie gestattet nun die Herstellung von Antikörpern mit einer einzigen Spezifität für ein interessierendes Antigen. In neuerer Zeit sind rekombinante Antikörpertechniken, wie das in-vitro-Screenen von Antikörperbanken entwickelt worden. Mit diesen Techniken lassen sich ebenfalls Antikörper mit einer einzigen Spezifität für ein interessierendes Antigen herstellen.

In dem erfindungsgemäßen Verfahren kann man das interessierende Antigen entweder *in vivo* oder *in vitro* auf das Antikörper-Repertoire einwirken lassen.

Einer Ausführungsform zufolge lässt man das Antigen auf das Repertoire einwirken, indem man ein Tier *in vivo* mit dem Antigen immunisiert. Dieser in-vivo-Ansatz kann des weiteren beinhalten, dass man aus den Lymphozyten eines Tieres eine Reihe von Hybridomen etabliert und ein Hybridom selektiert, das einen das Antigen spezifisch bindenden Antikörper sekretiert. Bei dem zu immunisierenden Tier kann es sich beispielsweise um eine Maus, Ratte, Kaninchen, Huhn, Kamelid oder Schaf handeln, oder um eine transgene Version eines der zuvor genannten Tiere, beispielsweise eine transgene Maus mit humanen Immunglobulingenen, die nach einem antigenen Stimulus humane Antikörper macht. Zu weiteren Typen von Tieren, die immunisiert werden können, gehören Mäuse mit schwerer kombinierter Immundefizienz (SCID), die mit humanen peripheren mononukleären Blutzellen (chimäre hu-PBMC-SCID-Mäuse) oder mit lymphoiden Zellen oder Vorläufern davon rekonstituiert worden sind, genauso wie Mäuse, die mit einer letalen Gesamtkörperbestrahlung behandelt, anschließend mit Knochenmarkszellen aus einer Maus mit schwerer kombinierter Immundefizienz (SCID) gegen Strahlung geschützt und anschließend mit funktionalen humanen Lymphozyten transplantiert worden sind (das sogenannte Trimera-System). Eine weiterer Typus eines zu immunisierenden Tieres ist ein Tier (z.B. eine Maus), in dessen Genom ein endogenes Gen, welches das interessierende Antigen kodiert, ausgeschaltet wurde ("knocked out"), z.B. durch homologe Rekombination, so dass dieses Tier nach Immunisierung mit dem Antigen das Antigen als fremd erkennt. Für den Fachmann ist klar, dass die mit diesen Verfahren hergestellten polyklonalen oder monoklonalen Antikörper charakterisiert und selektiert werden, indem man bekannte Screening-Verfahren verwendet, zu denen ELISA-Techniken gehören, ohne jedoch darauf beschränkt zu sein.

Einer weiteren Ausführungsform zufolge, lässt man das Antigen auf das Antikörper-Repertoire in vitro einwirken, indem man eine rekombinante Antikörperbank mit dem Antigen screent. Die rekombinante Antikörperbank kann beispielsweise auf der Oberfläche von Bakteriophagen oder auf der Oberfläche von Hefezellen oder auf der Oberfläche von bakteriellen Zellen exprimiert sein. In mannigfaltigen Ausführungsformen ist die rekombinante Antikörperbank beispielsweise eine scFv-Bank oder eine Fab-Bank. Gemäß einer weiteren Ausführungsform sind Antikörperbänke als RNA-Protein-Fusionen exprimiert.

Ein weiterer Ansatz zur Herstellung erfindungsgemäßer Antikörper beinhaltet eine Kombination aus *in vivo-* und *in vitro*-Ansätzen. Beispielsweise kann man das Antigen auf das Antikörper-Repertoire einwirken lassen, indem man ein Tier mit dem Antigen *in vivo* immunisiert und anschließend eine aus lymphoiden Zellen des Tieres hergestellte rekombinante Antikörperbank oder Einzeldomänen-Antikörperbank (z.B. mit schwerer und/oder leichter Kette) mit dem Antigen *in vitro* screent. Einem weiteren Ansatz zufolge lässt man das Antigen auf das Antikörper-Repertoire einwirken, indem man ein Tier mit dem Antigen *in vivo* immunisiert und anschließend eine aus lymphoiden Zellen des Tieres hergestellte rekombinante Antikörperbank oder Einzeldomänen-Bank einer Affinitätsreifung unterzieht. Einem weiteren Ansatz zufolge lässt man das Antigen auf das Antikörper-Repertoire einwirken, indem man ein Tier mit dem Antigen *in vivo* immunisiert, anschließend einzelne Antikörperproduzierende Zellen, die einen interessierenden Antikörper sekretieren, selektiert und aus diesen selektierten Zellen cDNAs für die variable Region der schweren und leichten Kette gewinnt (z.B. mittels PCR) und die variablen Regionen der schweren und leichten Kette *in vitro* in Säugerwirtszellen exprimiert (was als das Lymphozyten-Antikörper-Selektionsverfahren, oder SLAM für "Selected Lymphocyte Antibody Method", bezeichnet wird), wodurch sich die selektierten Antikörper-Gensequenzen weiter selektieren und manipulieren lassen. Außerdem können monoklonale Antikörper durch Expressionsklonierung selektiert werden, indem man die Antikörpergene für die schwere und leichte Kette in Säugerzellen exprimiert und diejenigen Säugerzellen selektiert, die einen Antikörper mit der gewünschten Bindungsaffinität sekretieren.

Demnach ist es ein Aspekt der vorliegenden Erfindung, definierte Antigene zum Screenen und Gegenscreenen zur Verfügung zu stellen. So können erfindungsgemäß diejenigen polyklonalen und monoklonalen Antikörper selektiert werden, die ein erfindungsgemäßes Oligomer oder Derivat davon, nicht aber andere Formen des A (1-42)-Proteins, APP, amyloide Fibrillen oder amyloide Plaques und eine Reihe weiterer nicht verwandter Antigene und Gewebe binden.

Dem Fachmann ist hinlänglich bekannt, dass Antikörperselektionen auf gut definierten Antigenen aufbauen. Werden hingegen weniger gut definierte Antigene verwendet, so sind sie nicht selektiv genug. Kurzum, das Einwirken und Selektieren in vitro ähnelt der Affinitätschromatographie, wobei "Liganden" für das gewünschte Antigen von denjenigen abgetrennt werden, die das Antigen nicht mit ausreichender Affinität binden. Der Anreicherungsgrad der gewünschten Antikörper aus dem riesigen Pool anderer Antikörper ist daher eine direkte Folge der Antigenqualität. Überraschenderweise stellen die erfindungsgemäßen Oligomere und Derivate davon Antigene dar, mit denen sich geeignete, relevante und selektive Antikörper anreichern und von Antikörpern, die andere mit dem A (1-42)-Protein in Zusammenhang stehende Formen sowie weitere nicht verwandte Antigene erkennen, effektiv abtrennen lassen.

Mit den erfindungsgemäßen Verfahren zur Herstellung von Antikörpern lassen sich verschiedene Antikörpertypen herstellen. Hierzu gehören im Wesentlichen humane Antikörper, chimäre Antikörper, humanisierte Antikörper und CDR-Graft-Antikörper sowie Antigen-bindende Teile davon.

Im Folgenden werden Verfahren zur Herstellung erfindungsgemäßer Antikörper beschrieben. Dabei wird zwischen In-vivo-Ansätzen, In-vitro-Ansätzen, oder einer Kombination aus beiden, unterschieden.

### In-vivo-Ansätze

Ausgehend von den in-vivo erzeugten Antikörper produzierenden Zellen können monoklonale Antikörper mittels standardisierter Techniken, wie der ursprünglich von Kohler und Milstein (1975, Nature 256:495-497) (siehe auch Brown et al. (1981) J. Immuno/ 127:539-46; Brown et al. (1980) J Biol Chem 255:4980-83; Yeh et al. (1976) PNAS 76:2927-31; und Yeh et al. (1982) Int. J. Cancer 29:269-75) beschriebenen Hybridomtechnik, hergestellt werden. Die Technologie zur Produktion monoklonaler Antikörperhybridome ist hinlänglich bekannt (siehe allgemein R. H. Kenneth, in Monoc/onal Antibodies: A New Dimension In Biological Analyses, Plenum Publishing Corp., New York, New York (1980); E. A. Lerner (1981) Yale J. Biol. Med., 54:387-402; M. L. Gefter et al. (1977) Somatic Cell Genet., 3:231-36). Kurzum, eine immortalisierte Zeillinie (typischerweise ein Myelom) wird mit Lymphozyten (typischerweise Splenozyten oder Lymphknotenzellen oder periphere Blutlymphozyten) eines mit dem erfindungsgemäßen Oligomer oder Derivat davon immunisierten Säugers fusioniert, und die Kulturüberstände der resultierenden Hybridomzellen werden gescreent, um ein Hybridom zu identifizieren, das einen monoklonalen Antikörper mit Spezifität für erfindungsgemäßes Oligomer oder für ein Derivat davon produziert. Dazu kann man ein beliebiges von vielen hinlänglich bekannten Protokollen für die Fusion von Lymphozyten und immortalisierten Zelllinien anwenden (siehe auch G. Galfre et al. (1977) Nature 266:550-52; Gefter et al. Somatic Cell Genet.*,* cited *supra;* Lerner, Yale J. Biol. Med.*,* cited *supra;* Kenneth, Monoclonal Antibodies*,* cited *supra*)*.* Darüber hinaus sind dem Fachmann mannigfaltige Variationen solcher Verfahren, die ebenfalls brauchbar sind, bekannt. Typischerweise stammt die immortalisierte Zelllinie (z.B. eine Myelomzelllinie) von der gleichen Säugerspezies ab wie die Lymphozyten. Beispielsweise kann man murine Hybridome etablieren, indem man Lymphozyten aus einer mit einer erfindungsgemäßen immunogenen Zubereitung immunisierten Maus mit einer immortalisierten Mauszelllinie fusioniert. Bevorzugte immortalisierte Zelllinien sind Maus-Myelomzelllinien, welche für Hypoxanthin, Aminopterin und Thymidin enthaltendes Kulturmedium (HAT-Medium) sensitiv sind. Man kann eine beliebige von vielen Myelomzelllinien als Fusionspartner standardmäßig verwenden, z.B. die P3-NS1/1-Ag4-1-, P3-x63-Ag8.653- or Sp2/O-Ag14-Myelomlinie. Diese Myelomzelllinien sind von der American Type Culture Collection (ATCC), Rockville, MD, erhältlich. Typischerweise werden HAT-sensitive Maus-Myelomzellen mit Maus-Splenozyten unter Verwendung von Polyethylenglykol (PEG) fusioniert. Die aus der Fusion resultierenden Hybridomzellen werden dann unter Verwendung von HAT-Medium selektiert, wodurch nicht fusionierte und nicht produktiv fusionierte Myelomzellen abgetötet werden (nicht fusionierte Splenozyten sterben nach mehreren Tagen ab, da sie nicht transformiert sind). Monoklonale Antikörperproduzierende Hybridomzellen, die erfindungsgemäßes Oligomer oder ein Derivat davon spezifisch erkennen, werden identifiziert, indem man die Hybridomkulturüberstände auf solche Antikörper screent, z.B. indem man einen Standard-ELISA-Assay verwendet, um diejenigen Antikörper zu selektieren, die erfindungsgemäßes Oligomer oder ein Derivat davon spezifisch binden können.

Je nach Art des gewünschten Antikörpers, können verschiedene Wirtstiere für die in-vivo-Immunisierung verwendet werden. Ein Wirt, der eine endogene Version des interessierenden Antigens selbst exprimiert, kann verwendet werden. Alternativ kann ein Wirt verwendet werden, der für eine endogene Version des interessierenden Antigens defizient gemacht wurde. Es wurde beispielsweise gezeigt, dass Mäuse, die über homologe Rekombination am entsprechenden endogenen Gen für ein bestimmtes endogenes Protein defizient gemacht wurden (d.h. Knockout-Mäuse) eine humorale Antwort gegen das Protein, mit dem sie immunisiert wurden, erzeugen und daher zur Herstellung hochaffiner monoklonaler Antikörper gegen das Protein verwendet werden können (siehe z.B. Roes, J. et al. (1995) J. Immunol. Methods 183:231-237; Lunn, M.P. et al. (2000) J. Neurochem. 75:404-412).

Für die Herstellung nicht humaner Antikörper gegen erfindungsgemäßes Oligomer oder ein Derivat davon sind viele nicht-menschliche Säuger als Wirte für die Antikörperherstellung geeignet. Hierzu gehören Mäuse, Ratten, Hühner, Kamelide, Kaninchen und Ziegen (und Knockout-Versionen davon), wenngleich Mäuse zur Hybridomherstellung bevorzugt sind. Des weiteren kann man zur Herstellung im Wesentlichen humaner Antikörper gegen ein humanes Antigen mit dualer Spezifität ein nicht-humanes Wirtstier verwenden, das ein humanes Antikörper-Repertoire exprimiert. Zu solchen nicht-humanen Tieren gehören transgene Tiere (z.B. Mäuse), die humane Immunglobulin-Transgene tragen (chimäre hu-PBMC-SCID-Mäuse) und Mensch/Maus-Bestrahlungschimären, die nachstehend eingehender beschrieben sind.

Einer Ausführungsform zufolge ist das Tier, das mit einem erfindungsgemäßen Oligomer oder Derivat davon immunisiert wird, ein nicht-humaner Säuger, vorzugsweise eine Maus, der durch humane Immunglobulingene transgen ist, so dass der nicht-humane Säuger nach einem antigenen Stimulus humane Antigkörper macht. Typischerweise werden in solche Tiere Immunglobulin-Transgene für schwere und leichte Kette mit humaner Keimbahnkonfiguration eingeführt, wobei die Tiere so verändert wurden, dass ihre endogenen Loki für schwere und leichte Kette inaktiv sind. Stimuliert man solche Tiere mit Antigen (z.B. mit einem humanen Antigen), werden Antikörper, die von den humanen Immunglobulinsequenzen abstammen (d.h. humane Antikörper), produziert. Aus den Lymphozyten solcher Tiere können mittels standardisierter Hypridomtechnologie humane monoklonale Antikörper gemacht werden. Zur weiteren Beschreibung transgener Mäuse mit humanen Immunglobulinen und ihre Verwendung bei der Herstellung humaner Antikörper siehe beispielsweise US-Patent Nr. 5,939,598, WO 96/33735, WO 96/34096, WO 98/24893 und WO 99/53049 (Abgenix Inc.), und US-Patent Nr. 5,545,806, Nr. 5,569,825, Nr. 5,625, 126, Nr. 5,633, 425, Nr. 5,661,016, Nr. 5,770,429, Nr. 5,814,318, Nr. 5,877,397 und WO 99/45962 (Genpharm Inc.); siehe ebenfalls MacQuitty, J.J. und Kay, R.M. (1992) Science 257:1188; Taylor, L.D. et al. (1992) Nucleic Acids Res. 20:6287-6295; Lonberg, N. et al. (1994) Nature 368:856-859; Lonberg, N. und Huszar, D. (1995) Int. Rev. Immunol. 13:65-93; Harding, F.A. und Lonberg, N. (1995) Ann. N.Y. Acad. Sci. 764:536-546; Fishwild, D. M. et al. (1996) Nature Biotechnology 14:845-851; Mendez, M. J. et al. (1997) Nature Genetics 15:146-156; Green, L.L. und Jakobovits, A. (1998) J. Exp. Med. 188:483-495; Green, L.L. (1999) J. Immunol. Methods 231:11-23; Yang, X.D. et al. (1999) J. Leukoc. Biol. 66:401-410; Gallo, M.L. et al.(2000) Eur. J. Immunol. 30:534-540.

Gemäß einer weiteren Ausführungsform kann das Tier, das mit erfindungsgemäßem Oligomer oder einem Derivat davon immunisiert wird, eine Maus mit schwerer kombinierter Immundefizienz (SCID) sein, die mit humanen peripheren mononukleären Blutzellen oder lymphoiden Zellen oder Vorläufern davon rekonstituiert wurde. Solche Mäuse, die als chimäre hu-PBMC-SCID-Mäuse bezeichnet werden, produzieren nachgewiesenermaßen humane Immunglobulinantworten nach einem antigenen Stimulus. Zur weiteren Beschreibung dieser Mäuse und ihrer Verwendung für die Erzeugung von Antikörpern siehe beispielsweise Leader, K.A. et al. (1992) Immunology 76:229-234; Bombil, F. et al. (1996) Immunobiol. 195:360-375; Murphy, W.J. et al. (1996) Semin. Immunol. 8:233-241; Herz, U. et al. (1997) Int. Arch. Allergy Immunol. 113:150-152; Albert, S.E. et al. (1997) J. Immunol. 159:1393-1403; Nguyen, H. et al. (1997) Microbiol. Immunol. 41:901-907; Arai, K. et al. (1998) J. ImmunoL Methods 217:79-85; Yoshinari, K. und Arai, K. (1998) Hybridoma 17:41-45; Hutchins, W.A. et al. (1999) Hybridoma 18:121-129; Murphy, W.J. et al. (1999) Clin. Immunol. 90:22-27; Smithson, S.L. et al. (1999) Mol. Immunol. 36:113-124; Chamat, S. et al. (1999) J. Infect. Diseases 180:268-277; und Heard, C. et al. (1999) Molec. Med. 5:35-45.

Gemäß einer weiteren Ausführungsform ist das Tier, das mit erfindungsgemäßem Oligomer oder einem Derivat davon immunisiert wird, eine Maus, die mit einer letalen Ganzkörperbestrahlung behandelt, anschließend mit Knochenmarkszellen aus Mäusen mit schwerer kombinierter Immundefizienz (SCID) gegen Strahlung geschützt, und anschließend mit funktionalen humanen Lymphozyten transplantiert worden ist. Dieser als das Trimera-System bezeichnete Chimärentyp wird verwendet, um humane monoklonale Antikörper herzustellen, indem man die Mäuse mit dem interessierenden Antigen immunisiert und anschließend monoklonale Antikörper unter Verwendung von standardisierter Hybridomtechnologie herstellt. Zur weiteren Beschreibung dieser Mäuse und ihrer Verwendung für die Erzeugung von Antikörpern siehe beispielsweise Eren, R. et al. (1998) Immunology 93:154-161; Reisner, Y und Dagan, S. (1998) Trends Biotechnol. 16:242-246; Ilan, E. et al. (1999) Hepatology 29:553-562; und Bocher, W.O. et al. (1999) Immunology 96:634-641.

### in-vitro-Ansätze

Alternativ zur Herstellung erfindungsgemäßer Antikörper durch Immunisierung und Selektion können erfindungsgemäße Antikörper identifiziert und isoliert werden, indem man rekombinante kombinatorische Immunglobulin-Bänke (Library)) mit einem erfindungsgemäßen Oligomer oder Derivat davon screent, um so Mitglieder der Immunogiobulin-Bank, die spezifisch an das Oligomer oder Derivat davon binden, zu isolieren. Kits zur Erzeugung und zum Screenen von Display-Banken sind kommerziell erhältlich (z.B. das Recombinant Phage Antibody System von Pharmacia, Katalog-Nr. 27-9400-01; und der SurfZAP® Phage Display Kit von Stratagene, Katalog-Nr. 240612). In vielen Ausführungsformen ist die Display-Bank eine scFv-Bank oder eine Fab-Bank. Die Phagendisplay-Technik zum Screenen rekombinanter Antikörperbänke ist hinlänglich beschrieben worden. Beispiele für Verfahren und Verbindungen, die bei der Erzeugung und dem Screenen von Antikörper-Display-Banken besonders vorteilhaft verwendet werden können, sind beispielsweise in McCafferty et al. WO 92/01047, US-Patent Nr. 5,969,108 und EP 589 877 (beschreibt insbesondere den Display von scFv), Ladner et al. US-Patent Nr. 5,223,409, Nr. 5,403,484, Nr. 5,571,698, Nr. 5,837,500 und EP 436 597 (beschreibt beispielsweise die pIII*-Fu*sion); Dower et al. WO 91/17271, US-Patent Nr. 5,427,908, US-Patent Nr. 5,580,717 und EP 527 839 (beschreibt insbesondere den Display von Fab); Winter *et al.* International Publication WO 92/20791 und EP 368,684 (beschreibt insbesondere die Klonierung von Sequenzen für variable Immunoglobulindomänen); Griffiths et al. US-Patent Nr. 5,885,793 und EP 589 877 (beschreibt insbesondere die Isolierung von humanen Antikörpern gegen humane Antigene unter Verwendung rekombinanter Bänke); Garrard et al. WO 92/09690 (beschreibt insbesondere Phagenexpressionstechniken); Knappik et al. WO 97/08320 (beschreibt die humane rekombinante Antikörperbank HuCal); Salfeld et al. WO 97/29131, (beschreibt die Herstellung eines rekombinanten humanen Antikörpers gegen ein humanes Antigen (humanen Tumor-Nekrose-Faktor alpha), sowie in-vitro-Affinitätsreifung des rekombinanten Antikörpers) und Salfeld et al. U.S. Provisional Application Nr. 60/126,603 und die hierauf basierenden Patentanmeldungen (beschreibt ebenfalls die Herstellung rekombinanter humaner Antikörper gegen humanes Antigen (humanes Interleukin-12), sowie die *in-vitro-*Affinitätsreifung des rekombinanten Antikörpers) zu finden.

Weitere Beschreibungen von Screenings rekombinanter Antikörperbänke sind in wissenschaftlichen Publikationen zu finden, wie Fuchs et al. (1991) Bio/Technology 9:1370-1372; Hay et al. (1992) Hum Antibod Hybridomas 3:81-85; Huse et al. (1989) Science 246:1275-1281; Griffiths et al. (1993) EMBO J 12:725-734; Hawkins et al. (1992) J Mol Biol 226:889-896; Clarkson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; Barbas et al. (1991) PNAS 88:7978-7982; McCafferty et al. Nature (1990) 348:552-554; und Knappik et al. (2000) J. Mol. Biol. 296:57-86.

Alternativ zur Verwendung von Bakteriophagen-Display-Systemen können rekombinante Antikörperbänke auf der Oberfläche von Hefezellen oder bakteriellen Zellen exprimiert werden. Verfahren zur Herstellung und zum Screenen von Bänken, die auf der Oberfläche von Hefezellen exprimiert sind, sind in WO 99/36569 beschrieben. Verfahren zur Herstellung und zum Screenen von Bänken, die auf der Oberfläche von bakteriellen Zellen exprimiert sind, sind in WO 98/49286 genauer beschrieben.

Sobald ein interessierender Antikörper aus einer kombinatorischen Bank identifiziert ist, werden die DNAs, welche die leichten und schweren Ketten des Antikörpers kodieren, mittels standardisierter molekularbiologischer Techniken isoliert, beispielsweise mittels PCR-Amplifikation von DNA aus der Display-Packung (z.B. dem Phagen), die man während des Screenens der Bank isoliert hat. Nukleotidsequenzen von Genen für leichte und schwere Antikörperketten, mit denen PCR-Primer hergestellt werden können, sind dem Fachmann bekannt. Viele solcher Sequenzen sind beispielsweise in Kabat, E.A., et al. (1991) Sequences of Proteins of Immunological Interest, Fifth Edition, U.S. Department of Health und Human Services, NIH Publication Nr. 91-3242 und der Datenbank für Sequenzen der Humankeimbahn VBASE beschrieben.

Ein erfindungsgemäßer Antikörper oder Antikörperteil kann hergestellt werden, indem man die Gene für leichte und schwere Immunglobulinketten in einer Wirtszelle rekombinant exprimiert. Um einen Antikörper rekombinant zu exprimieren, wird eine Wirtszelle mit einem oder mehreren rekombinanten Expressionsvektoren, die DNA-Fragmente tragen, welche die leichten und schweren Immunglobulinketten des Antikörpers kodieren, transfiziert, so dass die leichten und schweren Ketten in der Wirtszelle exprimiert und vorzugsweise in das Medium, in dem die Wirtszellen kultiviert werden, sekretiert werden. Aus diesem Medium können die Antikörper gewonnen werden. Man verwendet standardisierte rekombinante DNA-Methodik, um Gene für schwere und leichte Antikörperketten zu erhalten, diese Gene in rekombinante Expressionsvektoren einzusetzen und die Vektoren in Wirtszellen einzuführen. Eine derartige Methodik ist beispielsweise in Sambrook, Fritsch und Maniatis (Hrsg.), Molecular Cloning; A Laboratory Manual, Second Edition, Cold Spring Harbor, N.Y., (1989), Ausubel, F.M. et al. (Hrsg.) Current Protocols in Molecular Biology, Greene Publishing Associates, (1989) und im US-Patent Nr. 4,816,397 von Boss et al. beschrieben.

Sobald DNA-Fragmente, die VH- und VL-Segmente des interessierenden Antikörpers kodieren, erhalten werden, können diese DNA-Fragmente mit standardisierten rekombinanten DNA-Techniken weiter manipuliert werden, beispielsweise um die Gene für variable Regionen in Gene für Antikörperketten voller Länge, in Gene für Fab-Fragmente oder in ein scFv-Gen zu überführen. Bei diesen Manipulationen wird ein VL- oder VH-kodierendes DNA-Fragment operativ mit einem weiteren ein weiteres Protein, z.B. eine konstante Antikörperregion oder einen flexiblen Linker, kodierenden DNA-Fragment verknüpft. Der Begriff "operativ verknüpft" soll hier meinen, dass die beiden DNA-Fragmente so miteinander verbunden sind, dass die von den beiden DNA-Fragmenten kodierten Aminosäuresequenzen im Leseraster (in-frame) bleiben.

Die isolierte, die VH-Region kodierende DNA kann in ein Gen für eine schwere Kette voller Länge überführt werden, indem man die VH-Region kodierende DNA mit einem weiteren, konstante Regionen der schweren Kette (CH1, CH2 und CH3) kodierenden DNA-Molekül operativ verknüpft. Die Sequenzen von Genen für konstante Regionen humaner schwerer Ketten sind hinlänglich bekannt (siehe z.B. Kabat, E.A., et al. (1991) Sequences of Proteins of Immunological Interest, Fifth Edition, U.S. Department of Health und Human Services, NIH Publication Nr. 91-3242), und DNA-Fragmente, die diese Regionen überspannen, können mittels standardisierter PCR-Amplifikation erhalten werden. Die konstante Region der schweren Kette kann eine konstante Region aus IgG1, IgG2, IgG3, IgG4, IgA, IgE, IgM or IgD sein, wobei eine konstante Region aus IgG1 oder IgG4 bevorzugt ist. Um ein Gen für ein Fab-Fragment der schweren Kette zu ehalten, kann die VHkodierende DNA mit einem weiteren, lediglich die konstante Region CH1 der schweren Kette kodierenden DNA-Molekül operativ verknüpft werden.

Die isolierte, die VL-Region kodierende DNA kann in ein Gen für eine leichte Kette voller Länge (sowie ein Gen für eine Fab-leichte Kette) überführt werden, indem man die VL-kodierende DNA mit einem weiteren, die konstante Region CL der leichten Kette kodierenden DNA-Molekül operativ verknüpft. Die Sequenzen von Genen der konstanten Region humaner leichter Kette sind hinlänglich bekannt (siehe Kabat, E.A., et al. (1991) Sequences of Proteins of Immunological Interest, Fifth Edition, U.S. Department of Health und Human Services, NIH Publication Nr. 91-3242), und DNA-Fragmente, die diese Regionen überspannen, können mittels standardisierter PCR-Amplifikation erhalten werden. Die konstante Region der leichten Kette kann eine konstante kappa- oder lambda-Region sein, wobei eine konstante kappa-Region bevorzugt ist.

Um ein scFv-Gen zu erzeugen, können die VH- und VL-kodierenden DNA-Fragmente mit einem weiteren, einen flexiblen Linker, z.B. die Aminosäuresequenz (Gly₄-Ser)₃ kodierenden Fragment operativ verknüpft werden, so dass die VH- und VL-Sequenzen als fortlaufendes einzelkettiges Protein exprimiert werden, wobei die VL- und VH-Regionen über den flexiblen Linker miteinander verbunden sind (siehe Bird et al. (1988) Science 242:423-426; Huston et al. (1988) Proc. Natl. Acad. Sci. USA 85:5879-5883; McCafferty et al., Nature (1990) 348:552-554).

VH- und VL-Einzeldomänen mit Spezifität für erfindungsgemäßes Oligomer oder ein Derivat davon können mit den oben beschriebenen Verfahren aus Einzeldomänen-Banken isoliert werden. Zwei VH-Einzeldomänen-Ketten (mit oder ohne CH1) oder zwei VL-Ketten oder ein Paar aus einer VH- und einer VL-Kette mit der gewünschten Spezifität können verwendet werden, um erfindungsgemäße Oligomere oder Derivate davon aus dem Körper zu entfernen.

Um die erfindungsgemäßen rekombinanten Antikörper oder Antikörperteile zu exprimieren, können die DNAs, welche die leichten und schweren Ketten von partieller oder voller Länge kodieren, in Expressionsvektoren insertiert werden, so das die Gene mit transkriptionalen und translationalen Kontrollsequenzen operativ verknüpft sind. In diesem Zusammenhang soll der Begriff "operativ verknüpft" meinen, dass ein Antikörpergen in einem Vektor so ligiert ist, dass transkriptionale und translationale Kontrollsequenzen innerhalb des Vektors ihre beabsichtigte Funktion zur Regulation der Transkription und Translation des Antikörpergens erfüllen.

Der Expressionsvektor und die Expressionskontrollsequenzen werden so gewählt, dass sie mit der verwendeten Expressionswirtszelle kompatibel sind. Das Gen für die leichte Antikörperkette und das Gen für die schwere Antikörperkette können in getrennte Vektoren insertiert werden, oder beide Gene werden in denselben Expressionsvektor insertiert, was der Regelfall ist. Die Antikörpergene werden in den Expressionsvektor mittels standardisierter Verfahren insertiert (z.B. Ligation komplementärer Restriktionsschnittstellen am Antikörpergenfragment und Vektor, oder Ligation stumpfer Enden, falls keine Restriktionsschnittstellen vorhanden sind). Vor der Insertion der Sequenzen für die leichte und schwere Kette kann der Expressionsvektor bereits Sequenzen für konstante Antikörperregionen tragen. Beispielsweise ist es ein Ansatz, die VH- und VL-Sequenzen in Antikörpergene voller Länge zu überführen, indem man sie in Expressionsvektoren insertiert, die bereits die konstanten Regionen für schwere bzw. leichte Kette kodieren, so dass das VH-Segment mit dem oder den CH-Segment(en) innerhalb des Vektors operativ verknüpft ist, und auch das VL-Segment mit dem CL-Segment innerhalb des Vektors operativ verknüpft ist. Zusätzlich oder alternativ kann der rekombinante Expressionsvektor ein Signalpeptid kodieren, welches die Sekretion der Antikörperkette aus der Wirtszelle erleichtert. Das Gen für die Antikörperkette kann in den Vektor kloniert werden, so dass das Signalpeptid in Leseraster mit dem N-Terminus des Gens für die Antikörperkette verknüpft ist. Das Signalpeptid kann ein Immunglobulin-Signalpeptid oder ein heterologes Signalpeptid sein (d.h. ein Signalpeptid aus einem Nicht-Immunogiobulin-Protein). Zusätzlich zu den Genen für die Antikörperkette können die erfindungsgemäßen Expressionsvektoren regulatorische Sequenzen aufweisen, welche die Expression der Gene für die Antikörperkette in einer Wirtszelle kontrollieren. Der Begriff "regulatorische Sequenz" soll Promotoren, Enhancer und weitere Expressionskontrollelemente (z.B. Polyadenylierungssignale) einschließen, welche die Transkription oder Translation der Gene für die Antikörperkette kontrollieren. Solche regulatorischen Sequenzen sind beispielsweise in Goeddel; Gene Expression Technology: Methods in Enzymology 185, Academic Press, San Diego, CA (1990) beschrieben. Dem Fachmann ist bekannt, dass das Design des Expressionsvektors, wozu die Selektion regulatorischer Sequenzen gehört, von Faktoren abhängen kann, wie der Wahl der zu transformierenden Wirtszelle, der gewünschten Expressionsstärke des Proteins, etc. Zu bevorzugten regulatorischen Sequenzen für eine Expression in Säugerwirtszellen gehören virale Elemente, die zu einer starken Proteinexpression in Säugerzellen führen, wie Promotoren und/oder Enhancer, die vom Cytomegalovirus (CMV) (wie der CMV-Promoter/Enhancer), Simian-Virus 40 (SV40) (wie der SV40-Promotor/Enhancer), Adenovirus (z.B. der Späte Adenovirus-Hauptpromotor (AdMLP für Adenovirus Major Late Promoter) und Polyoma abstammen. Zur weiteren Beschreibung viraler regulatorischer Elemente und Sequenzen davon, siehe z.B. US-Patent Nr. 5,168,062 von Stinski, US-Patent Nr. 4,510,245 von Bell et al. und US-Patent Nr. 4,968,615 von Schaffner et al.

Zusätzlich zu den Genen für die Antikörperkette und die regulatorischen Sequenzen können die erfindungsgemäßen rekombinanten Expressionsvektoren zusätzliche Sequenzen aufweisen, wie Sequenzen, welche die Replikation des Vektors in Wirtszellen regulieren (z.B. Replikationsstartpunkte) und selektierbare Markergene. Die selektierbaren Markergene erleichtern die Selektion von Wirtszellen, in die der Vektor eingeführt wurde (siehe z.B. US-Patents Nr. 4,399,216, 4,634,665 und 5,179,017, alle von Axel et al.)*.* Zum Beispiel ist es üblich, dass das selektierbare Markergen eine Wirtszelle, in die der Vektor eingesetzt wurde, gegen Wirkstoffe wie G418, Hygromycin oder Methotrexat resistent macht. Zu bevorzugten selektierbaren Markergenen gehören das Gen für die Dihydrofolatreduktase (DHFR) (zur Verwendung in dhfr⁻-Wirtzellen mit Methotrexat-Selektion/Amplifikation) und das neo-Gen (zur G418-Selektion).

Für die Expression der leichten und schweren Ketten wird der oder werden die schwere und leichte Ketten kodierende(n) Expressionsvektor(en) mittels standardisierter Techniken in eine Wirtszelle transfiziert. Die verschiedenen Formen des Begriffs "Transfektion" sollen eine Vielzahl von Techniken erfassen, die gewöhnlicherweise zur Einführung exogener DNA in eine prokaryotische oder eukaryotische Wirtszelle verwendet werden, z.B. Elektroporation, Calcium-Phosphat-Fällung, DEAE-Dextran-Transfektion und ähnliches. Wenngleich es theoretisch möglich ist, die erfindungsgemäßen Antikörper entweder in prokaryotischen oder eukaryotischen Wirtszellen zu exprimieren, ist die Expression der Antikörper in eukaryotischen Zellen und insbesondere in Säugerwirtszellen bevorzugt, da die Wahrscheinlichkeit, dass ein korrekt gefalteter und immunologisch aktiver Antikörper zusammengesetzt und sekretiert wird, in solchen eukaryotischen Zellen und insbesondere Säugerzellen höher ist als in prokaryotischen Zellen. Über die prokaryotische Expression von Antikörpergenen ist berichtet worden, dass sie für die Produktion großer Ausbeuten aktiven Antikörpers ineffektiv sei (Boss, M.A. und Wood, C. R. (1985) Immunology Today 6:12-13).

Zu Säugerwirtszellen, die für die Expression erfindungsgemäßer rekombinanter Antikörper bevorzugt sind, gehören CHO-Zellen (einschließlich dhfr⁻-CHO-Zellen, die in Urlaub und Chasin, (1980) Proc. Natl. Acad. Sci. USA 77:4216-4220 beschrieben sind und mit einem DHFR-selektierbaren Marker verwendet werden, wie z.B. in R.J. Kaufman und P.A. Sharp (1982) Mol. Biol. 159:601-621 beschrieben ist), NS0-Myelomzellen, COS-Zellen und SP2-Zellen. Werden rekombinante Expressionsvektoren, welche die Antikörpegene kodieren, in Säugerwirtszellen eingeführt, so werden die Antikörper hergestellt, indem man die Wirtszellen so lange kultiviert, bis der Antikörper in den Wirtszellen exprimiert oder vorzugsweise der Antikörper in das Kulturmedium, in dem die Wirtszellen wachsen, sekretiert wird. Die Antikörper können aus dem Kulturmedium gewonnen werden, indem man standardisierte Verfahren zur Aufreinigung von Proteinen verwendet.

Es können ebenfalls Wirtszellen verwendet werden, um Teile intakter Antikörper, wie Fab-Fragmente oder scFv-Moleküle, herzustellen. Variationen der zuvor beschriebenen Vorgehensweise gehören selbstverständlich zur Erfindung. Beispielsweise kann es wünschenswert sein, eine Wirtszelle mit DNA zu transfizieren, die entweder die leichte Kette oder die schwere Kette (aber nicht beide) eines erfindungsgemäßen Antikörpers kodiert. Sind leichte oder schwere Ketten vorhanden, die für die Bindung des interessierenden Antigens nicht erforderlich sind, so kann die DNA, die entweder eine solche leichte oder eine solche schwere Kette oder beide kodiert, mittels rekombinanter DNA-Technologie zum Teil oder vollständig entfernt werden. Moleküle, die von solchen verkürzten DNA-Molekülen exprimiert werden, gehören ebenfalls zu den erfindungsgemäßen Antikörpern. Darüber hinaus können bifunktionale Antikörper hergestellt werden, in denen eine schwere und eine leichte Kette ein erfindungsgemäßer Antikörper sind und die andere schwere und leichte Kette Spezifität für ein anderes als das interessierende Antigen besitzen, indem man einen erfindungsgemäßen Antikörper mit einem zweiten Antikörper mittels standardisierter chemischer Verfahren quervernetzt.

In einem bevorzugten System zur rekombinanten Expression eines erfindungsgemäßen Antikörpers oder Antigen-bindenden Teils davon wird ein rekombinanter Expressionsvektor, der sowohl die schwere Antikörperkette als auch die leichte Antikörperkette kodiert, mittels Calcium-Phosphat-vermittelter Transfektion in dhfr⁻-CHO-Zellen eingeführt. Innerhalb des rekombinanten Expressionsvektors sind die Gene für die schwere und leichte Antikörperkette jeweils mit regulatorischen CMV-Enhancer/AdMLP-Promotor-Elementen operativ verknüpft, um eine starke Transkription der Gene zu bewirken. Der rekombinante Expressionsvektor trägt auch ein DHFR-Gen, mit dem sich CHO-Zellen, die mit dem Vektor transfiziert sind, selektieren lassen, indem man die Methotrexat-Selektion/Amplification verwendet. Die selektierten transformierten Wirtszellen werden kultiviert, so dass die schweren und leichten Antikörperketten exprimiert werden, und intakter Antikörper wird aus dem Kultumedium gewonnen. Man verwendet standardisierte molekularbiologische Techniken, um den rekombinanten Expressionsvektor herzustellen, die Wirtszellen zu transfizieren, die Transformanten zu selektieren, die Wirtszellen zu kultivieren und den Antikörper aus dem Kulturmedium zu gewinnen. So betrifft die Erfindung ein Verfahren zur Synthese eines erfindungsgemäßen rekombinanten Antikörpers, indem man eine erfindungsgemäße Wirtszelle in einem geeigneten Kulturmedium kultiviert, bis ein erfindungsgemäßer rekombinanter Antikörper synthetisiert ist. Das Verfahren kann des weiteren beinhalten, dass man den rekombinanten Antikörper aus dem Kulturmedium isoliert.

Alternativ zum Screenen rekombinanter Antikörperbänke durch Phage-Display können weitere, dem Fachmann bekannte Methoden zum Screenen großer kombinatorischer Bänke eingesetzt werden, um die erfindungsgemäßen Antikörper zu identifizieren. Bei einer Art eines alternativen Expressionssytems ist die rekombinante Antikörperbank in Form von RNA-Protein-Fusionen exprimiert, wie in WO 98/31700 von Szostak und Roberts, und in Roberts, R.W. und Szostak, J.W. (1997) Proc. Natl. Acad. Sci. USA 94:12297-12302 beschrieben. In diesem System erzeugt man durch in-vitro-Translation synthetischer mRNAs, die an ihrem 3'-Ende Puromycin, ein Peptidylakzeptor-Antibiotikum, tragen, zwischen einer mRNA und dem Peptid oder Protein, das sie kodiert, eine kovalente Fusion. So kann eine spezifische mRNA aus einem komplexen Gemisch von mRNAs (z.B. einer kombinatorischen Bank) anhand der Eigenschaften des kodierten Peptids oder Proteins (z.B. des Antikörpers oder eines Teils davon) wie dem Binden des Antikörpers oder Teils davon an erfindungsgemäßes Oligomer oder ein Derivat davon angereichert werden. Antikörper oder Teile davon kodierende Nukleinsäuresequenzen, die aus dem Screenen solcher Bänke gewonnen werden, können mit rekombinanten Mitteln in der oben beschriebenen Weise exprimiert werden (z.B. in Säugerwirtszellen) und darüber hinaus einer weiteren Affinitätsreifung unterzogen werden, indem man entweder in weiteren Durchgängen mRNA-Peptid-Fusionen screent, wobei man in die ursprünglich selektierte(n) Sequenz(en) Mutationen einführt, oder indem man andere Verfahren zur in-vitro-Affinitätsreifung rekombinanter Antikörper in der oben beschriebenen Weise verwendet.

### Kombinationen aus in vivo- und in vitro-Ansätzen

Die erfindungsgemäßen Antikörper können ebenfalls hergestellt werden, indem man eine Kombination aus in-vivo- und in-vitro-Ansätzen verwendet, wie Verfahren, in denen man zunächst erfindungsgemäßes Oligomer oder ein Derivat davon auf ein Antikörper-Repertoire in vivo in einem Wirtstier einwirken lässt, um die Produktion von oligomer- oder derivatbindenden Antikörpern zu stimulieren, und anschließend die weitere Antikörperselektion und/oder Antikörperreifung (d.h. Optimierung) mit Hilfe einer oder mehrerer in-vitro-Techniken bewerkstelligt wird. Einer Ausführungsform zufolge kann ein solches kombiniertes Verfahren beinhalten, dass man zunächst ein nicht-humanes Tier (z.B. eine Maus, Ratte, Kaninchen, Huhn, Kamelide, Ziege oder eine transgene Version davon oder eine chimäre Maus) mit dem erfindungsgemäßen Oligomer oder Derivat davon immunisiert, um eine Antikörper-Antwort gegen das Antigen zu stimulieren, und anschließend unter Verwendung von Immunglobulinsequenzen aus Lymphozyten, die durch die Einwirkung des Oligomers oder Derivats in vivo stimuliert worden sind, eine Phage-Display-Antikörperbank herstellt und screent. Der erste Schritt dieser kombinierten Vorgehensweise kann in der oben im Zusammenhang mit den in-vivo-Ansätzen beschriebenen Weise durchgeführt werden, während der zweite Schritt dieser Vorgehensweise in der oben in Zusammenhang mit den in-Vitro-Ansätzen beschriebenen Weise durchgeführt werden kann. Zu bevorzugten Methoden für die Hyperimmunisierung von nicht-humanen Tieren mit anschließendem in-vitro-Screening von Phage-Display-Bänken, die aus den stimulierten Lymphozyten hergestellt wurden, gehören diejenigen, die von BioSite Inc., siehe z.B. WO 98/47343, WO 91/17271, US-Patent Nr. 5,427,908 und US-Patent Nr. 5,580,717 beschrieben sind.

Einer weiteren Ausführungsform zufolge beinhaltet ein kombiniertes Verfahren, dass man zunächst ein nicht-humanes Tier (z.B. eine Maus, Ratte, Kaninchen, Huhn, Kamelide, Ziege oder eine Knockout- und/oder transgene Version davon, oder eine chimäre Maus) mit einem erfindungsgemäßen Oligomer oder Derivat davon immunisiert, um eine Antikörperantwort gegen das Oligomer oder Derivat davon zu stimulieren, und die Lymphozyten, welche die Antikörper mit der gewünschten Spezifität produzieren, selektiert, indem man (z.B. aus den immunisierten Tieren hergestellte) Hybridome screent. Die Gene für die Antikörper oder Einzeldomän-Antikörper werden aus den selektierten Klonen isoliert (mittels standardisierter Klonierungsverfahren, wie der Reversen Transkriptase-Polymerasekettenreaktion) und einer in-vitro-Affinitätsreifung unterzogen, um dadurch die Bindungseigenschaften des selektierten Antikörpers oder der selektierten Antikörper zu verbessern. Der erste Schritt dieser Vorgehensweise kann in der oben in Zusammenhang mit den in-vivo-Ansätzen beschriebenen Weise vollzogen werden, während der zweite Schritt dieser Vorgehensweise in der oben in Zusammenhang mit den in-vitro-Ansätzen beschriebenen Weise vollzogen werden kann, insbesondere indem man Verfahren zur in-vitro-Affinitätsreifung verwendet, wie diejenigen, die in WO 97/29131 und WO 00/56772 beschrieben sind.

In einem weiteren kombinierten Verfahren werden die rekombinanten Antikörper aus einzelnen isolierten Lymphozyten erzeugt, indem man eine Vorgehensweise verwendet, die dem Fachmann als Lymphozyten-Antikörper-Selektionsverfahren (SLAM) bekannt und in US-Patent Nr. 5,627,052, WO 92/02551 und Babcock, J.S. et al. (1996) Proc. Natl. Acad. Sci. USA 93:7843-7848 beschrieben ist. In diesem Verfahren wird ein nicht-humanes Tier (z.B. eine Maus, Ratte, Kaninchen, Huhn, Kamelide, Ziege, oder eine transgene Version davon, oder eine chimäre Maus) zunächst in vivo mit erfindungsgemäßem Oligomer oder einem Derivat davon immunisiert, um eine Immunantwort gegen das Oligomer oder Derivat zu stimulieren, und dann werden einzelne, interessierende Antikörper sekretierende Zellen selektiert, indem man einen antigenspezifischen hämolytischen Plaque-Assay verwendet. Hierzu können das Oligomer oder Derivat davon, oder interessierende strukturell verwandte Moleküle an Schaf-Erythrozyten gekoppelt werden, wobei man einen Linker wie Biotin verwendet, wodurch sich einzelne Zellen, die Antikörper mit geeigneter Spezifität sekretieren, unter Verwendung des hämolytischen Plaque-Assays identifizieren lassen. In Anschluss an die Identifizierung von Zellen, die interessierende Antikörper sekretieren, werden cDNAs für die variablen Regionen der leichten und schweren Ketten aus den Zellen durch Reverse Transkriptase-PCR gewonnen und diese variablen Regionen können dann in Zusammenhang mit geeigneten konstanten Immunglobulinregionen (z.B. humanen konstanten Regionen) in Säugerwirtszellen wie COS- oder CHO-Zellen exprimiert werden. Die mit den aus in vivo selektierten Lymphozyten abstammenden, amplifizierten Immungrobulinsequenzen transfizierten Wirtszellen können dann einer weiteren in-vitro-Analyse und -Selektion unterzogen werden, indem man beispielsweise die transfizierten Zellen ausbreitet, um Zellen zu isolieren, die Antikörper mit der gewünschten Spezifität exprimieren. Die amplifizierten Immunglobulinsequenzen können des weiteren in vitro manipuliert werden.

Analog zu den vorstehend beschriebenen Vorgehensweisen kann man auch Antikörper herstellen, die Spezifität für ein erfindungsgemäßes Oligomer oder Derivat davon und für strukturell verwandte synthetische Molekülen aufweisen. Dazu
i) stellt man ein Antigen bereit, das ein strukturelles Merkmal beinhaltet, welches dem erfindungsgemäßen Oligomer oder Derivat davon und den strukturell verwandten synthetischen Molekülen gemeinsam ist;
ii) lässt das Antigen auf ein Antikörper-Repertoire einwirken; und
iii) selektiert aus dem Repertoire einen Antikörper, der an zwei strukturell verwandte Moleküle bindet, wodurch man den Antikörper mit der gewünschten Spezifität erhält.

Die nach obigen Verfahren erhältlichen oligomerspezifischen Antikörper sind genauso Gegenstand der vorliegenden Erfindung wie deren Verwendung zur Herstellung eines Medikaments zur Behandlung Amyloid-ß-assoziierter dementieller Erkrankungen bzw. zur Herstellung einer Zusammensetzung zur Diagnose Amyloid-β-assoziierter dementieller Erkrankungen.

Zu den erfindungsgemäß erhältlichen Antikörpern gehören insbesondere Antiseren, die mit obigen Verfahren erhalten werden können. Dies können Vollseren sein, d.h. aus dem Wirt gewonnenes Blut nach Abtrennung der zellulären und gerinnbaren Bestandteile, oder Fraktionen dieses Serums, in denen insbesondere die Immunglobulin-Fraktion und vorzugsweise die Oligomer-erkennende Immunglobulin-Fraktion angereichert ist. Derartige Fraktionen können mit den oben im Zusammenhang mit der Antikörper-Aufreinigung beschriebenen Verfahren erhalten werden.

Die erfindungsgemäßen Antiseren sind polyklonal, d.h. sie enthalten Antikörper unterschiedlicher Spezifität, in der Regel unterschiedlicher Klassen und Subklassen, normalerweise sind sämtliche L-Ketten-Isotypen vertreten und es werden mehrere Proteinepitope erkannt.

Werden verschiedene der erfindungsgemäßen Oligomere als Immunogen verwendet, so sind die erfindungsgemäßen Antiseren in der Regel kreuzreaktiv.

Zu den erfindungsgemäß erhältlichen Antikörpern gehören einem weiteren Aspekt zufolge auch monoklonale Antikörper, insbesondere chimäre und humanisierte Antikörper, sowie oligomerbindende Fragmente davon.

Gegenstand der vorliegenden Erfindung sind Proteine und insbesondere Antikörper, die an ein erfindungsgemäßes Oligomer oder Derivat davon spezifisch binden, d.h. Antikörper mit Spezifität für ein erfindungsgemäßes Oligomer oder Derivat davon. Gegenstand der vorliegenden Erfindung sind auch Teile dieser Proteine beziehungsweise Antikörper, insbesondere antigenbindende Teile davon, d.h. Protein- bzw. Antikörperteile, die ein erfindungsgemäßes Oligomer oder ein Derivat davon binden.

Vorzugsweise wählt man den erfindungsgemäßen Antikörper so, dass er eine bestimmte Bindungskinetik aufweist (z. B. hohe Affinität, geringe Dissoziation, niedrige off-Geschwindigkeit, starke neutralisierende Aktivität) für die spezifische Bindung an erfindungsgemäßes Oligomer oder Derivat davon.

So werden Proteine und insbesondere Antikörper mit einer Affinität für das erfindungsgemäße Oligomer oder Derivat davon im Bereich von K_{D}=10⁻⁶-10⁻¹² M bevorzugt. Besonders bevorzugt sind hochaffine Proteine und insbesondere Antikörper, die mit höherer Affinität als K_{d}=10⁻⁸ M, mit höherer Affinität als K_{d}=10⁻⁹ M, mit höherer Affinität als K_{d}=10⁻¹⁰ M oder mit höherer Affinität als K_{d}=10⁻¹¹ M binden.

Einem anderen Aspekt zufolge sind diejenigen Proteine und insbesondere Antikörper bevorzugt, die andere Aβ(1-42)-Formen, insbesondere monomeres Aβ(1-42)-Protein und/oder monomeres Aβ(1-40)-Protein mit vergleichweise geringerer Affinität, insbesondere mit geringerer Affinität als K_{d}=10⁻⁸ M binden.

Demnach sind erfindungsgemäß vor allem diejenigen Proteine und insbesondere Antikörper bevorzugt, die das Oligomer oder Derivat davon mit höherer Affinität binden als monomeres Aβ(1-42)-Protein und/oder monomeres Aβ(1-40)-Protein. Affinitätsverhältnisse von 10, 100 oder 1000 sind besonders bevorzugt.

Einem weiteren Aspekt zufolge kann man die erfindungsgemäßen Antikörper so wählen, dass sie das Oligomer oder Derivat davon mit einer k_{off}-Geschwindigkeitskonstanten von 0,1 s⁻¹ oder weniger binden. Geschwindigkeitskonstanten k_{off} von 1 x 10⁻² s⁻¹ oder weniger, 1 x 10⁻³ s⁻¹ oder weniger, 1 x 10⁻⁴s⁻¹ oder weniger, oder 1 x 10⁻⁵ s⁻¹ oder weniger, sind in der angegebenen Reihenfolge zunehmend bevorzugt.

Ferner kann man erfindungsgemäße Antikörper so wählen, dass sie die Aktivität, insbesondere die neutoxische Aktivität, erfindungsgemäßer Oligomere oder Derivate davon mit einem IC₅₀ von 1 x 10⁻⁶ M oder weniger inhibieren. Hemmkonstanten IC₅₀ von 1 x 10⁻⁷ M oder weniger, 1 x 10⁻⁸ M oder weniger, 1 x 10⁹ M oder weniger, 1 x 10⁻¹⁰ M oder weniger, oder 1 x 10⁻¹¹ M oder weniger sind in der angegebenen Reihenfolge zunehmend bevorzugt.

Bei den Antikörpern handelt es sich vorzugsweise um isolierte Antikörper. Einem weiteren Aspekt zufolge sind die Antikörper neutralisierende Antikörper. Zu den erfindungsgemäßen Antikörpern gehören monoklonale und rekombinante Antikörper. Vielen Ausführungsformen zufolge kann der Antikörper eine Aminosäuresequenz beinhalten, die vollständig von einer einzigen Spezies abstammt, wie ein humaner Antikörper oder ein Mausantikörper. Weiteren Ausführungsformen zufolge kann der Antikörper ein chimärer Antikörper oder ein CDR-Graft-Antikörper oder eine andere Form eines humanisierten Antikörpers sein.

Der Begriff "Antikörper" soll sich auf Immunglobulinmoleküle beziehen, die aus 4 Polypeptidketten, zwei schweren (H) Ketten und zwei leichten (L) Ketten, gebildet sind. Die Ketten sind in der Regel durch Disulfid-Bindungen miteinander verknüpft. Jede schwere Kette setzt sich aus einer variablen Region der schweren Kette (hier als HCVR oder VH abgekürzt) und einer konstanten Region der schweren Kette zusammen. Die konstante Region der schweren Kette wird aus drei Domänen CH1, CH2 und CH3 gebildet. Jede leichte Kette setzt sich aus einer variablen Region der leichten Kette (hier als LCVR oder VL abgekürzt) und einer konstanten Region der leichten Kette zusammen. Die konstante Region der leichten Kette wird aus einer Domäne CL gebildet. Die VH- und VL-Regionen können weiter unterteilt werden in hypervariable Regionen, die als komplementaritätsbestimmende Regionen (CDR für Complementarity Determining Region) bezeichnet werden und mit konservierteren Regionen, die als Gerüstregionen (FR für Frame Work Region) bezeichnet werden, durchsetzt sind. Jede VH- und VL-Region wird aus drei CDRs und vier FRs gebildet, die vom N-Terminus zum C-Terminus in folgender Reihenfolge angeordnet sind: FR1, CDR1, FR2, CDR2, FR3, CDR3, FR4.

Der Begriff "antigenbindender Teil" eines Antikörpers (oder einfach "Antikörperteil") bezieht sich auf ein oder mehrere Fragmente eines Antikörpers mit Spezifität für ein erfindungsgemäßes Oligomer oder Derivat davon, wobei das oder die Fragmente nach wie vor die Fähigkeit besitzen, das Oligomer oder Derivat davon spezifisch zu binden. Es ist gezeigt worden, dass die antigenbindende Funktion eines Antikörpers von Fragmenten eines vollständigen Antikörpers wahrgenommen werden können. Zu Beispielen bindender Fragmente gehören im Sinne des Begriffs "antigenbindender Teil" eines Antikörpers (i) ein Fab-Fragment, d.h. ein aus den VL-, VH-, CL- und CH1-Domänen zusammengesetztes monovalentes Fragment; (ii) ein F(ab')₂-Fragment, d.h. ein bivalentes Fragment, welches zwei in der Hinge-Region über eine Disulfid-Brücke miteinander verknüpfte Fab-Fragmente beinhaltet; (iii) ein Fd-Fragment, das sich aus den VH- und CH1-Domänen zusammengesetzt; (iv) ein Fv-Fragment, das sich aus den FL- und VH-Domänen eines einzelnen Arms eines Antikörpers zusammengesetzt; (v) ein dAb-Fragment (Ward et al., (1989) Nature 341:544-546 ), das aus einer VH-Domäne oder VH, CH1, CH2, DH3, oder VH, CH2, CH3 besteht; und (vi) eine isolierte komplementaritätsbestimmente Region (CDR). Wenngleich die beiden Domänen des Fv-Fragments, nämlich VL und VH, von getrennten Genen kodiert sind, können Sie des weiteren mit einem synthetischen Linker unter Verwendung rekombinanter Verfahren miteinander verbunden werden, wodurch sie als eine einzige Proteinkette hergestellt werden können, worin die VL- und VH-Regionen sich zusammenfinden, um monovalente Moleküle zu bilden (als Einzelkette-Fv (ScFv) gekannt; siehe z.B. Bird et al. (1988) Science 242:423-426; und Huston et al. (1988) Proc. Natl. Acad. Sci. USA 85:5879-5883). Derartige Einzelketten-Antikörper sollen auch von dem Begriff "antigenbindender Teil" eines Antikörpers erfasst werden. Andere Formen von Einzelketten-Antikörpern wie "Diabodies" gehören ebenfalls dazu. Diabodies sind bivalente, bispezifische Antikörper, in denen VH- und VL-Domänen auf einer einzelnen Polypeptidkette exprimiert sind, wobei allerdings ein Linker verwendet wird, der zu kurz ist, als dass sich die beiden Domänen auf derselben Kette zusammenfinden können, wodurch man die Domänen zwingt, mit komplementären Domänen einer anderen Kette zu paaren und zwei antigenbindende Stellen zu bilden (siehe z.B. Holliger, P., et al. (1993) Proc. Natl. Acad. Sci. USA 90:6444-6448; Poljak, R.J., et al. (1994) Structure 2:1121-1123).

Des weiteren kann ein Antikörper oder antigenbindender Teil davon Teil eines größeren Immunadhäsionsmoleküls sein, das durch kovalente oder nicht kovalente Assoziierung des Antikörpers oder Antikörperteüs mit einem oder mehreren weiteren Proteinen oder Peptiden gebildet wird. Zu solchen Immunadhäsionsmolekülen gehört die Verwendung der Streptavidin-Kernregion, um ein tetrameres scFv-Molekül herzustellen (Kipriyanov, S.M., et al. (1995) Human Antibodies und Hybridomas 6:93-101) und die Verwendung eines Cysteinrestes, eines Markerpeptids und eines C-terminalen Polyhistidin-Tags, um bivalente und biotinylierte scFv-Moleküle zu machen (Kipriyanov, S.M., et al. (1994) Mol. Immunol. 31:1047-1058). Antikörperteile, wie Fab und F(ab')₂-Fragmente, können aus ganzen Antikörpern hergestellt werden, indem man konventionelle Techniken verwendet, wie die Verdauung mit Papain bzw. Pepsin. Darüber hinaus können Antikörper, Antikörperteile und Immunadhäsionsmoleküle erhalten werden, indem man standardisierte rekombinante DNA-Techniken verwendet. Ein "isolierter Antikörper mit Spezifität für ein erfindungsgemäßes Oligomer oder Derivat davon" meint einen Antikörper mit Spezifität für ein erfindungsgemäßes Oligomer oder Derivat davon, der im wesentlichen frei von anderen Antikörpern mit unterschiedlichen Antigenspezifitäten ist, also insbesondere einen Antikörper, der frei ist von Antikörpern, welche spezifisch an andere Formen des Aβ(1-42)-Proteins, wie oben beschrieben, binden.

Der Begriff "neutralisierender Antikörper" meint einen Antikörper, dessen Bindung an ein bestimmtes Antigen zur Inhibierung der biologischen Aktivität des Antigens führt. Diese Inhibierung der biologischen Aktivität des Antigens kann beurteilt werden, indem man einen oder mehrere Indikatoren für die biologische Aktivität des Antigens misst, wobei man einen geeigneten in vitro- oder in vivo-Assay verwendet.

Der Begriff "monoklonaler Antikörper" meint einen Antikörper, der von einem Hybridom abstammt (z. B. ein Antikörper, der von einem mittels Hybridomtechnologie, wie der standardisierten Hybridommethodik nach Kohler und Milstein, hergestellten Hybridom sekretiert wird). Ein von einem Hybridom abstammender Antikörper mit Spezifität für ein erfindungsgemäßes Oligomer oder Derivat davon wird daher als monoklonaler Antikörper bezeichnet.

Der Begriff "rekombinanter Antikörper" bezieht sich auf Antikörper, die mit rekombinanten Mitteln hergestellt, exprimiert, erzeugt oder isoliert werden, wie Antikörper, die unter Verwendung eines in eine Wirtszelle transfizierten, rekombinanten Expressionsvektors exprimiert werden; Antikörper, die aus einer rekombinanten kombinatorischen Antikörperbank isoliert sind; Antikörper, die aus einem Tier (z. B. einer Maus), die durch humane Immunglobulingene transgen ist, isoliert sind (siehe z. B. Taylor, L.D., et al. (1992) Nucl. Acids Res. 20:6287-6295); oder Antikörper, die auf irgend eine andere Weise, bei der bestimmte Immunoglobulin-Gensequenzen (wie humane Immunglobulin-Gensequenzen) mit anderen DNA-Sequenzen zusammengesetzt werden, hergestellt, exprimiert, erzeugt oder isoliert werden. Zu rekombinanten Antikörpern gehören beispielsweise chimäre, CDR-Graft- und humanisierte Antikörper.

Der Begriff "humaner Antikörper" bezieht sich auf Antikörper, deren variable und konstante Regionen Immunglobulinsequenzen der Humankeimbahn, wie beispielsweise von Kabat *et al.* (siehe Kabat, et al. (1991) Sequences of Proteins of Immunological Interest, Fifth Edition, U.S. Department of Health und Human Services, NIH Publication Nr. 91-3242) beschrieben, entsprechen oder davon abstammen. Die erfindungsgemäßen humanen Antikörper können allerdings Aminosäurereste beinhalten, die nicht von humanen Keimbahn-Immunglobulinsequenzen kodiert sind (z. B. Mutationen, die durch zufällige oder ortsspezifische Mutagenese in vitro oder durch somatische Mutation in vivo eingeführt sind), beispielsweise in den CDRs, und insbesondere in CDR3. Erfindungsgemäße rekombinante humane Antikörper haben variable Regionen und können auch konstante Regionen beinhalten, die von Immunglobulinsequenzen der Humankeimbahn abstammen (siehe Kabat, E.A., et al. (1991) Sequences of Proteins of Immunological Interest, Fifth Edition, U.S. Department of Health und Human Services, NIH Publication Nr. 91-3242). Bestimmten Ausführungsformen zufolge werden derartige rekombinante humane Antikörper allerdings einer in vitro-Mutagenese (oder falls ein durch humane Ig-Sequenzen transgenes Tier verwendet wird, einer somatischen in vivo-Mutagenese) unterzogen, so daß die Aminosäuresequenzen der VH- und VL-Regionen der rekombinanten Antikörper Sequenzen sind, die, wenngleich sie mit VH- und VL-Sequenzen der Humankeimbahn verwandt sind oder davon abstammen, innerhalb des humanen Antikörper-Keimbahnrepertoirs in vivo natürlicherweise nicht existieren. Bestimmten Ausführungsformen zufolge sind derartige rekombinante Antikörper das Resultat einer selektiven Mutagenese oder Rückmutation, oder beides.

Der Begriff "Rückmutation" bezieht sich auf ein Verfahren, bei dem einige oder sämtliche der somatisch mutierten Aminosäuren eines humanen Antikörpers mit den entsprechenden Keimbahnresten einer homologen Keimbahnantikörpersequenz ersetzt werden. Die Sequenzen für die schwere und leichte Kette eines erfindungsgemäßen humanen Antikörpers werden getrennt mit den Keimbahnsequenzen in der VBASE-Datenbank verglichen, um die Sequenzen mit der größten Homologie zu identifizieren. Abzeichungen im erfindungsgemäßen humanen Antikörper werden auf die Keimbahnsequenz zurückgeführt, indem man an definierten Nukleotidpositionen, die solche abweichenden Aminosäuren kodieren, mutiert. Die direkte oder indirekte Bedeutung jeder auf diese Weise als Kandidat für eine Rückmutation identifizierten Aminosäure für die Antigenbindung sollte untersucht werden, und eine Aminosäure, die nach Mutation eine wünschenswerte Eigenschaft des humanen Antikörpers beeinträchtigt, sollte in den endgültigen humanen Antikörper nicht miteinbezogen werden. Um die Anzahl der Aminosäuren für eine Rückmutation so gering wie möglich zu halten, können diejenigen Aminosäurepositionen unverändert bleiben, die zwar von der am nächsten kommenden Keimbahnsequenz abweichen aber mit der entsprechenden Aminosäuresequenz einer zweiten Keimbahnsequenz identisch sind, vorausgesetzt, daß die zweite Keimbahnsequenz mit der Sequenz des erfindungsgemäßen humanen Antikörpers zumindest in 10 und vorzugsweise in 12 Aminosäuren auf beiden Seiten der fraglichen Aminosäure identisch und co-linear ist. Rückmutationen können auf beliebiger Stufe der Antikörperoptimierung vorgenommen werden.

Der Begriff "chimärer Antikörper" bezieht sich auf Antikörper, die Sequenzen für die variable Region der schweren und leichten Kette aus einer Spezies beinhalten, in denen aber die Seuqenzen einer oder mehrerer der CDR-Regionen aus VH und/oder VL mit CDR-Sequenzen einer anderen Spezies ersetzt sind, wie Antikörper, die variable Regionen der schweren und leichten Kette aus Maus aufweisen, in denen eine oder mehrere der Maus-CDRs (z. B. CDR3) mit humanen CDR-Sequenzen ersetzt sind.

Der Begriff "humanisierter Antikörper" bezieht sich auf Antikörper, die Sequenzen der variablen Region schwerer und leichter Kette aus einer nichthumanen Spezies (z. B. Maus, Ratte, Kaninchen, Huhn, Kamelid, Ziege) beinhalten, in denen jedoch wenigstens ein Teil der VH- und/oder VL-Sequenz verändert worden ist, um mehr "humanähnlich" zu sein, d. h. variablen Sequenzen der humanen Keimbahn ähnlicher zu sein. Eine Art eines humanisierten Antikörpers ist ein CDR-Graft-Antikörper, bei dem humane CDR-Sequenzen in nichthumane VH- und VL-Sequenzen eingesetzt sind, um die entsprechenden nichthumanen CDR-Sequenzen zu ersetzen.

Ein Weg, die Bindungskinetik eines Antikörpers zu messen, ist mittels Oberflächenplasmonräsonanz. Der Begriff "Oberflächenplasmonräsonanz" bezieht sich auf ein optisches Phänomen, mit dem sich biospezifische Wechselwirkungen analysieren lassen, indem man Veränderungen von Proteinkonzentrationen mit einer Biosensormatrix nachweist, wobei man beispielsweise das BIAcore-System verwendet (Pharmacia Biosensor AB, Uppsala, Sweden und Piscataway, NJ). Für weitere Beschreibungen siehe Jönsson, U., et al. (1993) Ann. Biol. Clin. 51:19-26; Jönsson, U., et al. (1991) Biotechniques 11:620-627; Johnsson, B., et al. (1995) J. Mol. Recognit. 8:125-131; und Johnnson, B., et al. (1991) Anal. Biochem. 198:268-277.

Der Begriff "K_{off}" bezieht sich auf die off-Geschwindigkeitskonstante für die Dissoziation eines Antikörpers aus dem Antikörper/Antigen-Komplex.

Der Begriff "K_{d}" bezieht sich auf die Dissoziationskonstante einer bestimmten Antikörper-Antigen-Wechselwirkung.

Die Bindungsaffinität der erfindungsgemäßen Antikörper kann beurteilt werden, indem man standardisierte in vitro-Immunoassays, wie ELISA- oder BIAcore-Analysen, verwendet.

Neben Antikörpern kann das Protein ein vom T-Zellrezeptor abstammendes Molekül oder eine vom T-Zellrezeptor abstammende Rezeptordomäne, oder ein Fusionsprotein der Rezeptordomäne mit einem Fc-Teil eines Immunoglobulins sein.

Gegenstand der vorliegenden Erfindung sind auch pharmazeutische Mittel (Zusammensetzungen), die ein erfindungsgemäßes Protein und insbesondere einen erfindungsgemäßen Antikörper sowie gegebenenfalls einen pharmazeutisch verträglichen Träger beinhalten. Erfindungsgemäße pharmazeutische Zusammensetzungen können des weiteren wenigstens ein zusätzliches therapeutisches Agens, z. B. ein oder mehrere zusätzliche therapeutische Agenzien zur Behandlung einer Erkrankung, zu deren Linderung die erfindungsgemäßen Antikörper brauchbar sind, beinhalten. Bindet der erfindungsgemäße Antikörper beispielsweise an ein erfindungsgemäßes Oligomer, kann die pharmazeutische Zusammensetzung des weiteren ein oder mehrere zusätzliche therapeutische Agenzien beinhalten, die zur Behandlung von Erkrankungen brauchbar sind, bei denen die Aktivität des Oligomers von Bedeutung ist.

Zu pharmazeutisch verträglichen Trägern gehören sämtliche Lösungsmittel, Dispersionsmedien, Überzüge, antibakterielle und gegen Pilze gerichtete Agentien, isotonische und die Absorption verzögernde Agenzien, und dergleichen, sofern sie physiologisch kompatibel sind. Zu pharmazeutisch akzeptablen Trägern gehören beispielsweise Wasser, Kochsalzlösung, Phosphat-gepufferte Kochsalzlösung, Dextrose, Glyzerin, Ethanol und ähnliches, sowie Kombinationen davon. In vielen Fällen ist es bevorzugt, isotonische Agenzien, beispielsweise Zucker, Polyalkohole, wie Mannitol oder Sorbitol, oder Natriumchlorid mit zu verwenden. Pharmazeutisch verträgliche Träger können des weiteren geringere Mengen an Hilfssubstanzen, wie Netzmittel oder Emulgatoren, Konservierungsmittel oder Puffer beinhalten, die die Haltbarkeit oder Wirksamkeit der Antikörper erhöhen.

Die pharmazeutischen Zusammensetzungen können beispielsweise zur parenteralen Verabreichung geeignet sein. Hier werden die Antikörper vorzugsweise als injizierbare Lösungen mit einem Antikörpergehalt von 0,1 - 250 mg/ml zubereitet. Die injizierbaren Lösungen können in flüssiger oder lyophilisierter Form in einem Flintglas oder vial, einer Ampulle oder einer gefüllten Spritze als Dosierungsform zubereitet sein. Der Puffer kann L-Histidin (1 - 50 mM, vorzugsweise 5 - 10 mM) enthalten und einen pH-Wert von 5,0 - 7,0, vorzugsweise von 6,0, aufweisen. Zu weiteren geeigneten Puffern gehören, ohne darauf beschränkt zu sein, Natriumsuccinat-, Natriumcitrat-, Natriumphosphat- oder Kaliumphosphat-Puffer. Man kann Natriumchlorid verwenden, um die Tonizität der Lösung auf eine Konzentration von 0 - 300 mM (vorzugsweise 150 mM für eine flüssige Dosierungsform) einzustellen. Cryoschutzmittel können für eine lyophilisierte Dosierungsform mit einbezogen werden, z.B. Sukrose (z.B. 0 -10 %, vorzugsweise 0,5 -1,0 %). Zu weiteren geeigneten Cryoschutzmitteln gehören Trehalose und Laktose. Füllmittel können für eine lyophilisierte Dosierungsform mit einbezogen werden, z.B. Mannitol (z.B. 1-10 %, vorzugsweise 2 - 4 %). Stabilisatoren können sowohl in flüssigen als auch lyophilisierten Dosierungsformen verwendet werden, z.B. L-Methionin (z.B. 51 - 50 mM, vorzugsweise 5 -10 mM). Zu weiteren geeigneten Füllmitteln gehören Glycin und Arginin. Ebenso können Tenside verwendet werden, beispielsweise Polysorbat-80 (z.B. 0 - 0,05 %, vorzugsweise 0,005 - 0,01 %). Zu weiteren Tensiden gehören Polysorbat-20 und BRIJ-Tenside.

Die erfindungsgemäßen Zusammensetzungen können eine Vielzahl von Formen annehmen. Zu diesen gehören flüssige, halbfeste und feste Dosierungsformen, wie flüssige Lösungen (z. B. injizierbare und infundierbare Lösungen), Dispersionen oder Suspensionen, Tabletten, Pillen, Pulver, Liposome und Suppositorien. Die bevorzugte Form hängt von der beabsichtigten Verabreichungsart und der therapeutischen Anwendung ab. Typischerweise werden Zusammensetzungen in Form von injizierbaren oder infundierbaren Lösungen bevorzugt, beispielsweise Zusammensetzungen, die für die passive Immunisierung von Menschen mit anderen Antikörpern Ähnlichkeit besitzen. Der bevorzugte Verabreichungsweg ist parenteral (z. B. intravenös, subkutan, intraperitoneal, intramuskulär). Gemäß einer bevorzugten Ausführungsform wird der Antikörper durch intravenöse Infusion oder Injektion verabreicht. Einer weiteren bevorzugten Ausführungsform zufolge wird der Antikörper durch intramuskuläre oder subkutane Injektion verabreicht.

Therapeutische Zusammensetzungen müssen typischerweise steril und unter den Herstellungs- und Lagerungsbedingungen stabil sein. Die Zusammensetzungen können als Lösung, Mikroemulsion, Dispersion, Liposom oder einer weiteren für hohe Wirkstoffkonzentrationen geeigneten, geordneten Struktur formuliert werden. Sterile injizierbare Lösungen können hergestellt werden, indem man die aktive Verbindung (d. h. den Antikörper) in der benötigten Menge in ein geeignetes Lösungsmittel gegebenenfalls mit einem oder einer Kombination der vorstehend genannten Inhaltsstoffe, je nach Bedarf, einbringt und anschließend steril filtriert. Dispersionen werden in der Regel zubereitet, indem man die aktive Verbindung in ein steriles Vehikel einbringt, welches ein Grunddispersionsmedium und gegebenenfalls weitere benötigte Inhaltsstoffe enthält. Im Falle eines sterilen lyophilisierten Pulvers zur Herstellung steriler injizierbarer Lösungen stellen die Vakuumtrocknung und Sprühtrocknung bevorzugte Herstellungsverfahren dar, mit denen ein Pulver des aktiven Inhaltsstoffes und gegebenenfalls weiterer erwünschter Inhaltsstoffe aus einer zuvor steril filtrierten Lösung erhalten wird. Die richtige Fliessfähigkeit einer Lösung kann beibehalten werden, indem man beispielsweise einen Überzug wie Lecithin verwendet, im Fall von Dispersionen die benötigte Partikelgröße beibehält oder Tenside verwendet. Eine verlängerte Absorption injizierbarer Zusammensetzungen kann erreicht werden, indem man ein Agens, das die Absorption verzögert, beispielsweise Monostearatsalze und Gelatine, in die Zusammensetzung mit einbringt.

Die erfindungsgemäßen Antikörper können mit einer Vielzahl von Verfahren, die dem Fachmann bekannt sind, verabreicht werden, wenngleich für viele therapeutische Anwendungen die subkutane Injektion, intravenöse Injektion oder Infusion die bevorzugte Verabreichungsart darstellt. Der Fachmann weiß, dass Weg und/oder Art der Verabreichung vom gewünschten Resultat abhängen. Bestimmten Ausführungsformen zufolge kann die aktive Verbindung mit einem Träger zubereitet werden, der die Verbindung gegen rasche Freisetzung schützt, so zum Beispiel eine Formulierung mit kontrollierter Freisetzung, wozu Implantate, transdermale Pflaster und mikroverkapselte Abgabesysteme gehören. Es können biologisch abbaubare biokompatible Polymere verwendet werden, wie Ethylenvinylacetat, Polyanhydride, Polyglykolsäure, Collagen, Polyorthoester und Polymilchsäure. Die Verfahren zur Zubereitung solcher Formulierungen sind dem Fachmann allgemein bekannt, siehe zum Beispiel Sustained und Controlled Release Drug Delivery Systems, J.R. Robinson, ed., Marcel Dekker, Inc., New York, 1978.

Bestimmten Ausführungsformen zufolge kann ein erfindungsgemäßer Antikörper oral verabreicht werden, beispielsweise in einem inerten Verdünnungsmittel oder einem assimilierbaren essbaren Träger. Der Antikörper (und gewünschtenfalls weitere Inhaltsstoffe) können auch in einer Hart- oder Weichgelatinekapsel eingeschlossen, zu Tabletten verpresst oder direkt der Nahrung zugesetzt werden. Für die orale therapeutische Verabreichung können die Antikörper mit Exzipienten vermischt und in Form von schluckbaren Tabletten, Buccaltabletten, Kapseln, Elixieren, Suspensionen, Sirups und dergleichen verwendet werden. Soll ein erfindungsgemäßer Antikörper über einen anderen als den parenteralen Weg verabreicht werden, kann es erforderlich sein, eine Beschichtung aus einem Material zu wählen, das seine Inaktivierung verhindert.

Die erfindungsgemäßen Antikörper sind vorzugsweise in der Lage, sowohl in vitro als in vivo die Aktivität erfindungsgemäßer Oligomere oder Derivate davon, an die sie binden, zu neutralisieren. Die Antikörper können daher zur Inhibierung der Aktivität erfindungsgemäßer Oligomere oder Derivate davon verwendet werden, z. B. in einer die Oligomere oder Derivate davon enthaltenden Zellkultur oder in humanen Individuen oder anderen Säugern, in denen die Oligomere oder Derivate davon zugegen sind. Einer Ausführungsform zufolge ist Gegenstand der Erfindung ein Verfahren zur Inhibierung der Aktivität erfindungsgemäßer Oligomere oder Derivate davon, wobei man einen erfindungsgemäßen Antikörper auf ein Oligomer oder Derivat davon so einwirken lässt, dass die Aktivität des Oligomers oder Derivats davon inhibiert wird. Dabei kann die Aktivität beispielsweise in vitro inhibiert werden. Beispielsweise kann der erfindungsgemäße Antikörper einer Zellkultur, die das erfindungsgemäße Oligomer oder Derivat davon enthält oder unter Verdacht steht, es zu enthalten, zugesetzt werden, um die Aktivität des Oligomers oder des Derivats davon in der Kultur zu inhibieren. Alternativ kann die Aktivität des Oligomers oder Derivats davon in einem Individuum in vivo inhibiert werden.

So betrifft ein weiterer Gegenstand der vorliegenden Erfindung ein Verfahren zur Inhibierung der Aktivität erfindungsgemäßer Oligomere oder Derivate davon in einem Individuum, das an einer Erkrankung leidet, bei der Amyloid-β-Protein beteiligt ist und insbesondere die Aktivität des erfindungsgemäßen Oligomers oder Derivats davon von Bedeutung ist. Dieses Verfahren beinhaltet die Verabreichung wenigstens eines erfindungsgemäßen Antikörpers an das Individuum mit dem Ziel, die Aktivität des Oligomers oder Derivats davon, an die oder das der Antikörper bindet, zu inhibieren. Das Individuum ist vorzugsweise ein Mensch. Ein erfindungsgemäßer Antikörper kann einem menschlichen Individuum für therapeutische Zwecke verabreicht werden. Darüber hinaus kann ein erfindungsgemäßer Antikörper einem nichtmenschlichen Säuger für veterinärmedizinische Zwecke oder im Rahmen eines Tiermodell für eine bestimmte Erkrankung verabreicht werden. Derartige Tiermodelle können nützlich sein, um die therapeutische Wirksamkeit erfindungsgemäßer Antikörper zu beurteilen (z. B. um Dosierungen und zeitliche Abläufe der Verabreichung zu testen).

Zu Erkrankungen, bei der erfindungsgemäße Oligomere oder Derivate davon eine Rolle spielen, gehören insbesondere Erkrankungen, an deren Entstehung und/oder Verlauf ein erfindungsgemäßes Oligomer oder Derivat davon beteiligt ist. Damit sind insbesondere solche Erkrankungen gemeint, bei denen erfindungsgemäße Oligomere oder Derivate davon nachweislich oder vermutlich für die Pathophysiologie der Erkrankung verantwortlich sind oder einen Faktor darstellen, der zur Entstehung und/oder Verlauf der Erkrankung beiträgt. Dementsprechend gehören hierzu solche Erkrankungen, bei denen die Inhibierung der Aktivität erfindungsgemäßer Oligomere oder Derivate davon Symptome und/oder Progression der Erkrankung zu lindern vermag. Solche Erkrankungen lassen sich beispielsweise dadurch belegen , dass sich eine erhöhte Konzentration erfindungsgemäßer Oligomere oder Derivate davon in einem biologischen Fluid eines Individuums, das an einer bestimmten Erkrankung leidet (z. B. einer erhöhten Konzentration in Serum, Plasma, CSF, Urin, etc.). Dies kann beispielsweise nachgewiesen werden, indem man einen erfindungsgemäßen Antikörper verwendet. Die erfindungsgemäßen Oligomere und Derivate davon spielen eine entscheidende Rolle in der Pathologie, die mit einer Vielzahl von Erkrankungen zusammenhängt, an denen neurodegenerative Elemente, kognitive Defizite, neurotoxische Elemente und inflammatorische Elemente beteiligt sind.

Die erfindungsgemäßen Antikörper können zusammen mit einem oder mehreren zusätzlichen therapeutischen Agenzien verabreicht werden, die bei der Behandlung der zuvor beschriebenen Erkrankungen brauchbar sind.

Die pharmazeutischen Zusammensetzungen der vorliegenden Erfindung enthalten in der Regel eine therapeutisch wirksame Menge oder eine prophylaktisch wirksame Menge wenigstens eines erfindungsgemäßen Antikörpers. In Abhängigkeit von der gewünschten Behandlung, ob beispielsweise eine therapeutische oder prophylaktische Behandlung gewünscht ist, können Dosierungspläne gewählt und angepasst werden. Beispielsweise kann man eine Einzeldosis, mehrere getrennte Dosen über die Zeit verteilt oder eine ansteigende bzw. sinkende Dosierung je nach den Anforderungen der therapeutischen Situation verabreichen. Es ist insbesondere von Vorteil, parentale Zusammensetzungen in Einheitsdosierungsform zu formulieren, um die Verabreichung zu erleichtern und eine Gleichförmigkeit der Dosierung zu gewährleisten.

Eine therapeutisch oder prophylaktisch wirksame Menge eines erfindungsgemäßen Antikörpers kann beispielsweise im Bereich von 0,1-20 mg/kg und vorzugsweise 1 - 10 mg/kg liegen, ohne darauf beschränkt zu sein. Natürlich können diese Mengen je nach Art und Schwere des zu lindernden Zustandes variieren.

Im Rahmen der diagnostischen Verwendung der Antikörper dient die qualitative oder quantitative spezifische Oligomer-Bestimmung insbesondere der Diagnose krankheitsrelevanter Amyloid-β(1-42)-Formen. Spezifität bedeutet in diesem Zusammenhang die Möglichkeit, ein bestimmtes Oligomer oder Oligomer-Gemisch mit ausreichender Sensitivität nachweisen zu können. Die erfindungsgemäßen Antikörper weisen vorteilhafterweise Sensitivitäten von weniger als 10 ng/ml Probe, vorzugsweise von weniger als 1 ng/ml Probe und besonders bevorzugt von weniger als 100 pg/ml Probe auf. Damit ist gemeint, dass sich zumindest die jeweils angegebene Konzentration an Oligomer pro ml Probe mit den erfindungsgemäßen Antikörpern nachweisen lässt, vorteilhafterweise auch geringere Konzentrationen.

Die Bestimmung erfolgt immunologisch. Prinzipiell kann dies mit jedem analytischen bzw. diagnostischen Testverfahren erfolgen, bei dem Antikörper eingesetzt werden. Hierzu gehören Agglutinations- und Präzipitations-Techniken, Immunoassays, immunhistochemische Verfahren und Immunoblot-Techniken, z.B. Western-Blotting oder Dot Blot-Verfahren. Auch in vivo-Verfahren gehören dazu, beispielsweise bildgebende Verfahren.

Von Vorteil ist der Einsatz in Immunoassays. Geeignet sind sowohl kompetitive Immunoassays, d.h. Antigen und markiertes Antigen (Tracer) konkurrieren um die Antikörperbindung, als auch Sandwich-Immunoassays, d.h. die Bindung spezifischer Antikörper an das Antigen wird mit einem zweiten, meist markierten Antikörper nachgewiesen. Diese Assays können sowohl homogen, d.h. ohne eine Trennung in feste und flüssige Phase, als auch heterogen sein, d.h. gebundene Markierungen werden von ungebundenen getrennt, beispielsweise über festphasengebundene Antikörper. Die verschiedenen heterogenen und homogenen Immunoassay-Formate können je nach Markierung und Meßmethode bestimmten Klassen zugeordnet werden, beispielsweise RIAs (Radioimmunoassays), ELISA (Enzyme linked immunosorbent assay), FIA (Fluoreszenz-Immunoassay), LIA (Lumineszenz-Immunoassay), TRFIA (zeitlich aufgelöster FIA), IMAC (Immunaktivierung), EMIT (Enzyme multiplied immune test), TIA (Turbodimetrischer Immunoassay), I-PCR (Immuno-PCR).

Zur erfindungsgemäßen Oligomer-Bestimmung sind kompetitive Immunoassays bevorzugt. Dabei konkurriert markiertes Oligomer (Tracer) mit dem zu quantifizierenden Oligomer der Probe um die Bindung an den verwendeten Antikörper. Aus der Menge des verdrängten Tracers lässt sich mit Hilfe einer Standardkurve die Antigenmenge, sprich die Oligomermenge, in der Probe bestimmen.

Von den für diese Zwecke zur Verfügung stehenden Markierungen haben sich Enzyme als vorteilhaft erwiesen. Beispielsweise können Systeme auf Basis von Peroxidasen, insbesondere der Meerrettich-Peroxidase, der alkalischen Phosphatase und der ß-D-Galactosidase verwendet werden. Für diese Enzyme stehen spezifische Substrate zur Verfügung, deren Umsetzung sich z.B. photometrisch verfolgen lässt. Geeignete Substratsysteme basieren auf p-Nitrophenylphosphat (p-NPP), 5-Brom-4-chlor-3-indolylphosphat/Nitroblau-Tetrazolium (BCIP/NPT), Fast-Red/Naphthol-AS-TS-Phosphat für die alkalische Phosphatase; 2,2-Azino-bis-(3-ethylbenzthiazolin-6-sulfonsäure) (ABTS), o-Phenylendiamin (OPT), 3,3',5,5'-Tetramethylbenzidin (TMB), o-Dianisidin, 5-Aminosalicylsäure, 3-Dimethylaminobenzoesäure (DMAB) und 3-Methyl-2-benzothiazolinhydrazon (MBTH) für Peroxidasen; o-Nitrophenyl-β-D-galactosid (o-NPG), p-Nitrophenyl-β-D-Galactosid und 4-Methylumbelliphenyl-ß-D-galactosid (MUG) für die ß-D-Galactosidase. Diese Substratsysteme sind in vielen Fällen in gebrauchsfertiger Form kommerziell erhältlich, beispielsweise in Form von Tabletten, die auch weitere Reagenzien, wie zweckmäßige Puffer und ähnliches enthalten können.

Als Tracer werden markierte Oligomere verwendet. In diesem Sinne kann man zur Bestimmung eines bestimmten Oligomers das zu bestimmende Oligomer markieren und als Tracer einsetzen.

Die Kopplung von Markierungen an Oligomere zur Herstellung von Tracern kann in an sich bekannter Weise erfolgen. Auf obige Ausführungen zur Derivatisierung erfindungsgemäßer Oligomere wird sinngemäß Bezug genommen. Darüber hinaus stehen eine Reihe zur Konjugation an Proteine zweckmäßig modifizierte Markierungen zur Verfügung, beispielsweise Biotin-, Avidin-, Extravidin- oder Streptavidin-konjugierte Enzyme, Maleimid-aktivierte Enzyme und ähnliches. Diese Markierungen können direkt mit dem Oligomer oder - falls erforderlich - mit entsprechend derivatisiertem Oligomer zum Tracer umgesetzt werden. Wird beispielsweise ein Streptavidin-Peroxidase-Konjugat verwendet, so erfordert dies zunächst eine Biotinylierung des Oligomers. Entsprechendes gilt für die umgekehrte Anordnung. Auch zu diesem Zweck sind dem Fachmann geeignete Verfahren bekannt.

Wird ein heterogenes Immunoassay-Format gewählt, so kann der Antigen-Antikörper-Komplex zwecks Trennung beispielsweise über einen an den Träger gekoppelten anti-idiotypischen Antikörper, z.B. einen gegen Kaninchen-IgG gerichteten Antikörper, an den Träger gebunden werden. Träger, insbesondere Mikrotiterplatten, die mit entsprechenden Antikörpern beschichtet sind, sind bekannt und teilweise kommerziell erhältlich.

Ein weiterer Gegenstand der vorliegenden Erfindung sind Immunoassay-Sets mit wenigstens einem vorstehend beschriebenen Antikörper und weiteren Komponenten. Es handelt sich hierbei um eine Zusammenstellung, in der Regel als Verpackungseinheit, von Mitteln zur Durchführung einer erfindungsgemäßen Oligomer-Bestimmung. Zwecks möglichst einfacher Handhabung werden diese Mittel vorzugsweise im wesentlichen gebrauchsfertig bereitgestellt. Eine vorteilhafte Anordnung bietet den Immunoassay in Kit-Form an. Ein Kit umfasst in der Regel mehrere Behältnisse zur getrennten Anordnung von Komponenten. Alle Komponenten können in gebrauchsfertiger Verdünnung, als Konzentrat zum Verdünnen oder als Trockensubstanz oder Lyophilisat zum Lösen oder Suspendieren bereitgestellt werden; einzelne oder sämtliche Komponenten können eingefroren sein oder bei Umgebungstemperatur bis zum Gebrauch aufbewahrt werden. Seren sind vorzugsweise schockgefroren, beispielsweise bei -20°C, so dass in diesen Fällen ein Immunoassay vor Gebrauch vorzugsweise bei Gefriertemperaturen zu halten ist.

Weitere, dem Immunoassay beigefügte Komponenten richten sich nach Art des Immunoassays. In der Regel sind zusammen mit dem Antiserum Standardprotein, gegebenenfalls erforderlicher Tracer und Kontrollserum beigefügt. Ferner können Mikrotiterplatten, vorzugsweise mit Antikörper beschichtet, Puffer, beispielsweise zum Testen, zum Waschen oder zur Umsetzung des Substrats, und das Enzymsubstrat selbst beigefügt sein.

Allgemeine Prinzipien von Immunoassays und die Erzeugung und Verwendung von Antikörpern als Hilfsmittel in Labor und Klinik finden sich beispielsweise in Antibodies, A Laboratory Manual (Harlow, E., and Lane, D., Ed., Cold Spring Harbor Laboratory, Cold Spring Harbor, NY, 1988).

Des weiteren sind auch Substanzen, welche die Aggregation der erfindungsgemäßen Oligomere inhibieren oder deren Disaggregation beschleunigen, von Interesse. Derartige Substanzen stellen insbesondere mögliche Therapeutika für die Behandlung obiger Amyloid-ß-assoziierter Erkrankungen dar, wie z. B. der Alzheimer Erkrankung.

Gegenstand der vorliegenden Erfindung ist daher auch ein Verfahren zur Charakterisierung einer Substanz oder eines Substanzgemisches, wobei man
i) die Substanz oder das Substanzgemisch in geeigneter Weise zur Verfügung stellt;
ii) die Substanz oder das Substanzgemisch auf wenigstens ein erfindungsgemäßes Oligomer, Derivat davon oder eine Zusammensetzung einwirken lässt; und
iii) bestimmt, ob die Substanz oder bestimmte Teile des Substanzgemisches an das Oligomer, Derivat davon oder wenigstens ein in der Zusammensetzung enthaltenes Oligomer oder Derivat davon binden.

Dieses Verfahren kann auch an Gemischen biologischen Ursprungs, z.B. Zellpräparation und -extrakten durchgeführt werden, um natürliche Bindungspartner mit Affinität für die erfindungsgemäßen Oligomere, z.B. Zelloberflächenantigene, insbesondere Rezeptoren, und lösliche Liganden, beispielsweise bestimmte Proteine und Mediatoren, zu identifizieren.

Zusätzlich zur bloßen Bindung der Substanzen können auch weitere Wechselwirkungen mit und Einflüsse auf die erfindungsgemäßen Oligomere Gegenstand des Verfahrens sein. So kann man insbesondere bestimmen, ob
- die Substanz die Aggregation von Amyloid-ß-Protein zu den erfindungsgemäßen Oligomeren zu modulieren, insbesondere zu inhibieren vermag;
- die Substanz die Disaggregation der erfindungsgemäßen Oligomere zu modulieren, insbesondere zu fördern vermag;
- die erfindungsgemäßen Oligomere funktionale Veränderungen an einem Bindungspartner herrufen, beispielsweise an einem Rezeptor eine agonistische, partiell agonistische, antagonistische oder invers agonistische Wirkung besitzen.

Bei diesen Verfahren handelt es sich in der Regel um *in vitro*-Screening-Verfahren, mit denen man aus einer Vielzahl verschiedener Substanzen diejenigen auslesen kann, die im Hinblick auf eine künftige Anwendung am aussichtsreichsten zu sein scheinen. Beispielsweise können mittels kombinatorischer Chemie umfangreiche Stoffbanken angelegt werden, die Myriaden potentieller Wirkstoffe umfassen. Das Durchmustern kombinatorischer Substanzbibliotheken nach Stoffen mit gewünschter Aktivität ist automatisierbar. Screening-Roboter dienen der effizienten Auswertung der vorzugsweise auf Mikrotiterplatten angeordneten Einzelassays. So betrifft die vorliegende Erfindung auch Screening-Verfahren, d.h. sowohl Primär- als auch Sekundärscreening-Verfahren, bei denen vorzugsweise wenigstens eines der nachfolgend beschriebenen Verfahren zur Anwendung kommt. Kommen mehrere Verfahren zur Anwendung, so kann das zeitlich versetzt oder gleichzeitig an ein und derselben Probe oder an verschiedenen Proben einer zu untersuchenden Substanz geschehen.

Eine besonders effektive Technologie zur Durchführung derartiger Verfahren ist der im Bereich des Wirkstoffscreenings bekannte Scintillation Proximity Assay, kurz SPA genannt. Kits und Komponenten zur Durchführung dieses Assays können kommerziell bezogen werden, beispielweise bei Amersham Pharmacia Biotech. Im Prinzip werden solubilisierte oder membrangebundene Rezeptoren auf Scintillationssubstanz enthaltenden, kleinen Fluoromikrosphären immobilisert. Bindet beispielsweise ein Radioligand an die immobilisierten Rezeptoren, so wird die Scintillationssubstanz zur Lichtemission angeregt, da die räumliche Nähe zwischen Scintillationssubstanz und Radioligand gegeben ist.

Eine weitere besonders effektive Technologie zur Durchführung derartiger Verfahren ist die im Bereich des Wirkstoffscreenings bekannte FlashPlateR-Technologie. Kits und Komponenten zur Durchführung dieses Assays können kommerziell bezogen werden, beispielweise bei NENR Life Science Products. Dieses Prinzip basiert ebenfalls auf Mikrotiterplatten (96er oder 384er), die mit Scintillationssubstanz beschichtet sind.

Die nach diesem Verfahren als ein an das Oligomer, Derivat davon oder wenigstens ein in einer entsprechenden Zusammensetzung enthaltendes Oligomer oder Derivat davon bindender Ligand identifizierbaren Substanzen oder Teile aus Substanzgemischen sind genauso Gegenstand der vorliegenden Erfindung wie deren Verwendung zur Herstellung eines Medikaments zur Behandlung Amyloid-ß-assoziierter, insbesondere dementieller Erkrankungen bzw. zur Herstellung einer Zusammensetzung zur Diagnose Amyloid-ß-assoziierter, insbesondere dementieller Erkrankungen.

Die folgenden Beispiele sollen die Erfindung veranschaulichen, ohne sie in ihrem Umfang einzuschränken.

In den Zeichnungen zeigt
- Figur 1: eine SDS-PAGE einer Aβ(1-42)-Oligomeren A-Präparation (Bahn A); Aβ(1-42)-Oligomeren B-Präparation (Bahn B); von Standardproteinen (molekularen Markerproteinen, Bahn C);
- Figur 2: eine SDS-PAGE einer Aβ(1-42)-Oligomeren A-Präparation (Bahn A); Aβ(1-42)-Oligomeren A-CL-Präparation (Bahn A'); Aβ(1-42)-Oligomeren B-Präparation (Bahn B); Aβ(1-42)-Oligomeren B-CL-Präparation (Bahn B'); von Standardproteinen (molekularen Markerproteinen, Bahn C);
- Figur 3: eine SDS-PAGE einer Biotin-Aβ(1-42)-Oligomeren B-Präparation (Bahn A); von Standardproteinen (molekularen Markerproteinen, Bahn B);
- Figur 4: eine SDS-PAGE einer Fluorescein-Aß(1-42)-Oligomeren B-Präparation (Bahn A); von Standardproteinen (molekularen Markerproteinen, Bahn B);
- Figur 5: eine Gelpermeationschromatographie von Aß(1-42)-Lyophilisat enthaltender Lösung im Vergleich zu Aß(1-42)-Oligomere B enthaltender Präparation;
- Figur 6: eine NATIVE-PAGE einer Aß(1-42)-Oligomeren B-Präparation (Bahn A); von Standardproteinen (molekularen Markerproteinen, Bahn B);
- Figur 7: die Bindung von (A) monomerem Aß(1-42)-Protein und (B) den Aβ(1-42)-Oligomeren A sowie (C) Aß(1-42)-Oligomeren B an die Oberfläche der humanen Neuroblastom-Zell-Linie IMR-32;
- Figur 8: die neurotoxische Wirkung in % nach Behandlung von corticalen Neuronen der Maus mit Aß(1-42)-Oligomeren B, wobei der Fehlerbalken dem 95% Konfidenzintervall entspricht;
- Figur 9: eine SDS-PAGE einer Aß(1-42)-Präparation, die mit Trypsin (Bahn 2), Chymotrypsin (Bahn 3), Thermolysin (Bahn 4), Elastase (Bahn 5), Papain (Bahn 6) behandelt oder unbehandelt (Bahn 7) war; sowie von Standardproteinen (molekularen Markerproteinen, Bahn 1);

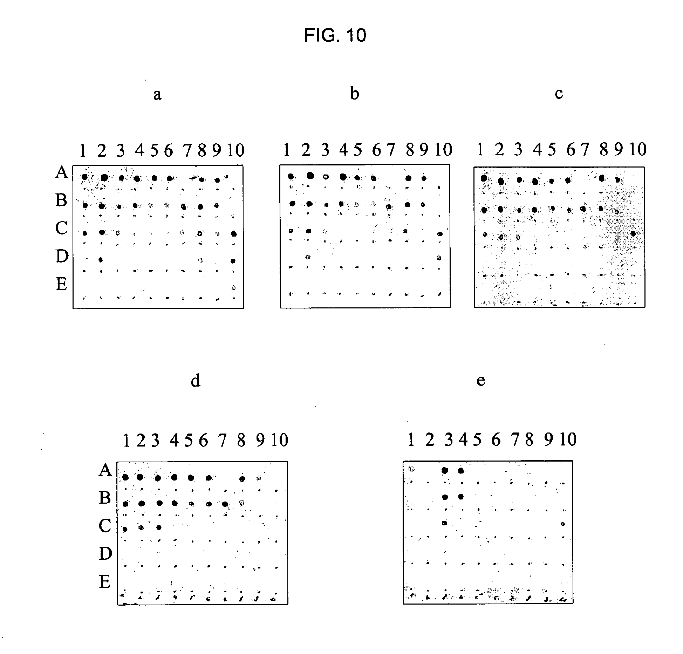

- Figur 10: Dot Blots zur Reaktivität von 100 pmol (Zeile A); 10 pmol (Zeile B); 1pmol (Zeile C); 0,1 pmol (Zeile D) bzw. 0,01 pmol (Zeile E) Aβ(1-42)-Oligomeren B-Präparation aus Beispiel 6b (Spalte 1); HFIP-behandeltem Aß(1-42)-Monomer aus Beispiel 1a (Spalte 2); mit Thermolysin gespaltener Aß(1-42)-Oligomeren B-Präparation aus Beispiel 15a (Spalte 3); mit Glutaraldehyd vernetzter Aβ(1-42)-Oligomeren B-Präparation aus Beispiel 14a (Spalte 4); gemäß M.P. Lambert et al., J. Neurochem. 79, 595-605 (2001)) bei 4 °C oder Raumtemperatur oder 37°C hergestellter ADDL (Spalte 5, 6 bzw. 7); in 0,1 % NH₄OH gelöstem Aβ(1-42) (Spalte 8); einer Aβ(1-42)-Fibrillen-Präparation aus Beispiel 27 (Spalte 9); und in PBS verdünntem APP der Fa. Sigma (Spalte 10) mit a) dem monoklonalen Antikörper 6E10; b) dem polyklonalen Antiserum (d1) aus Beispiel 25d; c) dem polyklonalen Antiserum (c1) aus Beispiel 25c; d) dem polyklonalen Antiserum (a1) aus Beispiel 25a; und e) dem polyklonalen Antiserum (a2) aus Beispiel 25a;
- Figur 11: eine SDS-PAGE einer Aβ(20-42)-Oligomeren B-Präparation aus Beispiel 15a (Bahn B); Aβ(12-42)-Oligomeren B-Präparation aus Beispiel 15b (Bahn C), Aβ(1-42)-Oligomeren B-Präparation aus Figur 12 Beispiel 6b (Bahn A); sowie von Standardproteinen (molekularen Markerproteinen, Bahn D); eine Gelpermeationschromatografie der Aß(1-42)-Oligomeren B-Präparation aus Beispiel 6b im Vergleich zur Aβ(1-42)-Oligomeren B-CL-Präparation aus Beispiel 14a.

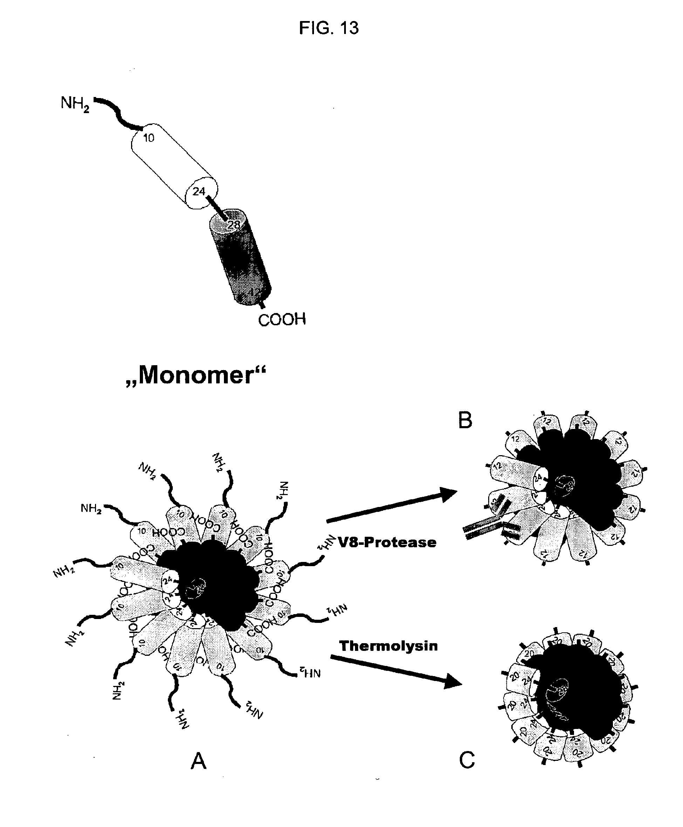

- Figur 13: eine schematische Darstellung des monomeren Aβ(1-42)-Proteins (links); des Aβ(1-42)-Oligomeren (12-mer, A); und der durch proteolytische Spaltung erhältlichen Aβ(12-42)-Oligomeren (12-mer, C) und Aß(20-42)-Oligomeren (12-mer, B);
- Figur 14: das excitatorische postsynaptische Potential (fEPSP) in Abhängigkeit von der Zeit unter Einwirkung von Aβ(1-42)-Oligomeren B auf Hippocampusschnitte;
- Figur 15: eine Immunfluoreszenzaufnahme, welche die Bindung von Aβ(1-42)-Oligomeren B an hippocampale Rattenneuronen (a) im Vergleich zur unspezifisch gebundenen Fluoreszenz (b) zeigt.

Sofern nicht anders angegeben, sind Konzentrationen erfindungsgemäßer Oligomere in Mol monomeren Aß(1-42)-Polypeptids ausgedrückt. Der Begriff β-Amyloid(1-42)-Protein entspricht dem Begrif Amyloid-β(1-42)-Protein. Sofern nicht anders angegeben, sind die verwendeten Proteine (Polypeptide) humanen Ursprungs.

### Beispiel 1

### a) Herstellung einer Stammsuspension von humanem Aβ(1-42)

2 mg humanes β-Amyloid(1-42)-Protein (kurz: Aβ(1-42); peptidsynthetisches Material, Lyophilisat, Fa. Bachem, Deutschland) werden in 800 µl 1,1,1,3,3,3-Hexafluor-2-propanol gelöst und 30 min bei 37°C in einem Eppendorfgefäß inkubiert. Danach wird im Vakuumkonzentrator (Speed Vac) zur Trockne eingedampft. Der Rückstand wird mit 88 µl DMSO aufgenommen, wobei eine 5 mM Aβ(1-42)-Stammsuspension entsteht. Diese kann bei -20°C aufbewahrt werden.

### b) Herstellung einer Stammsuspension von Ratten-Aß(1-42)

2 mg Ratten-β-Amyloid(1-42)-Protein (kurz: Ratten-Aß(1-42); peptidsynthetisches Material, Lyophilisat, Fa. Bachem, Deutschland) werden in 800 µl 1,1,1,3,3,3-Hexafluor-2-propanol gelöst und 30 min bei 37°C in einem Eppendorfgefäß inkubiert. Danach wird im Vakuumkonzentrator (Speed Vac) zur Trockne eingedampft. Der Rückstand wird mit 88 µl DMSO aufgenommen, wobei eine 5 mM Ratten-Aß(1-42)-Stammsuspension entsteht. Diese kann bei -20°C aufbewahrt werden.

### Beispiel 2

### a) Herstellung von Aβ(1-42)-Oligomeren mit Molekulargewichten von 15 kDa and 20 kDa [Aβ(1-42)-Oligomere A]; Verwendung von SDS

60 µl der Stammlösung aus Beispiel 1a werden mit 690 µl PBS-Puffer (20 mM Natriumphosphat, 140 mM NaCl, pH 7,4) versetzt und mit 75 µl 2%iger Natriumdodecylsulfat (SDS)-Lösung auf einen Gehalt von 0,2 % SDS eingestellt. Danach wird 5 Stunden bei 37°C inkubiert und 10 min bei 10.000 g zentrifugiert. Diese Aβ(1-42)-Oligomer A-Präparation (ca. 400 µM Aβ(1-42)) kann bei -20°C aufbewahrt werden.

### b) Herstellung von Ratten-Aβ(1-42)-Oligomeren mit Molekulargewichten von 15 kDa and 20 kDa [Ratten-Aβ(1-42)-Oligomere A]; Verwendung von SDS

60 µl der Stammlösung aus Beispiel 1 b werden mit 690 µl PBS-Puffer (20 mM Natriumphosphat, 140 mM NaCl, pH 7,4) versetzt und mit 75 µl 2%iger Natriumdodecylsulfat (SDS)-Lösung auf einen Gehalt von 0,2 % SDS eingestellt. Danach wird 5 Stunden bei 37°C inkubiert und 10 min bei 10.000 g zentrifugiert. Diese Ratten-Aß(1-42)-Oligomer A-Präparation (ca. 400 µM Ratten-Aβ(1-42)) kann bei -20°C aufbewahrt werden.

### c) Herstellung von Aβ(1-42)-Oligomeren mit Molekulargewichten von 15 kDa und 20 kDa [Aβ(1-42)-Oligomere A]; Verwendung von Laurylsäure

60 µl der Stammlösung aus Beispiel 1 a werden mit 690 µl PBS-Puffer (20 mM Natriumphosphat, 140 mM NaCl, pH 7,4) versetzt und mit 75 µl 5%iger Laurylsäure-Lösung auf einen Gehalt von 0,5 % Laurylsäure eingestellt. Danach wird 5 Stunden bei 37°C inkubiert und 10 min bei 10.000 g zentrifugiert. Diese Aβ(1-42)-Oligomer A-Präparation (ca. 400 µM Aβ(1-42)) kann bei -20°C aufbewahrt werden.

### d) Herstellung von Aβ(1-42)-Oligomeren mit Molekulargewichten von 15 kDa and 20 kDa [Aβ(1-42)-Oligomere A]; Verwendung von Ölsäure

60 µl der Stammlösung aus Beispiel 1 a werden mit 690 µl PBS-Puffer (20 mM Natriumphosphat, 140 mM NaCl, pH 7,4) versetzt und mit 75 µl 5%iger Ölsäure-Lösung auf einen Gehalt von 0,5 % Ölsäure eingestellt. Danach wird 5 Stunden bei 37°C inkubiert und 10 min bei 10.000 g zentrifugiert. Diese Aβ(1-42)-Oligomer A-Präparation (ca. 400 µM Aβ(1-42)) kann bei -20°C aufbewahrt werden.

### e) Herstellung aon Aβ(1-42)-Oligomeren mit Molekulargewichten von 15kDa und 20 kDa [Aβ(1-42)-Oligomere A]; Verwendung von Lauroylsarcosin

60 µl der Stammlösung aus Beispiel 1 a werden mit 690 µl PBS-Puffer (20 mM Natriumphosphat, 140 mM NaCl, pH 7,4) versetzt und mit 75 µl 5%iger-Lauroylsarcosin-Lösung auf einen Gehalt von 0,5 % Lauroylsarcosin eingestellt. Danach wird 5 Stunden bei 37 °C inkubiert und 10 min bei 10.000 g zentrifugiert. Diese Aβ(1-42)-Oligomer A-Präparation (ca. 400 µM Aβ(1-42)) kann bei -20 °C aufbewahrt werden.

### Beispiel 3

### a) Alternatives Herstellverfahren zur Herstellung von Aβ(1-42)-Oligomeren mit Molekulargewichten von 15 kDa and 20 kDa [Aβ(1-42)-Oligomere A]

1 mg humanes β-Amyloid(1-42)-Protein (kurz: Aβ(1-42); peptidsynthetisches Material, Lyophilisat, Fa. Bachem, Deutschland) werden in 220 µl 10 mM wässriger HCl-Lösung aufgenommen und 10 min bei Raumtemperatur inkubiert. Unlösliche Bestandteile werden bei 10.000 g, 5 min abzentrifugiert. Der Überstand (1 mM Aβ(1-42)) enthält das Aβ(1-42)-Protein und wird wie folgt weiterverarbeitet:

1 µl des Überstandes werden mit 9 µl PBS-Puffer und 1 µl 2%iger SDS-Lösung versetzt und 16 h bei 37°C inkubiert. Die so erhaltene hAβ(1-42)-Oligomer A-Präparation (100 µM) kann bei -20°C aufbewahrt werden.

### b) Alternatives Herstellverfahren zur Herstellung von Ratten-Aß(1-42)-Oligomeren mit Molekulargewichten von 15 kDa and 20 kDa [Ratten-Aß(1-42)-Oligomere A]

1 mg Ratten-β-Amyloid(1-42)-Protein (kurz: Ratten-Aß(1-42); peptidsynthetisches Material, Lyophilisat, Fa. Bachem, Deutschland) werden in 220 µl 10 mM wässriger HCl-Lösung aufgenommen und 10 min bei Raumtemperatur inkubiert. Unlösliche Bestandteile werden bei 10.000 g, 5 min abzentrifugiert. Der Überstand (1 mM Ratten-Aβ(1-42)) enthält das Ratten-Amyloid β(1-42)-Protein und wird wie folgt weiterverarbeitet:

1 µl des Überstandes werden mit 9 µl PBS-Puffer und 1 µl 2%iger SDS-Lösung versetzt und 16 h bei 37°C inkubiert. Die so erhaltene Ratten-Aβ(1-42)-Oligomer A-Präparation (100 µM) kann bei -20°C aufbewahrt werden.

### Beispiel 4

### a) Alternatives Herstellverfahren zur Herstellung von Aβ(1-42)-Oligomeren mit Molekulargewichten von 15 kDa und 20 kDa [Aß(1-42)-Oligomere A]

1 mg humanes β-Amyloid(1-42)-Protein (kurz: Aβ(1-42); peptidsynthetisches Material, Lyophilisat, Fa. Bachem, Deutschland) werden in 44 µl 1% SDS/H₂O gelöst (5 mM Aβ(1-42)). 5 µl der Lösung werden mit 40 µl PBS und 5 µl 2% SDS versetzt und 16 h bei 37°C inkubiert. Unlösliche Bestandteile werden 5 min bei 10.000 g abzentrifugiert. Die so erhaltene Aβ(1-42)-Oligomer A-Präparation (500 µM Aβ(1-42)) kann bei -20°C aufbewahrt werden.

### b) Alternatives Herstellverfahren zur Herstellung von Ratten-Aß(1-42)-Oligomeren mit Molekulargewichten von 15 kDa und 20 kDa [Ratten-Aß(1-42)-Oligomere A]

1 mg Ratten-β-Amyloid(1-42)-Protein (kurz: Ratten-Aß(1-42); peptidsynthetisches Material, Lyophilisat, Fa. Bachem, Deutschland) werden in 44 µl 1% SDS/H₂O gelöst (5 mM Ratten-Aß(1-42)). 5 µl der Lösung werden mit 40 µl PBS und 5 µl 2% SDS versetzt und 16 h bei 37°C inkubiert. Unlösliche Bestandteile werden 5 min bei 10.000 g abzentrifugiert. Die so erhaltene Ratten-Aß(1-42)-Oligomer A-Präparation (500 µM Ratten-Aβ(1-42)) kann bei -20°C aufbewahrt werden.

### Beispiel 5

### a) Herstellung von Aβ(1-42)-Oligomeren mit Molekulargewichten von 38 kDa und 48 kDa [Aß(1-42)-Oligomere B]

Eine gemäß Beispiel 2a erhaltene Aβ(1-42)-Oligomer A-Lösung wird mit 2,475 ml Wasser verdünnt (0,05% SDS-Gehalt, 0,1 mM Aβ(1-42)) und 20 Stunden bei 37°C inkubiert. Aliquots dieser Aβ(1-42)-Oligomer B-Präparation können bei -80°C eingefroren und für weitere Untersuchungen gelagert werden.

### b) Herstellung von Ratten-Aβ(1-42)-Oligomeren mit Molekulargewichten von 38 kDa und 48 kDa [Ratten-Aß(1-42)-Oligomere B]

Eine gemäß Beispiel 2b erhaltene Ratten-Aβ(1-42)-Oligomer A-Lösung wird mit 2,475 ml Wasser verdünnt (0,05% SDS-Gehalt, 0,1 mM Ratten-Aß(1-42)) und 20 Stunden bei 37°C inkubiert. Aliquots dieser Ratten-Aβ(1-42)-Oligomer B-Präparation können bei -80°C eingefroren und für weitere Untersuchungen gelagert werden.

### c) Alternative Herstellung von Aβ(1-42)-Oligomeren mit Molekulargewichten von 38 kDa und 48 kDa [Aβ(1-42)-Oligomere B]

Eine gemäß Beispiel 2c erhaltene Aβ(1-42)-Oligomer A-Lösung wird mit 2,475 ml Wasser verdünnt (0,125% Laurylsäure-Gehalt, 0,1 mM Aβ(1-42)) und 20 Stunden bei 37°C inkubiert. Aliquots dieser Aβ(1-42)-Oligomer B-Präparation können bei - 80°C eingefroren und für weitere Untersuchungen gelagert werden.

### d) Alternative Herstellung von Aβ(1-42)-Oligomeren mit Molekulargewichten von 38 kDa und 48 kDa [Aβ(1-42)-Oligomere B]

Eine gemäß Beispiel 2d erhaltene Aβ(1-42)-Oligomer A-Lösung wird mit 2,475 ml Wasser verdünnt (0,125% Ölsäure-Gehalt, 0,1 mM Aβ(1-42)) und 20 Stunden bei 37°C inkubiert. Aliquots dieser Aβ(1-42)-Oligomer B-Präparation können bei -80°C eingefroren und für weitere Untersuchungen gelagert werden.

### e) Alternative Herstellung von Aβ(1-42)-Oligomeren B mit Molekulargewichten von 38 kDa und 48 kDa [Aβ(1-42)-Oligomere B]

Eine gemäß Beispiel 2e erhaltene Aβ(1-42)-Oligomer-A-Lösung wird mit 2,475 ml Wasser verdünnt (0,125 % Laurylsarcosin-Gehalt, 0,1 mM Aβ(1-42) )und 20 Stunden bei 37°C inkubiert. Aliquots dieser Aβ(1-42)-Oligomer B-Präparation können bei -80 °C eingefroren und für weitere Untersuchungen gelagert werden.

### f) Herstellung von SDS-freien Aβ(1-42)-Oligomeren B mit Molekulargewichten von 38 kDa und 48 kDa [Aβ(1-42)-Oligomere B]

10 µl einer nach Beispiel 6b hergestellten Aβ(1-42)-Oligomeren B-Präparation werden mit 250 µl eines Essigsäure/Methanol/Wasser-Gemisches im Verhältnis 4 % / 33 % / 63 % versetzt und 30 min bei 0 °C auf Eis inkubiert. Nach Zentrifugation (10 min bei 10.000 g) wird der Überstand abgenommen und der gefällte Proteinrückstand in 200 µl Puffer (20 mM Na-Phosphat, 140 mM NaCl, pH 7,4) aufgenommen. Die so erhaltene Präparation enthält die gelösten Aβ(1-42)-Oligomeren B in SDS-freier Form und kann bei -20°C gelagert werden.

### Beispiel 6

### a) Dialyse und Aufkonzentration von Aβ(1-42)-Oligomeren mit Molekulargewichten von 38 kDa und 48 kDa [Aβ(1-42)-Oligomere B]

Eine gemäß Beispiel 5a hergestellte Aβ(1-42)-Oligomer B-Präparation wird mit 30 ml PBS-Puffer, der 0,1 % Pluronic® F68 (Fa. BASF) enthält, versetzt und im Centriprep YM, 30 KD, der Fa. Amicon auf 3 ml konzentriert. Eventuelle Rückstände werden durch Zentrifugation (5 min bei 10000 g) entfernt. Der Überstand wird entnommen. Aliquots dieser Aβ(1-42)-Oligomer B-Präparation können bei -80°C eingefroren und für weitere Untersuchungen gelagert werden.

### b) Herstellung eines Konzentrats von Aβ(1-42)-Oligomeren B mit Molekulargewichten von 38 kDa und 48 kDa [Aβ(1-42)-Oligomere B]

72,6 ml einer gemäß Beispiel 5a erhaltenen Aß(1-42)-Oligomeren B-Präparation werden über ein 30 KD-Centriprep-Röhrchen YM (Amicon) auf 2 ml aufkonzentriert. Das Konzentrat wird 10 min bei 10.000 g abzentrifugiert. Der Überstand wird abgenommen und 16 h bei 6°C im Dialyseschlauch gegen 1 l Puffer (5 mM Natriumphosphat, 35 mM Natriumchlorid, pH 7.4) dialysiert. Das Dialysat wird 10 min bei 10.000 g abzentrifugiert Der Überstand wird abgenommen und kann für weitere Untersuchungen bei -80°C gelagert werden.

### Beispiel 7

### Herstellung von Biotin-Aβ(1-42)-Stammsuspension

0,5 mg Biotin-β-Amyloid(1-42)-Protein (kurz: Biotin-Aβ(1-42); peptidsynthetisches Material, Lyophilisat, AnaSpec) werden in 200 µl 1,1,1,3,3,3-Hexafluor-2-propanol gelöst und 30 min bei 37°C in einem Eppendorfgefäß inkubiert. Danach wird im Vakuumkonzentrator (Speed Vac) zur Trockne eingedampft. Der Rückstand wird mit 20,5 µl DMSO aufgenommen, wobei eine 5 mM Biotin-Aβ(1-42)-Stammsuspension entsteht. Diese kann bei -20°C aufbewahrt werden.

### Beispiel 8

### Herstellung von Biotin-Aβ(1-42)-Oligomeren mit Molekulargewichten von 17 kDa und 22 kDa [Biotin-Aß(1-42)-Oligomere A]

2 µl der Stammsuspension aus Beispiel 7 werden mit 23 µl PBS-Puffer (20 mM Natriumphosphat, 140 mM NaCl, pH 7,4) versetzt und mit 2,4 µl 2%iger SDS-Lösung auf einen Gehalt von 0,2 % SDS eingestellt. Danach wird 6 Stunden bei 37°C inkubiert. Unlösliche Bestandteile werden bei 10.000 g, 5 min abzentrifugiert.Die so erhaltene Biotin-Aβ(1-42)-Oligomer A-Präparation kann bei -20°C aufbewahrt werden.

### Beispiel 9

### Herstellung von Biotin-Aβ(1-42)-Oligomeren mit Molekulargewichten von 42 kDa und 52 kDa [Biotin-Aβ(1-42)-Oligomere B]

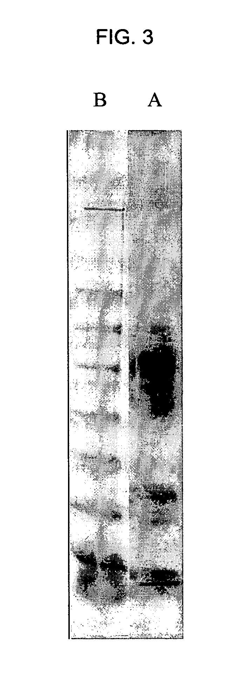

Eine gemäß Beispiel 8 erhaltene Biotin-Aβ(1-42)Oligomer A-Lösung wird mit 82 µl Wasser verdünnt (0,05% SDS-Gehalt, 0,1 mM Aβ) und 16 Stunden bei 37°C inkubiert. Unlösliche Bestandteile werden bei 10.000 g, 5 min abzentrifugiert. Die BiotinAβ(1-42)-Oligomer B-Präparation kann bei -20°C eingefroren und für weitere Untersuchungen gelagert werden (Fig. 3).

### Beispiel 10

### Herstellung von Fluorescein-Aß(1-42)-Stammsuspension

0,5 mg Fluorescein-β-Amyloid(1-42)-Protein (kurz: Fluorescein-Aß(1-42); peptidsynthetisches Material, Lyophilisat, AnaSpec) werden in 200 µl 1,1,1,3,3,3-Hexafluor-2-propanol gelöst und 30 min bei 37°C in einem Eppendorfgefäß inkubiert. Danach wird im Vakuumkonzentrator (Speed Vac) zur Trockne eingedampft. Der Rückstand wird mit 20,5 µl DMSO aufgenommen, wobei eine 5 mM Fluorescein-Aβ(1-42)-Stammsuspension entsteht. Diese kann bei -20°C aufbewahrt werden.

### Beispiel 11

### Herstellung von Fluorescein-Aβ(1-42)-Oligomeren mit den Molekulargewichten 17 kDa and 22 kDa [Fluorescein-Aβ(1-42)-Oligomer A]

2 µl der Stammsuspension aus Beispiel 10 werden mit 23 µl PBS-Puffer (20 mM Natriumphosphat, 140 mM NaCl, pH 7,4) versetzt und mit 2,4 µl 2%iger SDS-Lösung auf einen Gehalt von 0,2 % SDS eingestellt. Danach wird 6 Stunden bei 37°C inkubiert. Unlösliche Bestandteile werden bei 10.000 g, 5 min abzentrifugiert. Die so erhaltene Fluorescein-Aß(1-42)Oligomer A-Präparation kann bei -20°C aufbewahrt werden.

### Beispiel 12

### Herstellung von Fluorescein-Aβ(1-42)-Oligomeren mit Molekulargewichten von 42 kDa and 52 kDa [Fluorescein-Aβ(1-42)-Oligomere B]

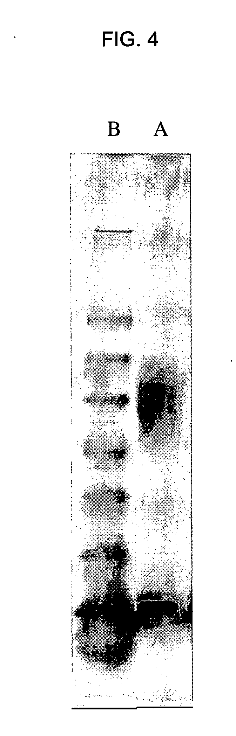

Eine gemäß Beispiel 11 erhaltene Fluorescein-Aβ(1-42)-Oligomer A-Lösung wird mit 82 µl Wasser verdünnt (0,05% SDS-Gehalt, 0,1 mM Aβ(1-42)) und 16 Stunden bei 37°C inkubiert. Die Fluorescein-Aβ(1-42)-Oligomer B-Präparation kann bei -80°C eingefroren und für weitere Untersuchungen gelagert werden (Fig. 4).

### Beispiel 13

### Quervernetzung von Aβ(1-42)-Oligomeren A aus Beispiel 2a [Aβ(1-42)-Oligomere A-CL]

10 µl einer gemäß Beispiel 2a hergestellten Aβ(1-42)-Oligomeren A-Lösung werden mit 7,5 µl PBS, 0,2 % SDS auf einen 100 µM Aß(1-42)-Gehalt verdünnt. Zu dieser Lösung werden 1 µl 10 mM frisch angesetzter Glutardialdehyd-Lösung in Wasser gegeben, und es wird 3 h bei RT gerührt. Zur Absättigung des überschüssigen Glutardialdehyds wird die Probe mit 1 µl 100 mM Ethanolamin-Lösung in Wasser, pH 7,4, versetzt und 1 h gerührt. Die so erhaltene Präparation enthält quervernetzte Aβ(1-42)-Oligomere A und wird mit Aβ(1-42)-Oligomer A-CL-Präparation bezeichnet.

### Beispiel 14

### a) Quervernetzung von Aβ(1-42)-Oligomeren B aus Beispiel 5a [Aβ(1-42)-Oligomere B-CL]

10 µl einer gemäß Beispiel 5a hergestellten Aβ(1-42)-Oligomeren B-Lösung werden mit 1 µl 10 mM frisch angesetzter Glutardialdehyd-Lösung in Wasser versetzt und 3 h bei RT gerührt. Zur Absättigung des überschüssigen Glutardialdehyds wird die Probe mit 1 µl 100 mM Ethanolamin-Lösung in Wasser, pH 7,4, versetzt und 1 h gerührt. Die so erhaltene Präparation enthält quervernetzte Aβ(1-42)-Oligomere B und wird mit Aß(1-42)-Oligomer B-CL-Präparation bezeichnet.

### b) Alternative Vorgehensweise zur Quervernetzung von Aβ(1-42)-Oligomeren [Aβ(1-42)-Oligomere B-CL]

72,6 ml einer nach Beispiel 5a hergestellten Aβ(1-42)-Oligomeren B-Lösung werden mit 7,26 ml frisch angesetzter 10 mM Glutardialdehyd-Lösung in Wasser versetzt und 2 h bei RT gerührt. Zur Absättigung des überschüssigen Glutardialdehyds wird die Probe mit 726 µl Puffer(20 mM Na-Phosphat, 140 mM NaCl, 500 mM Ethanolamin, pH 7,4) versetzt und 30 min bei RT gerührt. Der Ansatz wir über ein 15 ml 30 kDa-Centriprep-Röhrchen auf 3 ml aufkonzentriert. Das Konzentrat wird 10 min bei 10.000 g abzentrifugiert. Der Überstand wird abgenommen und 16 h bei 6 °C im Dialyseschlauch gegen 1 l 5 mM Na-Phosphat, 35 mM NaCl, ph 7,4 dialysiert. Danach wird das Dialysat 10 min bei 10.000 g abzentrifugiert und der Überstand abgenommen. Der Überstand kann für weitere Untersuchungen bei -80°C gelagert werden. Die so erhaltene Präparation enthält quervernetzte Aβ(1-42)-Oligomere B und wird mit Aβ(1-42)-Oligomer B-CL-Präparation bezeichnet.

### Beispiel 15

### a) Herstellung von verkürzten Aβ(20-42)-Oligomeren ausgehend von Aβ(1-42)-Oligomeren B durch Spaltung mit Thermolysin:

1,59 ml einer nach Beispiel 6b hergestellten Aβ(1-42)-Oligomeren B-Präparation werden mit 38 ml Puffer (50 mM MES/NaOH, pH 7,4) und 200 µl einer 1 mg/ml Thermolysin-Lösung (Fa. Roche) in Wasser versetzt. Der Ansatz wird 20 h bei RT gerührt. Danach werden 80 µl einer 100 mM EDTA-Lösung, pH 7,4, in Wasser zugegeben und des Weiteren mit 400 µl einer 1 %igen SDS-Lösung auf einen SDS-Gehalt von 0,01 % eingestellt. Der Ansatz wird über ein 15 ml 30 kDa-Centriprep-Röhrchen auf ca. 1 ml aufkonzentriert. Das Konzentrat wird mit 9 ml Puffer (50 mM MES/NaOH, 0,02 % SDS, pH 7,4) versetzt und wieder auf 1 ml aufkonzentriert. Das Konzentrat wird 16 h bei 6°C gegen 1 l Puffer (5 mM Na-Phosphat, 35 mM NaCl) im Dialyseschlauch dialysiert. Das Dialysat wird mit einer 2%ige SDS-Lösung in Wasser auf einen SDS-Gehalt von 0,1 % eingestellt. Die Probe wird 10 min bei 10.000 g abzentrifugiert und der Überstand abgenommen.

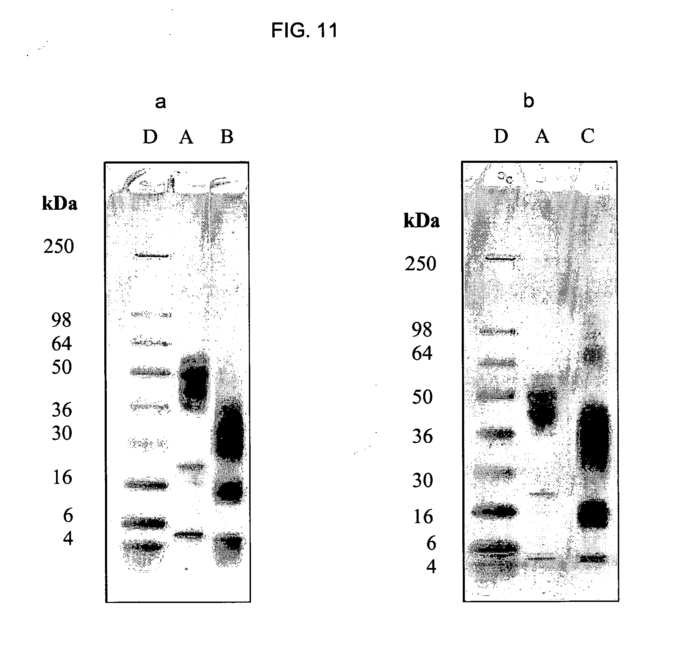

Das so erhaltene Material wird weiter analysiert (SDS-Polyacrylamidgelelektrophorese; vgl. Figur 11); Die massenspektrometrische Analyse des entstandenen verkürzten Oligomers ergibt, dass das Oligomer sich aus verkürztem Aβ(20-42) zusammensetzt.

### b) Herstellung von verkürzten Aβ(12-42)-Oligomeren ausgehend von Aβ(1-42)-Oligomeren B durch Spaltung mit Endoproteinase GluC:

2 ml einer nach Beispiel 6b hergestellten Aß(1-42)-Oligomere B-Präparation werden mit 38 ml Puffer (5 mM Natriumphosphat, 35 mM Natriumchlorid, pH 7,4) und 150 µl einer 1 mg/ml Endoproteinase GluC (Fa. Roche) in Wasser versetzt. Der Ansatz wird 6 h bei RT gerührt. Anschließend werden nochmals 150 µl einer 1mg/ml Endoproteinase GluC (Fa. Roche) in Wasser zugesetzt. Der Ansatz wird weitere 16 h bei RT gerührt. Danach werden 8 µl einer 5 M DIFP-Lösung zugegeben. Der Ansatz wird über ein 15 ml 30 kDa-Centriprep-Röhrchen auf ca. 1 ml aufkonzentriert. Das Konzentrat wird mit 9 ml Puffer (5 mM Natriumphosphat, 35 mM Natriumchlorid, pH 7,4) versetzt und wieder auf 1 ml aufkonzentriert. Das Konzentrat wird 16 h bei 6°C gegen 1 l Puffer (5 mM Na-Phosphat, 35 mM NaCl) im Dialyseschlauch dialysiert. Das Dialysat wird mit einer 1 %igen SDS-Lösung in Wasser auf einen SDS-Gehalt von 0,1 % eingestellt. Die Probe wird 10 min bei 10.000 g abzentrifugiert und der Überstand abgenommen.

Das so erhaltene Material wird weiter analysiert (SDS-Polyacrylamidgelelektrophorese; vgl. Figur 11). Die massenspektrometrische Analyse des entstandenen verkürzten Oligomers ergibt, dass das Oligomer sich aus verkürztem Aβ(12-42) zusammensetzt.

### Charakterisierung von Aß(1-42)-Oligomeren

### Beispiel 16

### SDS-Polyacrylamidgelelektrophorese (SDS-PAGE)

Zur Charakterisierung des Molekulargewichtes unter denaturierenden Bedingungen werden die Oligomere A und B enthaltenden Präparationen aus den Beispielen 2a, 5a, 9, 12, 13 und 14a unter denaturierenden Bedingungen nach Standardbedingungen in einer 4 - 20%igen Tris-Glycin-SDS-PAGE analysiert.

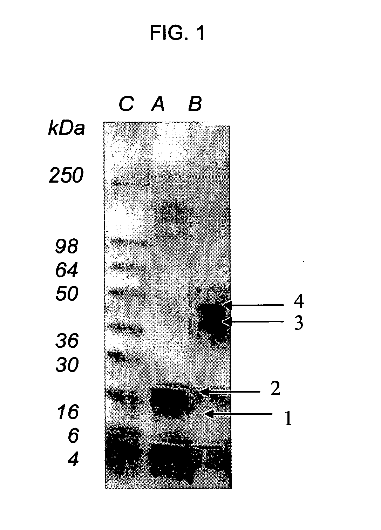

Die Auswertung der SDS-PAGE (Fig. 1) ergibt, dass in der Oligomer A-Präparation noch vorhandenes Ausgangsprotein Aβ(1-42) als Bande bei etwa 4 kDa erscheint, während bei etwa 15 kDa (schwächere Bande) und bei etwa 20 kDa (Hauptbande) die Oligomere A1 bzw. A2 aus Beispiel 2a, die in der Fig. 1 mit 1 und 2 bezeichnet sind, zu sehen sind.

In der Oligomer B-Präparation ist vergleichsweise wenig Ausgangsprotein Aβ(1-42) nachzuweisen (relativ schwache Bande bei etwa 4 kDa). Hingegen erscheinen bei etwa 38 kDa und etwa 48 kDa die Oligomere B1 bzw. B2 aus Beispiel 5a, die in der Fig. 1 mit 3 und 4 bezeichnet sind (siehe Pfeile). Für die mit Biotin und Fluorescein derivatisierten Oligomere B aus den Beispielen 9 und 12 ergeben sich mit etwa 42 kDa und etwa 52 kDa entsprechend höhere Molekulargeichte (Fig. 3, 4).

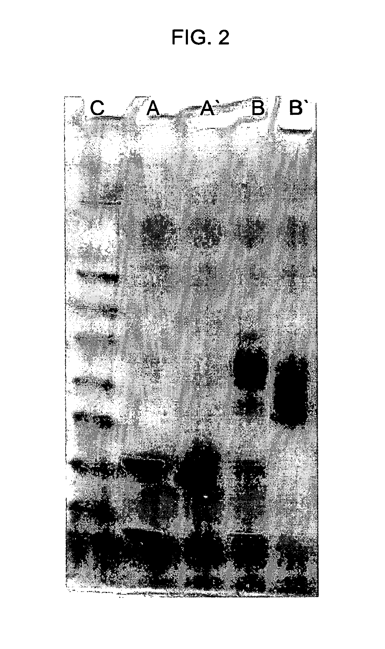

Die Analyse der die jeweiligen Quervernetzungsprodukte A-CL und B-CL enthaltenden Präparationen (Beispiele 13 und 14a; Fig. 2) zeigt, dass die beiden Proben ihren Oligomerisierungsgrad (vgl. Bahn A mit A' und Bahn B mit B') im wesentlichen beibehalten. Das zu den Oligomeren A und B leicht unterschiedliche Laufverhalten lässt sich durch eine leicht veränderte SDS-Bindungskapazität und durch Modifikation der Aminogruppen des N-Terminus bzw des Lysinrestes erklären.

Die Analyse des verkürzten Aβ(20-42)-Oligomeren (aus Beispiel 15a) zeigt, dass die 38/48 kDa Doppelbande (Fig. 11, Bahn A) durch die proteolytische Abspaltung des N-terminalen Peptids mit Thermolysin in eine 28/38 kDa Doppelbande (Fig. 11; Bahn B) überführt wird. Analog zeigt die Analyse des verkürzten Aβ(12-42)-Oligomeren (aus Beispiel 15b), dass die 38/48 kDa Doppelbande (Fig. 11; Bahn A) durch die proteolytische Abspaltung des N-terminalen Peptids mit Endoprotease Glu-C in eine 33/40 kDa Doppelbande (Fig. 11; Bahn C) überführt wird.

### Beispiel 17

### Gelpermeationschromatographie

Um das Molekulargewichtsverhalten unter nicht denaturierenden Bedingungen näher zu untersuchen, wird eine Gelpermeationschromatographie (GPC) im FPLC-Verfahren mit einer Superose 12 HR10/30 Säule durchgeführt. Die GPC wird bei 4°C gefahren.

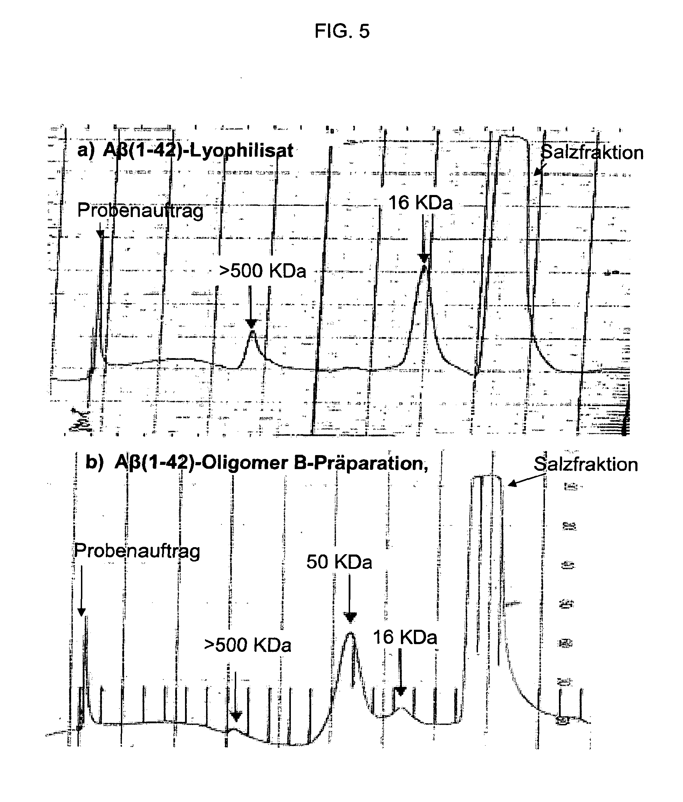

Die Säule wird mit 5 Säulenvolumina PBS-Puffer äquilibriert (Fluß 0,5 ml/min, Detektion UV, 214 nm) und zunächst mit Eichproteinen kalibriert. Anschließend wird die Aβ(1-42)-Oligomer B-Präparation aus Beispiel 5a (Fig. 5 unten) und zum Vergleich in gleicher Konzentration frisch eingewogenes und in PBS gelöstes Aβ(1-42)-Lyophilisat nach 5-minütigem Abzentrifugieren der unlöslichen Bestandteile bei 10.000 g analysiert (Fig. 5 oben).

Die Auswertung ergibt, dass die Aβ(1-42)-Oligomer B-Präparation eine durch einen Hauptpeak im Molekulargewichtsbereich um etwa 50 kDa gekennzeichnete Proteinfraktion aufweist. Wie unter denaturierenden Bedingungen in der SDS-PAGE, unterscheidet sich diese Proteinfraktion signifikant von dem durch einen Hauptpeak im Molekulargewichtsbereich um etwa 16 kDa gekennzeichneten monomeren Aβ(1-42)-Protein.

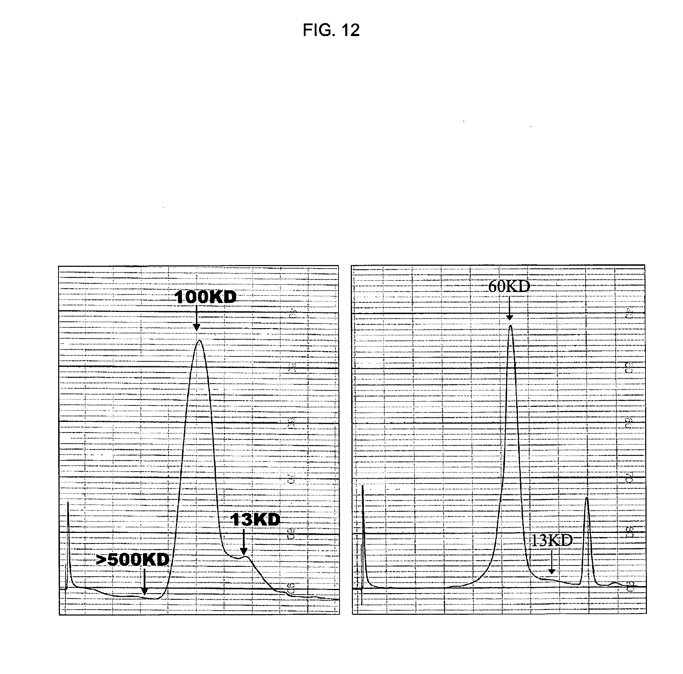

Um das Molekulargewichtsverhalten unter nicht denaturierenden Bedingungen von Aβ(1-42)-Oligomeren B aus Beispiel 6b sowie von Aβ(1-42)-Oligomeren B-CL aus Beispiel 14a zu untersuchen, wird eine Gelpermeationschromatografie (GPC) im FPLC-Verfahren mit einer Superose 12 HR10/30-Säule bei Raumtemperatur durchgeführt. Die Säule wird mit 5 Säulenvolumina PBS-Puffer äquilibriert (Fluss 0,5 ml/min, Detektion UV, 214 nm) und zunächst mit Eichproteinen kalibriert. Anschließend wird Aβ(1-42)-Oligomer B aus Beispiel 6b mit PBS Puffer auf 1 mg/ml verdünnt sowie Aβ(1-42)-Oligomer B-CL aus Beispiel 14a mit PBS-Puffer auf 1 mg/ml verdünnt und analysiert (Fig. 12).

Die Auswertung ergibt, dass die Aβ(1-42)-Oligomer B--Präparation mit verringertem SDS-Gehalt eine durch einen Hauptpeak im Molekulargewichtsbereich um etwa 100 kDa gekennzeichnete Proteinfraktion aufweist. Vergleichend dazu weist die Aβ(1-42)-Oligomer B-CL-Präparation mit verringertem SDS-Gehalt eine durch einen Hauptpeak im Molekulargewichtsbereich um etwa 60 kDa gekennzeichnete Proteinfraktion auf.

### Beispiel 18

### Native Polyacrylamidgelelekrophorese (NATIVE-PAGE) von Aβ(1-42)- Oligomeren B aus Beispiel 5a

Zur Charakterisierung des Molekulargewichtes unter nativen Bedingungen werden die Oligomere B enthaltende Präparationen aus Beispiel 5a unter nicht denaturierenden Bedingungen in einem 4-20% Tris-Glycin-Gel analysiert.

Das in den Präparationen enthaltende Detergenz wird durch das nicht ionische Detergenz Triton X-100 neutralisiert. Hierzu werden 1 µl (4%Triton X-100) zu 10 µl der Präparation aus Beispiel 5a pipettiert und 5 min bei Raumtemperatur inkubiert. Danach werden 10 µl mit gleichem Volumen an nativem Probenpuffer (4 ml 1M Tris, pH 6,8, 8 ml Glycerin, 1 ml Bromphenolblau in 50 ml H₂O) versetzt und die Elektrophorese (Laufpuffer:7,5 g Tris, 36 g Glycin auf 2,5 l H₂O) durchgeführt.

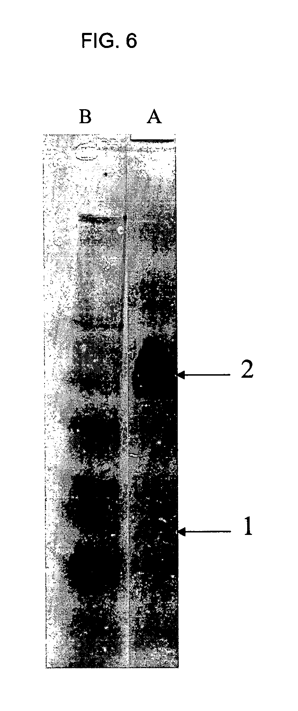

Die Auswertung der NATIVE-PAGE ergibt, dass in der Oligomer B-Präparation noch vorhandenes Ausgangspeptid Aβ(1-42) als Bande bei etwa 28 kDa erscheint (Bahn A, mit 1 bezeichnet), während bei einem apparenten Molekulargewicht von 64-90 kDa (Bahn A, mit 2 bezeichnet) die Hauptbanden der Präparation aus Beispiel 5a zu erkennen sind (Figur 6).

Es ist von Bedeutung, dass den erfindungsgemäßen Oligomeren, insbesondere den Oligomeren B, auch in nativen Molekulargewichts-Analyseverfahren wie der Gelpermeationschromatographie (siehe Beispiel 17) oder der nativen Gelelektrophorese (siehe Beispiel 18) Molekulargewichte zugeordnet werden können.

Die Oligomere B, denen in der SDS-PAGE Molekulargewichte von etwa 38 und 48 kDa zugeordnet werden können, werden nach dem Komplexieren des SDS in der nativen Gelelektrophorese als Bande im Molekulargewichtsbereich von etwa 64 - 90 kDa bezogen auf ausgewählte Standardproteine detektiert. Es ist nach diesem Verfahren nicht zu erwarten, dass ein exaktes Molekulargewicht abzulesen ist, da das Laufverhalten der Oligomere durch ihre Eigenladungen neben der Größe wesentlich bestimmt wird. Es kann allerdings aus dem Ergebnis geschlossen werden, dass eine definierte oligomere Spezies vorliegt.

### Beispiel 19

### a) Stabilität von Aβ(1-42)-Oligomeren B bei verschiedenen Proteinkonzentrationen in physiologischen Puffern

Die nach Beispiel 6b erhaltenen Aβ(1-42)-Oligomere B werden unter folgenden Bedingungen auf Stabilität in PBS-Puffer getestet.

5 mg/ml werden mit PBS in 2er Verdünnungsschritten bis auf 0,08 mg/ml verdünnt. Alle erhaltenen Lösungen werden 24 Stunden bei Raumtemperatur inkubiert. Im Anschluß wurde das Bandenmuster in einer SDS-PAGE im Vergleich zu einer eingefrorenen Kontrollpräparation analysiert. Das Bandenmuster ist in allen Proben identisch.

### b) Stabilität von Aβ(1-42)-Oligomeren B bei verschiedenen Temperaturen und nach verschiedenen Zeiten in physiologischen Puffern

Die nach Beispiel 6b erhaltenen Aβ(1-42)-Oligomere B werden mit PBS-Puffer auf 0,5 mg/ml verdünnt und 24 h oder 96 h bei Raumtemperatur oder bei 37 °C inkubiert. Im Anschluss wurde das Bandenmuster in einer SDS-PAGE analysiert. Das Bandenmuster ist in allen Proben identisch.

### Beispiel 20

### Proteolyse von Aβ(1-42)-Oligomeren B mittels verschiedener Proteasen (Trypsin, Chymotrypsin, Thermolysin, Elastase, Papain)

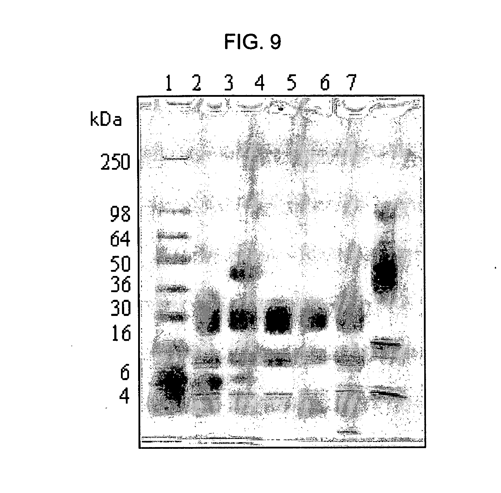

Aliquots der nach Beispiel 6b erhaltenen Aβ(1-42)-Oligomeren B-Präparation werden mit Puffer (20 mM Na-Phosphat, 140 mM NaCl, pH 7,4) auf 0,5 mg/ml verdünnt und unter folgenden Bedingungen jeweils mit 1/50 der Gewichtsmenge der in Fig. 9 angegebenen Proteaselösungen 20 h bei 37°C und pH 7,4 inkubiert. 1 µg Aliquots der Ansätze werden danach in einer SDS-PAGE analysiert (Fig. 9).

Die SDS-PAGE zeigt, dass ausgehend von der Aβ(1-42)-Oligomere B-Präparation alle Proteasen unter den gewählten limitierten Proteolysebedingungen die Doppelbande bei 38/48 kDa auf eine kDa Doppelbande bei etwa 32/28 kDa zurückführen.

### Beispiel 21

### Stabilität von Aβ(1-42)-Oligomeren B in Rattenplasma

4 µl der nach Beispiel 6b hergestellten Aβ(1-42)-Oligomeren B-Präparation werden mit 76 µl Rattenplasma 0 h, 1 h, 2h, 4h und 8h bei Raumtemperatur inkubiert. Die Inkubationen werden durch Einfrieren in Trockeneis gestoppt.

Anschließend werden alle Proben nach SDS-Probenpuffer-Zugabe in einer SDS-PAGE analysiert. Danach erfolgt eine Auswertung der Stabilität über die Färbung der Aβ(1-42)-Oligomeren B im Western Blot. Zur Detektion wurde der gegen Aβ(1-42) gerichtete Antikörper 6E10 (Fa. Signet) eingesetzt. Die Banden werden durch einen an Alkalischer Phosphatase gekoppelten anti-Maus-IgG-Antikörper und Zugabe des Substrats NBT/BCIP sichtbar gemacht. Sowohl Molekulargewicht als auch Intensität des beobachteten Bandenmusters bleiben über die Zeit von 2h, 4h und 8 h nahezu unverändert.

Dieses Ergebnis lässt darauf schließen, dass die Aβ(1-42)-Oligomere B eine hohe Plasmastabilität aufweisen. Die biologische Halbwertszeit im Plasma liegt demnach im Bereich von etwa 8 Stunden oder länger.

### Beispiel 22

### Bindung von Biotin-Aß(1-42) Oligomeren mit Molekulargewichten von 17 kDa and 22 kDa [Biotin-Aβ(1-42)-Oligomere A] bzw. von 42 kDa and 52 kDa [Biotin-Aβ(1-42)-Oligomere B] an die Oberfläche humaner neuronaler Zellen

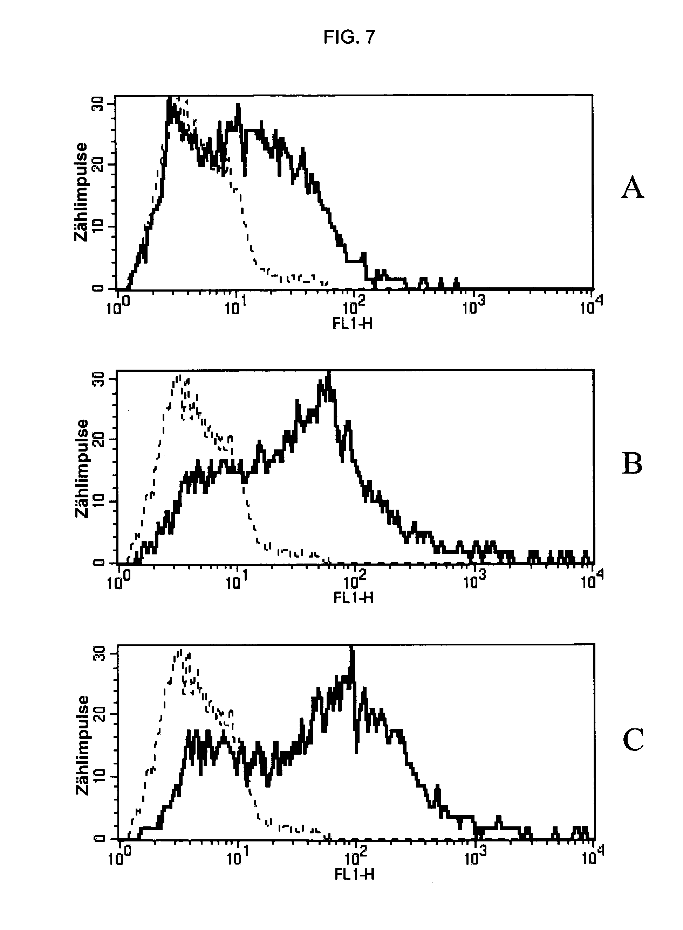

Die Bindung von humanem β-Amyloid(1-42)-Protein und den beiden Aβ(1-42)-Oligomer A- und Aβ(1-42)-Oligomer B-Präparationen an die humane Neuroblastoma-Zell-Linie IMR-32 (ATCC Number: CCL-127) wird mittels FACScan (Beckton Dickinson) untersucht. Eine Suspension von IMR-32 Zellen (1,5 x 10⁶Zellen/0,1 ml PBS) wird mit Biotin-markiertem, humanem β-Amyloid(1-42)-Protein (peptidsynthetisches Material, Lyophilisat, AnaSpec) und den die Biotin-markierte Oligomere A bzw. B enthaltenden Präparationen (Beispiel 8 bzw. 9) 20 Minuten bei 37°C inkubiert. Anschließend werden die Zellen mit Puffer (PBS plus 1 % BSA) gewaschen und mit an Streptavidin Isothiocyanate-gekoppeltem Fluorescein (Sigma) 20 Minuten bei Raumtemperatur inkubiert. Nach einem Waschschritt mit Puffer wurde die Bindung an die Oberfläche von IMR-32 Zellen durch FACScan analysiert. Die unterbrochene Linie zeigt die Hintergrundfluoreszenz in Abwesenheit der Biotinmarkierten Komponenten. Die Zugabe der einzelnen Präparationen führte zu einer starken Zunahme der Zell-assoziierten Fluoreszenz und ist durch die dicke Linie dargestellt. Die Bindung der Oligomeren A (Fig. 7B) und B (Fig . 7C) an die Zelloberflächen unterscheidet sich deutlich von der Bindung des monomeren β-Amyloid(1-42)-Proteins (Fig. 7A). Die Daten zeigen spezifische Bindungsstellen für die Oligomere auf humanen Zelloberflächen.

### Beispiel 23

### Nachweis von Aβ(1-42)-Oligomeren B in Rattengehirnhomogenaten nach icv-Applikation

10 nmol einer nach Beispiel 6b erhaltenen Aβ(1-42)-Oligomeren B-Präparation werden Ratten icv als Bolus appliziert. Die Gehirne werden nach 15 min bzw nach 120 min präpariert. Gehirne von nicht behandelten Kontrolltieren werden ebenfalls präpariert. Dazu werden jeweils 1 g Rattenhirn mit 9 ml Aufschlusspuffer A (200 ml 5 mM Natriumphosphat, 35 mM NaCl, 300 mM Sucrose, auf pH 7,4 eingestellt, werden mit 4 Tabletten des Proteaseinhibitor-Cocktails Complete®, Fa Roche, versetzt) in einem 50 ml Falconröhrchen versetzt und 2 min im Ultraschall (UltraTurrax) auf Eis aufgeschlossen. Die Lösung wird 20 min stehen gelassen, kurz geschüttelt und in 8 x 1 ml Aliquots aufgeteilt (= Homogenat).

Um den Nachweis quantitativ zu führen, wird zunächst eine Eichreihe der Aβ(1-42)-Oligomeren B-Präparation in PBS im Konzentrationsbereich von 1,58 ng/µl - 0,005 ng/µl angesetzt.

Des Weiteren wird als Positivkontrolle ein Homogenat aus einem Kontrollgehirn einer nicht behandelten Ratte mitgeführt und in gleicher Weise eine Eichreihe der Aβ(1-42)-Oligomeren B-Präparation in diesem Gehirnhomogenat angesetzt. Danach werden die Homogenate 1 h bei 100.000 g in der Ultrazentrifuge zentrifugiert und die Überstände für die nachfolgenden Analysen eingesetzt.

Aus dem Vergleich der an beiden Eichreihen gemessenen Werte (PBS versus Kontrollgehirn-Homogenat-Überstand) lässt sich zunächst der Gehalt an Aβ(1-42)-Oligomeren B in der Positivkontrolle quantitativ und im Vergleich dazu dann auch in der Gerhirnprobe behandelter Ratten wie folgt bestimmen.

### 1) Dot Blot-Nachweis

1 µl der Eichreihen-Proben und der Probenextrakte aus den behandelten Tieren werden auf Nitrocellulose-Papier getropft, und Aβ(1-42) wird mit dem Antikörper 6E10 (anti-Aβ(1-42); Fa. Signet) nachgewiesen. Gefärbt wird mit einer an anti-Maus-IgG gekoppelten Alkalischen Phosphatase und durch Zugabe des Färbereagenzes NBT/BCIP.

Während in den Gehirnextrakten von nicht behandelten Ratten kein Aβ(1-42) (0,01 nmol/g) nachweisbar ist, sind in den 15 min nach Behandlung getöten Ratten durch Vergleich der Färbungsintensität mit den entsprechenden Konzentrationen der Positivkontrolle etwa 0,4 nmol/g Aβ(1-42) und in den 120 min nach Behandlung getöteten Ratten nach der gleichen Methode noch ca. 0,2 nmol/g nachweisbar. Daraus ergibt sich für die exogen applizierten Aβ(1-42)-Oligomere B eine mittlere biologische Halbwertszeit von etwa 105 min.

### 2) Western Blot-Nachweis

Alle im Dot Blot anlysierten Proben werden ebenfalls im Western Blot analysiert. Der Western Blot wird ebenfalls mit dem mMAb 6E10 (anti-Aβ(1-42); Fa. Signet) und des Weiteren mit einer an anti-Maus-IgG gekoppelten Alkalischen Phosphatase und durch Zugabe des Färbereagenzes NBT/BCIP entwickelt.

Anti Aβ(1-42)-reaktive Banden treten nur im Bereich 38/48 kDa auf, was dem apparenten Molekulargewicht der Aβ(1-42)-Oligomere B entspricht, d.h die oligomere Struktur bleibt bei in vivo Applikation auch nach 2 Stunden noch erhalten.

Weiterhin können die gleichen Konzentrationen in den Gehirnen der 15 min-Ratten (0,4 nmol/g) sowie der 120 min-Ratten (0,2 nmol/g) durch Vergleich der Färbeintensitäten mit entsprechend intensiv gefärbten Banden der Aβ(1-42)-Oligomere B in der Positivkontrolle wie beim Dot Blot-Verfahren abgeschätzt werden.

### Beispiel 24

### Neurotoxische Wirkung von Aβ(1-42)-Oligomeren mit Molekulargewichten von 38 kDa und 48 kDa [Aβ(1-42)-Oligomere B] auf corticale Neuronen der Maus

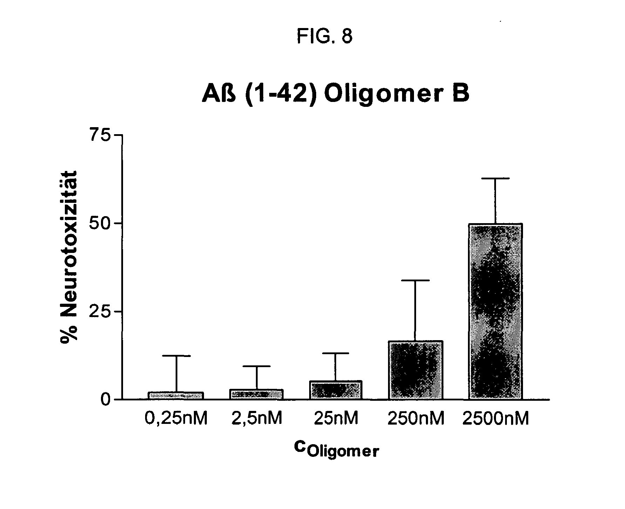

Die Präparation und Kultivierung von murinen corticalen Neuronen erfolgt als Mischkultur mit glialen Zellen in Anlehnung an die Literatur (Choi et al. (1987) J. Neurosci. 7, 357-368). Bei Embryonen werden am 14.-15. Entwicklungstag die Cortices von Hirnhaut und tieferliegenden Hirnbereichen mechanisch befreit. Die Zellen werden durch 5-7-minütige Inkubation in einer 0,05%igen Trypsinlösung bei 37°C sowie anschließendes, mehrmaliges Pipettieren durch eine Pasteurpipette mit reduzierter Öffnung vereinzelt. Nach Bestimmung der Zellzahl werden 430.000 Zellen in 0,5 ml Erhaltungsmedium (Minimum Essential Medium mit 0,8 mM Glutamin, 18 mM Glukose, 23 mM NaHCO₃ und 10% Pferdeserum) pro 2cm² auf Zellkulturmaterial ausgesät, das mit poly-L-Ornithin und Laminin beschichtet worden ist. Die Anzucht und späteren Inkubationen der Zellen erfolgen bei 37°C, 5% CO₂ in einem luftbefeuchteten Zellkulturbruschrank. Nach 3-5-tägiger in Kultur wird die Vermehrung der Glia-Zellen durch eintägige Inkubation mit (+)-5-Fluor-2'-Desoxyuridin/Uridin-Gemisch (je 10µM) unterbrochen. Nach 14 Tagen in Kultur wird die toxische Wirkung von Aβ(1-42)-Oligomeren B untersucht. Dazu werden die Zellen 15 Minuten in Hirnzellpuffer (120 mM NaCl, 5,4 mM KCI, 1,8 mM CaCl₂, 15 mM Glukose, 25 mM HEPES, pH 7,2) inkubiert. Kontrollzellen 1 werden die gleiche Zeit mit 300 µM L-Glutamat in Hirnzellpuffer inkubiert. Die Stammlösung der Aβ-Oligomere wird mit serumfreien Medium (Minimum Essential Medium mit 0,8 mM Glutamin, 20 mM Glukose, 26 mM NaHCO₃) auf verschiedene Endkonzentrationen verdünnt und 24 h inkubiert. Die mit L-Glutamtat behandelten Kontrollzellen 1 werden parallel in serumfreien Medium inkubiert. Eine weitere Gruppe von Zellen (Kontrollzellen 2) wird nur mit Hirnzellpuffer und serumfreien Medium behandelt. Nach 24h werden die Zellkulturüberstände entfernt, die verbleibenden Zellen durch 20-minütige Inkubation in destilliertem Wasser zerstört und die Aktivität des Enzyms Lactatdehydrogenase (LDH) in beiden Lösungen enzymatisch bestimmt. Zur Auswertung wird das Verhältnis der LDH-Aktivität der Zellkulturüberstände zur Summe der LDH-Aktivität aus Überstand und verbliebenen Zellen bestimmt und der Mittelwert der vierfachen Bestimmungen gebildet. Es werden 3 Experimente durchgeführt, wobei jede Versuchbedingung vierfach vorhanden ist (n=12). Der Mittelwert bei den mit Glutamat behandelten Zellen (Kontrollzellen 1) wird mit 100% Neuronentod, der Mittelwert der Zellen, die weder mit L-Glutamat noch mit Aβ-Oligomeren behandelt worden sind (Kontrollzellen 2), mit 0% Neuronentod gleichgesetzt und die Werte der mit Aß-Oligomeren behandelten Zellen entsprechend umgerechnet. Der Mittelwert der Neurotoxizität aller Bestimmungen bei den jeweils verwendeten Konzentrationen zeigt eine deutliche toxische Wirkung der Aß(1-42)-Oligomeren mit Molekulargewichten von 38 kDa und 48 kDa [Aβ(1-42)-Oligomer B], vgl. Fig. 8.

### Beispiel 25

### Herstellung von Antikörpern

Die zur Immunisierung verwendeten Cocktails enthalten in allen Fällen Adjuvant (Fa. Biogenes) mit im Wesentlichen folgenden Bestandteilen:
95 % Paraffinöl
2,4 % Tween 40
0,1 % Cholesterin
0,1 % Lipopolysaccharid

Das Adjuvans wird mit einer Lösung des Antigens im Verhältnis von 2:1 vermischt, bis eine stabile Emulsion erhalten wird. Die Emulsion wird injiziert und bildet ein Depot, aus dem das Antigen kontinuierlich freigesetzt wird.

### a) Herstellung polyklonaler Antiseren durch Immunisierung von Kaninchen mit verkürzten Aβ(20-42)-Oligomeren B aus Beispiel 15a.

2 Kaninchen werden gemäss Standardprotokoll mit nicht konjugierter Aβ(20-42)-Oligomer B-Präparation aus Beispiel 15a immunisiert:
1. Tag Erstimmunsierung und Entnahme des Preimmunserums
7. Tag 1. Boost
14. Tag 2. Boost
28. Tag 3. Boost und Blutung
35. Tag Blutabnahme

Die Antiseren werden aus dem Kaninchenblut durch Abstehen bei Raumtemperatur und anschließende Zentrifugation bei Raumtemperatur erhalten. Das so erhaltene Serum wird als Serum (a1) bezeichnet.

Es werden 2 mg Aβ(20-42)-Oligomer B-Präparation aus Beispiel 15a an LPH, mit Glutardialdehyd unter Standardbedingungen gekoppelt und 2 weitere Kaninchen werden gemäss Standardprotokoll mit diesem konjugiertem Aβ(20-42)-Oligomer B immunisiert:
1. Tag Erstimmunsierung und Entnahme des Preimmunserums
7. Tag 1. Boost
14. Tag 2. Boost
28. Tag 3. Boost und Blutung
35. Tag Blutabnahme

Die Antiseren werden aus dem Kaninchenblut durch Abstehen bei Raumtemperatur und anschließende Zentrifugation bei Raumtemperatur erhalten. Das so erhaltene Serum wird als Serum (a2) bezeichnet.

### b) Herstellung polyklonaler Antiseren durch Immunisierung von Kaninchen mit verkürzten Aβ(12-42)-Oligomeren B aus Beispiel 15b.

2 Kaninchen werden gemäß Standardprotokoll mit nicht konjugierter Aβ(12-42)-Oligomer B-Präparation aus Beispiel 15b immunisiert:
1. Tag Erstimmunsierung und Entnahme des Preimmunserums
7. Tag 1. Boost
14. Tag 2. Boost
28. Tag 3. Boost und Blutung
35. Tag Blutabnahme

Die Antiseren werden aus dem Kaninchenblut durch Abstehen bei Raumtemperatur und anschließende Zentrifugation bei Raumtemperatur erhalten. Das so erhaltene Serum wird als Serum (b1) bezeichnet.

Es werden 2 mg Aβ(12-42)-Oligomer B-Präparation aus Beispiel 15b an LPH mit Glutardialdehyd unter Standardbedingungen gekoppelt und 2 weitere Kaninchen werden gemäss Standardprotokoll mit diesem konjugiertem Aβ(12-42)-Oligomer B immunisiert:
1. Tag Erstimmunsierung und Entnahme des Preimmunserums
7. Tag 1. Boost
14. Tag 2. Boost
28. Tag 3. Boost und Blutung
35. Tag Blutabnahme

Die Antiseren werden aus dem Kaninchenblut durch Abstehen bei Raumtemperatur und anschließende Zentrifugation bei Raumtemperatur erhalten. Das so erhaltene Serum wird als Serum (b2) bezeichnet.

### c) Herstellung polyklonaler Antiseren durch Immunisierung von Kaninchen mit Aβ(1-42)-Oligomeren B-CL aus Beispiel 14a.

2 Kaninchen werden gemäß Standardprotokoll mit nicht konjugierter Aβ(1-42)-Oligomeren B-CL-Präparation aus Beispiel 14a immunisiert:
1. Tag Erstimmunisierung und Entnahme des Preimmunserums
7. Tag 1. Boost
14. Tag 2. Boost
28. Tag 3. Boost und Blutung
35. Tag Blutabnahme

Die Antiseren werden aus dem Kaninchenblut durch Abstehen bei Raumtemperatur und anschließende Zentrifugation bei Raumtemperatur erhalten. Das so erhaltene Serum wird als Serum (c1) bezeichnet.

### d) Herstellung polyklonaler Antiseren durch Immunisierung von Kaninchen mit Aβ(1-42)-Oligomeren-B-Präparation aus Beispiel 6b.

2 Kaninchen werden gemäß Standardprotokoll mit nicht konjugierter Aβ(1-42)-Oligomeren-B-Präparation aus Beispiel 6b immunisiert.
1. Tag Erstimmunisierung und Entnahme des Preimmunserums
7. Tag 1. Boost
14. Tag 2. Boost
28. Tag 3. Boost und Blutung
35. Tag Blutabnahme

Die Antiseren werden aus dem Kaninchenblut durch Abstehen bei Raumtemperatur und anschließende Zentrifugation bei Raumtemperatur erhalten. Das so erhaltene Serum wird als Serum (d1) bezeichnet.

Es werden 2 mg Aβ(1-42)-Oligomeren-B-Präparation aus Beispiel 6b an LPH, mit Glutardialdehyd unter Standardbedingungen gekoppelt und 2 weitere Kaninchen werden gemäß Standardprotokoll mit dieser konjugierten Aβ(1-42)-Oligomer B-Präparation immunisiert:
1. Tag Erstimmunisierung und Entnahme des Preimmunserums
7. Tag 1. Boost
14. Tag 2. Boost
28. Tag 3. Boost und Blutung
35. Tag Blutabnahme

Die Antiseren werden aus dem Kaninchenblut durch Abstehen bei Raumtemperatur und anschließende Zentrifugation bei Raumtemperatur erhalten. Das so erhaltene Serum wird als Serum (d2) bezeichnet.

### Beispiel 26

Charakterisierung polyklonaler Antiseren aus den Immunisierungen von Beispiel 25 auf ihre Kreuzreaktion mit verschiedenen Aβ(1-42)-Formen.

Zur Charakterisierung der polyklonalen Antiseren wurden zu den verschiedenen Aβ(1-42)-Formen Verdünnungsreihen im Bereich von 100 pmol/µl-0,01 pmol/µl in PBS hergestellt. Jeweils 1 µl der Proben wurden auf auf eine Nitrocellulose-Membran aufgetragen: der Nachweis erfolgte mit den entsprechenden Kaninchenseren aus Beispiel 25. Als Vergleich wurde ein Nachweis mit dem mMAb 6E10 (Fa. Signet) durchgeführt. Gefärbt wurde mit einer an anti-Kaninchen-IgG gekoppelten alkalischen Phosphatase (für den Vergleich: eine an anti-Maus-IgG gekoppelte Alkalische Phosphatase) und Zugabe des Färbereagenzes NBT/BCIP. Figur 10 zeigt die so erhaltenen Dot Blots, die sich zahlenmäßig wie folgt auswerten lassen:

| Antigen | 6E10 | Serum (a1) | Serum (a2) | Serum (d1) | Serum (c1) |
|---|---|---|---|---|---|
| Oligomer | 0.1 | 0.5 | 10 | 0.1 | 0.5 |
| Monomer | 0.1 | 10 | 100 | 0.1 | 1 |
| Fibril | 1 | 100 | 100 | 0.5 | 1 |
| APP | 0.01 | 1 | 1 | 0.1 | 0.1 |
| Aβ(1-40)* | 0.1 | 100 | n.b. | 0.5 | 10 |

| | | | | | |
|---|---|---|---|---|---|
| * nicht in Fig. 10 gezeigt | | | | | |

In der Tabelle geben die Zahlen die minimal sichtbare Menge Antigen in [pmol; außer für APP jeweils auf Monomer bezogen] an (Nachweisgrenze).

Der Vergleich der immunologischen Reaktionen mit Kaninchenserum (a1), welches durch Immunisierung mit nicht konjugiertem Aß(20-42)-Oligomer aus Beispiel 15a erhalten wurde, zeigt deutlich, dass die Antikörper, die gegen diese Oligomere gerichtet sind, nur schwach mit den anderen Formen, wie Fibrillen, APP, und Monomer, kreuzreagieren. Dagegen ist eine deutlich stärkere Kreuzreaktion mit dem Aß(1-42)-Oligomeren B (siehe Reihe 1) und außerdem eine Kreuzreaktion mit dem stabilisierten CL-Antigen erkennbar. Diese Daten weisen auf eine deutlich unterschiedliche Struktur der hier vorliegenden Oligomerenform im Vergleich zu APP, Monomer, sowie der Fibrillenstruktur hin. Die Antikörper binden an oligomer-spezifische Strukturen.

Die immunologischen Reaktionen mit Kaninchenserum (a2), welches durch Immunisierung mit konjugiertem Aß(20-42)-Oligomer aus Beispiel 15a erhalten wurde, zeigt ebenfalls, dass die Antikörper, die gegen diese Oligomere gerichtet sind, nur schwach mit den anderen Formen, wie Fibrillen und Monomer kreuzreagieren. Dagegen ist eine schwache Kreuzreaktion mit dem Aβ(1-42)-Oligomeren B (siehe Reihe 1) und außerdem eine Kreuzreaktion mit dem stabilisierten CL-Antigen sowie mit APP erkennbar. Diese Daten weisen auf eine ebenfalls deutlich unterschiedliche Struktur der hier vorliegenden Oligomerenform im Vergleich zu Monomer sowie der Fibrillenstruktur hin.

Im Vergleich dazu zeigen die immunologischen Reaktionen mit Kaninchenserum (d1), welches durch Immunisierung mit Aβ(1-42)-Oligomer aus Beispiel 6b erhalten wurde, sowie Kaninchenserum (c1), welches durch Immunisierung mit Aβ(1-42)-Oligomer B-CL aus Beispiel 14a erhalten wurde, dass die Antikörper, die gegen diese Oligomere gerichtet sind, mit den anderen Formen, wie Fibrillen, APP, und Monomer, vergleichbar stark wie mit den Aβ(1-42)-Oligomeren B (siehe Reihe 1) kreuzreagieren. Diese Antikörper zeigen eine zu mMAb 6E10 (Fa. Signet) vergleichbare Immunreaktion und binden nicht an oligomer-spezifische Strukturen.

In entsprechender Weise wurden Mäuse mit Aβ(1-42)-Oligomeren aus Beispiel 6b immunisiert und in an sich bekannter Weise monoklonale Antikörper etabliert. Dabei sekretierten 2 von 10 Hybrdidoma monoklonale Antikörper, deren Bndungsprofil insbesondere in Bezug auf die oben getesteten Reaktivitäten denen des Antiserums (a1) ähnlich sind.

### Beispiel 27

### Herstellung von Aβ(1-42)-Fibrillen

100 µl 2 mg/ml Aß(1-42)-Lösung in 0.1 % NH₄OH werden mit 300 µl Puffer (20 mM Na-Phosphat, 140 mM NaCl, pH 7.4) auf 0,5 mg/ml verdünnt und mit 0,1 M HCL auf pH 7.4 eingestellt (100 µM Aβ(1-42)). Die Probe wird 24 h bei 37 °C inkubiert und danach 10 min bei 10.000 g abzentrifugiert. Der erhaltene Proteinrückstand wird mit 400 µl Puffer (20 mM Na-Phosphat, 140 mM NaCl, pH 7.4) resuspendiert. Die so erhaltene Aß(1-42)-Fibrillen-Präparation kann bei -20°C aufbewahrt werden und für weitere Untersuchungen eingesetzt werden.

### Beispiel 28

### Effekte von Aβ(1-42)-Oligomere B-CL (aus Beispiel 14b) an Hippokampusschnitten

400 µM dicke, transversale Hippokampusschnitte wurden in einer Tauchschnittkammer bei 34 °C für mindestens 1 h unter Perfusion von begaster Ringerlösung (NaCl 124 mM, KCI 4,9 mM, MgSO₄ 1,3 mM, CaCl₂ 2,5 mM, KH₂PO₄ 1,2 mM, NaHCO₃ 25,6 mM, Glucose 10 mM) adaptiert. Mit Hilfe einer monopolaren Stimulationselektrode wurden danach die Schaffer-Kollaterale erregt und das excitatorische postsynaptische Potential (fEPSP) im Stratum Radiatum abgeleitet. Testpulse wurden mit 30 % der Reiszstärke gegeben, die ein maximales fEPSP erzeugt. Zur Induktion von Langzeit-Potenzierung wurden dreimal alle 10 Minuten 100 Pulse mit einer Breite der Einzelpulse von 200 µs appliziert (starker Tetanus). Die resultierende Potenzierung der fEPSPs wurde für mindestens. 240 Minuten aufgezeichnet. Das Einspülen des Oligomeren B-CL aus Beispiel 14b (500 nM) begann 20 Minuten vor dem ersten Tetanus und wurde 10 Minuten nach dem letzten Tetanus beendet. Der Anstieg (slope) der fEPSPs wurde ermittelt und gegen die Zeit aufgetragen.

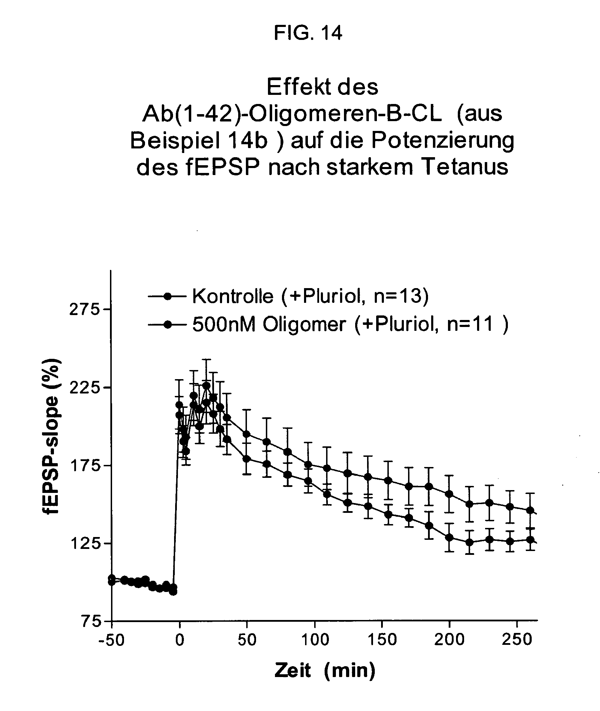

Die Abbildung Fig 14 zeigt, dass der Einwasch von Aβ(1-42)-Oligomeren die Langzeitpotenzierung im Hippokampus vor allem in der anhaltenden Phase ("maintenance phase") unterdrückt. Dementsprechend haben die Aβ(1-42)-Oligomere B-CL einen Einfluß auf die Informationsspeicherung von Nervenzellen (zelluläres Gedächtnis).

### Beispiel 29

### Bindung von Aβ(1-42)-Oligomeren B (aus Beispiel 5b) an primäre hippocampale Ratten-Neuronen

Es wurde die Bindung der Ratten-Aβ(1-42)-Oligomere B aus Beispiel 5b an primäre hippocampale Rattenneuronen untersucht. Die Hippocampus-Neuronen wurden in Neurobasalmedium mit B27-Supplement auf Poly-L-Lysin-beschichteten Deckgläschen kultiviert und am Tag 14 der Kultur verwendet. Die Bindung der Oligomere an die Zellmembran der Neuronen erfolgte durch Zugabe von 200 nM (totale monomere Aß-Konzentration) Oligomeren in frisches Kulturmedium und einer Inkubation für 15 min. bei 37 °C. Nach Abnahme des Aβ-haltigen Mediums wurde zweimal mit Medium gewaschen und die Zellen anschließend in 3.7 % Formaldehyd fixiert. Nach weiterem Waschen der Zellen wurden unspezifische Bindungsstellen mit 10 % normalem Esel-Serum in PBS-Puffer 90 min bei Raumtemperatur blockiert. Als 1. Antikörper wurde 6E10 (aus Maus) in einer Verdünnung von 1:2000 2 h bei Raumtemperatur appliziert. Die Zellen wurden erneut gewaschen und mit dem 2. Antikörper (aus Esel), welcher gegen Maus gerichtet ist und an den der Fluoreszenzfarbstoff Cy3 gekoppelt ist, 2 h bei Raumtemperatur inkubiert. Nach erneutem Waschen der Zellen wurden die Deckgläschen mit den Neuronen auf einem Objektträger mit Eindeckmedium fixiert. Die hippocampalen Neuronen mit gebundenen Aß-Oligomeren wurden im Fluoreszenzmikroskop dargestellt. Als Kontrolle wurde ein Ansatz verwendet, bei dem der 1. Antikörper 6E10 weggelassen wurde. Diese Kontrolle zeigt somit die unspezifische Fluoreszenz, die nicht auf Aβ basiert.

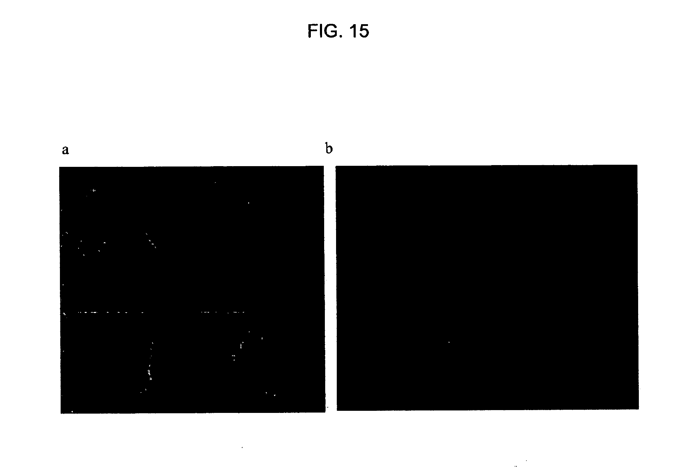

Wie in Abb. 15a zu sehen ist, binden die Oligomere an die Zelloberfläche der Neuronen in einer punktierten Weise. Im Gegensatz dazu zeigt die Kontrolle ohne den 1. Antikörper 6E10 nur eine geringe unspezifische Fluoreszenz durch Bindung des 2. Antikörpers an die Neuronen (Abb. 15b).

Zu weiteren beispielhaften und nicht beschränkenden Ausführungsformen der Erfindung gehören:
E-1. Oligomere des Amyloid-β(1-42)-Proteins mit einem apparenten Molekulargewicht in der SDS-Gelelektrophorese von etwa 15 kDa, oder ein Derivat davon.
E-2. Oligomere des Amyloid-β(1-42)-Proteins mit einem apparenten Molekulargewicht in der SDS-Gelelektrophorese von etwa 20 kDa, oder ein Derivat davon.
E-3. Oligomere des Amyloid-β(1-42)-Proteins mit einem apparenten Molekulargewicht in der SDS-Gelelektrophorese von etwa 38 kDa, oder ein Derivat davon.
E-4. Oligomer edes Amyloid-β(1-42)-Proteins mit einem apparenten Molekulargewicht in der SDS-Gelelektrophorese von etwa 48 kDa, oder ein Derivat davon.
E-5. Zusammensetzungen, umfassend ein Gemisch aus einem Oligomer nach Ausführungsform E-1 und einem Oligomer nachE-2, oder Derivaten davon.
E-6. Zusammensetzungen, umfassend ein Gemisch aus einem Oligomer nach Ausführungsform E-3 und einem Oligomer nach Ausführungsform E-4, oder Derivaten davon.
E-7. Oligomere oder Zusammensetzungen nach einer der Ausführungsformen E-1 bis E-6, dadurch gekennzeichnet, dass die Derivate eine nachweisbare Markierung aufweisen.
E-8. Oligomere oder Zusammensetzungen nach Ausführungsform E-7, dadurch gekennzeichnet, dass die Markierung eine fluoreszierende, lumineszierende, colorimetrische, radioaktive oder magnetische Markierung, oder eine Markierung mit Affiniät zu komplementären Bindungspartnern ist.
E-9. Oligomere oder Zusammensetzungen nach einer der Ausführungsformen E-1 bis E-8, dadurch gekennzeichnet, dass das Oligomer oder das Derivat quervernetzt ist.
E-10. Oligomere oder Zusammensetzungen nach einer der Ausführungsformen E-1 bis E-8, dadurch gekennzeichnet, dass das Derivat durch proteolytische Spaltung des Oligomers erhältlich ist.
E-11. Oligomere oder Zusammensetzungen nach Ausführungsform E-10, dadurch gekennzeichnet, dass das Derivat ein Oligomer eines Aß(x-42)-Fragments ist, wobei x für 8 bis 24, vorzugsweise für 10 bis 22 und insbesondere für 12 bis 20 steht.
E-12. Oligomere oder Zusammensetzungen nach Ausführungsform E-11, dadurch gekennzeichnet, dass das Derivat ein Oligomer des Aß(12-42)-Fragments oder des Aß(20-42)-Fragments ist.
E-13. Verfahren zur Herstellung von Oligomeren eines gegebenenfalls derivatisierten Amyloid-β(1-42)-Proteins, dadurch gekennzeichnet, dass man ein Detergenz auf monomeres Amyloid-β(1-42)-Protein oder ein Derivat davon einwirken lässt und gegebenenfalls wenigstens ein Oligomer oder Derivat davon in an sich bekannter Weise gewinnt.
E-14. Verfahren zur Herstellung von Oligomeren eines gegebenenfalls derivatisierten Amyloid-β(1-42)-Proteins, dadurch gekennzeichnet, dass man ein Detergenz auf monomeres Amyloid-β(1-42)-Protein oder ein Derivat davon einwirken lässt, anschließend die Detergenzwirkung verringert und weiter inkubiert, und gegebenenfalls wenigstens ein Oligomer oder Derivat davon in an sich bekannter Weise gewinnt.
E-15. Verfahren nach Ausführungsform E-14, dadurch gekennzeichnet, dass das Detergenz ionisch ist.
E-16. Verfahren nach Ausführungsform E-15, dadurch gekennzeichnet, dass das Detergenz Natriumdodecylsulfat ist.
E-17. Verfahren nach einer der Ausführungsformen E-13 bis E-16, dadurch gekennzeichnet, dass die Einwirkungszeit des Detergenz etwa 1 bis 20 Stunden beträgt.
E-18. Verfahren nach Ausführungsform E-17, dadurch gekennzeichnet, dass die Einwirkungstemperatur etwa 20 bis 50°C beträgt.
E-19. Verfahren nach einer der Ausführungsformen E-14 bis E-16, dadurch gekennzeichnet, dass die weitere Inkubationszeit etwa 10 bis 30 Stunden beträgt.
E-20. Verfahren nach Ausführungsform E-19, dadurch gekennzeichnet, dass die Inkubationstemperatur etwa 20 bis 50°C beträgt.
E-21. Verfahren nach einer der Ausführungsformen E-13 bis E-20, dadurch gekennzeichnet, dass das Amyloid-β(1-42)-Protein zuvor zumindest partiell entfaltet.
E-22. Verfahren nach Ausführungsform E-21, dadurch gekennzeichnet, dass man dazu ein Wasserstoffbrücken-brechendes Agenz, vorzugsweise HFIP, auf das Protein einwirken lässt.
E-23. Verfahren nach einer der Ausführungsformen E-13 bis E- 22, dadurch gekennzeichnet, dass man ein Quervernetzungsmittel auf das Oligomer einwirken lässt.
E-24. Verfahren nach einer der Ausführungsformen E-13 bis E-23, dadurch gekennzeichnet, dass man eine Protease auf das Oligomer einwirken lässt.
E-25. Verwendung eines Oligomers, eines Derivates davon oder einer Zusammensetzung nach einer der Ausführungsformen E-1 bis E-12 in einem diagnostischen *in vitro* Nachweisverfahren.
E-26. Verwendung eines Oligomers, eines Derivates davon oder einer Zusammensetzung nach einer der Ausführungsformen E-1 bis E-12 in einem diagnostischen *in vivo* Nachweisverfahren.
E-27. Verwendung eines Oligomers, eines Derivates davon oder einer Zusammensetzung nach einer der Ausführungsformen E-1 bis E-12 für die Erzeugung von oligomerspezifischen Antikörpern.
E-28. Verfahren zur Charakterisierung einer Substanz oder eines Substanzgemisches, wobei man die Substanz oder das Substanzgemisches zur Verfügung stellt; die Substanz oder das Substanzgemisch auf ein Oligomer, Derivat davon oder eine Zusammensetzung nach einer der Ausführungsformen E-1 bis E-12 einwirken lässt; und bestimmt, ob die Substanz oder bestimmte Teile des Substanzgemisches an das Oligomer, Derivat davon oder wenigstens ein in der Zusammensetzung enthaltenes Oligomer oder Derivat davon binden.
E-29. Substanzen oder Teile eines Substanzgemisches, dadurch gekennzeichnet, dass die Substanzen oder der Teile mit einem Verfahren nach Ausführungsform E-28 als ein an das Oligomer, Derivat davon oder wenigstens ein in der Zusammensetzung enthaltenes Oligomer oder Derivat davon bindender Ligand identifizierbar sind.
E-30. Verwendung einer Substanz oder eines Teils eines Substanzgemisches nach Ausführungsform E-29 zur Herstellung eines Medikaments zur Behandlung Amyloid-β-assoziierter, insbesondere dementieller Erkrankungen.
E-31. Verwendung einer Substanz oder eines Teils eines Substanzgemisches nach Ausführungsform E-29 zur Herstellung einer Zusammensetzung zur Diagnose Amyloid-β-assoziierter, insbesondere dementieller Erkrankungen.
E-32. Verfahren zur Herstellung von Antikörpern, wobei man einen Wirt mit wenigstens einem Oligomer, Derivat davon oder einer Zusammensetzung nach einer der Ausführungsformen E-1 bis E-12 immunisiert; und ein als Antwort auf die Immunisierung gebildetes Antikörper-haltiges Serum des Wirts gewinnt.
E-33. Verfahren nach Ausführungsform E-32, dadurch gekennzeichnet, dass man wenigstens einen Antikörper des Serums selektiert, der das als Immunogen verwendete Oligomer, Derivat davon oder wenigstens ein in der als Immunogen verwendeten Zusammensetzung enthaltenes Oligomer oder Derivat davon erkennt.
E-34. Verfahren zur Herstellung von Antikörpern mit Spezifität für ein Oligomer oder Derivat davon nach einer der Ausführungsformen E-1 bis E-12, wobei man ein das Oligomer oder Derivat davon beinhaltendes Antigen bereitstellt; das Antigen auf ein Antikörper-Repertoire einwirken lässt; und aus dem Repertoire einen Antikörper selektiert, der an das Oligomer oder Derivat davon spezifisch bindet.
E-35. Verfahren nach Ausführungsform E-34, wobei man das Antigen auf das Antikörper-Repertoire *in vivo* einwirken lässt, indem man ein Tier mit dem Antigen immunisiert.
E-36. Verfahren nach Ausführungsform E-34, wobei man des Weiteren aus Lymphozyten des Tieres eine Reihe von Hybridomen etabliert und ein Hybridom selektiert, das einen spezifisch an das Oligomer oder Derivat davon bindenden Antikörper sekretiert.
E-37. Verfahren nach Ausführungsform E-34, wobei das Tier ausgewählt ist unter Mäusen, Ratten, Hühnern, Kameliden, Cynomolus-Affen, Kaninchen und Ziegen.
E-38. Verfahren nach Ausführungsform E-37, wobei das Tier eine Knockout-Maus mit APP-Defizienz ist.
E-39. Verfahren nach Ausführungsform E-37, wobei das Tier eine transgene Maus mit humanen Immunglobulingenen ist, die nach antigenem Stimulus menschliche Antikörper erzeugt.
E-40. Verfahren nach Ausführungsform E-37, wobei das Tier eine Maus mit schwerer kombinierter Immundefizienz (SCID) ist, die mit humanen peripheren mononukleären Blutzellen oder lymphoiden Zellen oder Vorläufern davon rekonstituiert ist.
E-41. Verfahren nach Ausführungsform E-37, wobei das Tier eine Maus ist, die mit einer letalen Ganzkörperbestrahlung behandelt wurde, anschließend mit Knochenmarkszellen aus einer Maus mit schwerer kombinierter Immundefizienz (SCID) gegen Strahlung geschützt wurde, und der anschließend funktionale humane Lymphozyten oder Vorläufer davon transplantiert wurden.
E-42. Verfahren nach Ausführungsform E-34, wobei man das Antigen auf das Antikörper-Repertoire in vitro einwirken lässt, indem man eine rekombinante Antikörperbank mit dem Antigen screent.
E-43. Verfahren nach Ausführungsform E-42, wobei die rekombinante Antikörperbank auf der Oberfläche eines Bakteriophagen exprimiert ist.
E-44. Verfahren nach Ausführungsform E-42, wobei die rekombinante Antikörperbank auf der Oberfläche von Hefezellen exprimiert ist.
E-45. Verfahren nach Ausführungsform E-42, wobei die rekombinante Antikörperbank auf der Oberfläche von bakteriellen Zellen exprimiert ist.
E-46. Verfahren nach Ausführungsform E-42, wobei die rekombinante Antikörperbank als RNA-Protein-Fusionen exprimiert ist.
E-47. Verfahren nach Ausführungsform E-42, wobei die rekombinante Antikörperbank eine scFv-Bank oder eine Fab-Bank ist.
E-48. Verfahren nach Ausführungsform E-42, wobei die rekombinante Antikörperbank eine Einzeldomänen-Bank ist.
E-49. Verfahren nach Ausführungsform E-34, wobei man das Antigen auf das Antikörper-Repertoire einwirken lässt, indem man ein Tier mit dem Antigen *in vivo* immunisiert, und anschließend eine aus den lymphoiden Zellen des Tieres hergestellte rekombinante Antikörperbank mit dem Antigen screent.
E-50. Verfahren nach Ausführungsform E-34, wobei man das Antigen auf das Antikörper-Repertoire einwirken lässt, indem man ein Tier mit dem Antigen *in vivo* immunisiert und anschließend eine aus dem lymphoiden Zellen des Tieres hergestellte rekombinante Antikörperbank *in vitro* einer Affinitätsreifung unterzieht.
E-51. Verfahren nach Ausführungsform E-34, wobei man das Antigen auf das Antikörper-Repertoire einwirken lässt, indem man ein Tier mit dem Antigen immunisiert und anschließend einzelne Zellen, die den antigenbindenden Antikörper sekretieren, selektiert und aus den Einzelzellen cDNAs für die variablen Regionen der schweren und leichten Kette gewinnt.
E-52. Antikörper, erhältlich mit einem Verfahren nach einer der Ausführungsformen E-32 bis E-51.
E-53. Proteine, die spezifisch an ein Oligomer oder Derivat davon nach einer der Ausführungsformen E-1 bis E-12 binden.
E-54. Proteine nach Ausführungsform E-53, wobei die Proteine das Oligomer oder Derivat davon mit einer Affinität im Bereich von K_{D}=10⁻⁶-10⁻¹² M binden.
E-55. Proteine nach Ausführungsform E-54, wobei die Proteine das Oligomer oder Derivat davon mit höherer Affinität als K_{d}=10⁻⁸ M binden.
E-56. Proteine nach Ausführungsform E-54, wobei die Proteine das Oligomer oder Derivat davon mit mit höherer Affinität als K_{d}=10⁻⁹ M binden.
E-57. Proteine nach Ausführungsform E-54, wobei die Proteine das Oligomer oder Derivat davon mit höherer Affinität als K_{d}=10⁻¹⁰ M binden.
E-58. Proteine nach Ausführungsform E-54, wobei die Proteine das Oligomer oder Derivat davon mit höherer Affinität als K_{d}=10⁻¹¹ M binden.
E-59. Proteine nach einer der Ausführungsformen E-53 bis E-58, wobei die Proteine monomeres A□(1-42)-Protein und/oder monomeres A□(1-40)-Protein mit geringerer Affinität als K_{d}=10⁻⁸ M binden.
E-60. Proteine nach einer der Ausführungsformen E-53 bis E-59, wobei die Affinität der Proteine für das Oligomer oder Derivat davon mindestens 10-fach höher sind als für monomeres A□(1-42)-Protein und/oder monomeres A□(1-40)-Protein.
E-61. Proteine nach einer der Ausführungsformen E-53 bis E-59, wobei die Affinität der Proteine für das Oligomer oder Derivat davon mindestens 100-fach höher sind als für monomeres A□(1-42)-Protein und/oder monomeres A□(1-40)-Protein.
E-62. Proteine nach einer der Ausführungsformen 53 bis 59, wobei die Affinität der Proteine für das Oligomer oder Derivat davon mindestens 1000-fach höher sind als für monomeres A□(1-42)-Protein und/oder monomeres A□(1-40)-Protein.
E-63. Proteine nach einer der Ausführungsformen E-53 bis E-62, wobei die Proteine das Oligomer oder Derivat davon mit einer k_{off}-Geschwindigkeitskonstanten von 0,1 s-1 oder weniger, mittels Oberflächenplasmonresonanz bestimmt, binden.
E-64. Proteine nach einer der Ausführungsformen E-53 bis E-63, wobei die Proteine die Aktivität des Oligomers oder Derivats davon mit einer Hemmkonstante IC₅₀ von 1 x 10⁻⁵ oder weniger inhibieren.
E-65. Proteine nach einer der Ausführungsformen E-53 bis E-64, wobei die Proteine Antikörper siind, oder ein antigenbindender Teil davon.
E-66. Antikörper nach Ausführungsform E-65, wobei die Antikörper ein im Wesentlichen humaner Antikörper sind, oder ein antigenbindender Teil davon.
E-67. Antikörper nach Ausführungsform E-65, wobei die Antikörper ein chimärer Antikörper sind, oder ein antigenbindender Teil davon.
E-68. Antikörper nach Ausführungsform E-65, wobei die Antikörper ein CDR-Graft-Antikörper sind, oder ein antigenbindender Teil davon.
E-69. Proteine nach einer der Ausführungsformen E-53 bis E-64, wobei die Proteine ein vom T-Zellrezeptor abstammendes Molekül oder eine vom T-Zellrezeptor abstammende Rezeptordomäne, oder ein Fusionsprotein der Rezeptordomäne mit einem Fc-Teil eines Immunoglobulins sind.
E-70. Pharmazeutische Mittel, enhaltend ein Protein nach einer der Ausführungsformen E-53 bis E-69 und gegebenenfalls einen pharmazeutisch verträglichen Träger.
E-71. Verwendung eines Proteins nach einer der Ausführungsformen E-53 bis E-69 zur Herstellung eines Medikaments zur Behandlung Amyloid-β-assoziierter, insbesondere dementieller Erkrankungen.
E-72. Verwendung eines Proteins nach einer der Ausführungsformen E-53 bis E-69 zur Herstellung einer Zusammensetzung zur Diagnose Amyloid-β-assoziierter, insbesondere dementieller Erkrankungen.
E-73. Vakzin, umfassend wenigstens ein Oligomer oder wenigstens ein Derivat davon oder eine Zusammensetzung nach einer der Ausführungsformen E-1 bis E-12.
E-74. Verwendung eines Oligomer oder Derivates davon oder einer Zusammensetzung nach einer der Ausführungsformen E-1 bis E-12 zur Herstellung eines Vakzins zur Behandlung Amyloid-β-assoziierter, insbesondere dementieller Erkrankungen.

## Patentansprüche

1. Antikörper, der spezifisch an ein oligomeres Amyloid-β(1-42)-Protein mit einem apparenten Molekulargewicht in der SDS-Gelelektrophorese von etwa 38 kDa und/oder 48 kDa bindet; oder ein antigenbindender Teil davon.

2. Antikörper, der an ein oligomeres Amyloid-β(1-42)-Protein mit einem apparenten Molekulargewicht in der SDS-Gelelektrophorese von etwa 38 kDa und/oder 48 kDa mit einer Affinität bindet, die höher als die Affinität des Antikörpers für monomeres Aß(1-42)-Protein ist; oder ein antigenbindender Teil davon.

3. Antikörper, der spezifisch an ein oligomeres Amyloid-β(1-42)-Protein mit einem apparenten Molekulargewicht in der SDS-Gelelektrophorese von etwa 15 kDa und/oder 20 kDa bindet; oder ein antigenbindender Teil davon.

4. Antikörper, der an ein oligomeres Amyloid-β(1-42)-Protein mit einem apparenten Molekulargewicht in der SDS-Gelelektrophorese von etwa 15 kDa und/oder 20 kDa mit einer Affinität bindet, die höher als die Affinität des Antikörpers für monomeres Aß(1-42)-Protein ist; oder ein antigenbindender Teil davon.

5. Antikörper, der spezifisch an ein oligomeres Amyloid-β(1-42)-Protein, oligomeres Amyloid-β(1-43)-Protein oder oligomeres Amyloid-β(1-44)-Protein bindet, wobei das oligomere Protein erhältlich ist, indem man ein Detegenz auf monomeres Amyloid-β(1-42)-Protein, monomeres Amyloid-β(1-43)-Protein oder monomeres Amyloid-β(1-44)-Protein einwirken lässt, die Detegenzwirkung verringert und weiter inkubiert, und das oligomere Protein gewinnt; oder ein antigenbindender Teil davon.

6. Antikörper, der an ein oligomeres Amyloid-β(1-42)-Protein, oligomeres Amyloid-β(1-43)-Protein oder oligomeres Amyloid-β(1-44)-Protein mit einer Affinität bindet, die höher als die Affinität des Antikörpers für monomeres Aβ(1-42)-Protein ist, wobei das oligomere Protein erhältlich ist, indem man ein Detegenz auf monomeres Amyloid-β(1-42)-Protein, monomeres Amyloid-β(1-43)-Protein oder monomeres Amyloid-β(1-44)-Protein einwirken lässt, die Detegenzwirkung verringert und weiter inkubiert, und das oligomere Protein gewinnt; oder ein antigenbindender Teil davon.

7. Antikörper, der spezifisch an ein oligomeres Amyloid-β(1-42)-Protein, oligomeres Amyloid-β(1-43)-Protein oder oligomeres Amyloid-β(1-44)-Protein bindet, wobei das oligomere Protein erhältlich ist, indem man ein Detegenz auf monomeres Amyloid-β(1-42)-Protein, monomeres Amyloid-β(1-43)-Protein oder monomeres Amyloid-β(1-44)-Protein einwirken lässt, und das oligomere Protein gewinnt; oder ein antigenbindender Teil davon.

8. Antikörper, der an ein oligomeres Amyloid-β(1-42)-Protein, oligomeres Amyloid-β(1-43)-Protein oder oligomeres Amyloid-β(1-44)-Protein mit einer Affinität bindet, die höher als die Affinität des Antikörpers für monomeres Aβ(1-42)-Protein ist, wobei das oligomere Protein erhältlich ist, indem man ein Detegenz auf monomeres Amyloid-β(1-42)-Protein, monomeres Amyloid-β(1-43)-Protein oder monomeres Amyloid-β(1-44)-Protein einwirken lässt, und das oligomere Protein gewinnt; oder ein antigenbindender Teil davon.

9. Antikörper oder antigenbindender Teil davon nach einem der Ansprüche 5 bis 8, wobei das Detergenz Natriumdodecylsulfat ist.

10. Antikörper oder antigenbindender Teil davon nach einem der Ansprüche 1 bis 9, wobei der Antikörper das oligomere Protein mit höherer Affinität als K_{d}=10⁻⁸ M, mit höherer Affinität als K_{d}=10⁻⁹ M, mit höherer Affinität als K_{d}=10⁻¹⁰ M, oder mit höherer Affinität als K_{d}=10⁻¹¹ M bindet.

11. Antikörper oder antigenbindender Teil davon nach einem der Ansprüche 1 bis 10, wobei der Antikörper monomeres Aβ(1-42)-Protein und/oder monomeres Aβ(1-40)-Protein mit geringerer Affinität als K_{d}=10⁻⁸ M bindet.

12. Antikörper oder antigenbindender Teil davon nach einem der Ansprüche 1 bis 11, wobei die Affinität des Antikörpers für das oligomere Protein mindestens 10-fach höher ist als für monomeres Aβ(1-42)-Protein und/oder monomeres Aβ(1-40)-Protein, mindestens 100-fach höher ist als für monomeres Aβ(1-42)-Protein und/oder monomeres Aβ(1-40)-Protein, oder mindestens 1000-fach höher ist als für monomeres Aβ(1-42)-Protein und/oder monomeres Aβ(1-40)-Protein.

13. Antikörper oder antigenbindender Teil davon nach einem der Ansprüche 1 bis 12, wobei der Antikörper ein polyklonaler oder monoklonaler Antikörper, oder ein im Wesentlichen humaner Antikörper, ein chimärer Antikörper, ein CDR-Graft-Antikörper oder ein humanisierter Antikörper ist.

14. Pharmazeutisches Mittel, umfassend einen Antikörper oder einen antigenbindenden Teil davon nach einem der Ansprüche 1 to 13 und gegebenenfalls einen pharmazeutisch verträglichen Träger.

15. Antikörper oder antigenbindender Teil davon nach einem der Ansprüche 1 bis 13 zur Verwendung bei der Behandlung einer Amyloidose, beispielsweise der Alzheimer-Krankheit.


SEQUENZPROTOKOLL

<110>  Abbott GmbH  Co. KG

<120>  Amyloid-β(1-42)-Oligomere, Derivate davon und Antikörper
dafür, Verfahren zu deren Herstellung und deren Verwendung

<130>  M-44023

<150>  DE 103 03 974.0

<151>  2003-01-31

<160>  2

<170>  PatentIn version 3.1

<210>  1

<211>  42

<212>  PRT

<213>  Homo sapiens

<400>  1

<210>  2

<211>  42

<212>  PRT

<213>  Rattus sp.

<400>  2

Asp Ala Glu Phe Gly His Asp Ser Gly Phe Glu Val Arg His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val Ile Ala
        35                  40